# EUROPEAN PATENT SPECIFICATION

(11) **EP 0 626 942 B1**
(45) Date of publication and mention of the grant of the patent: **23.04.1997**
(21) Application number: 93904230.5
(22) Date of filing: 19.02.1993
(51) Int. Cl.: C07C 233/63, C07C 233/58, C07C 237/22, C07D 207/16, C07D 211/60, C07D 209/20, C07K 5/06, A61K 31/16

(54) **BICYCLO[2.2.2.]OCTANE DERIVATIVES AS CHOLESTOCYSTOKININ INHIBITORS**
BICYCLO[2.2.2.]OKTAN DERIVATE ALS CHOLESTOCYSTOKININ HARNSTOFFE
DERIVES DE BICYCLO[2.2.2.]OCTANE UTILISES COMME INHIBITEURS DE CHOLESTOCYSTOKININE

(30) Priority: 20.02.1992 GB 9203608; 19.06.1992 GB 9213093; 24.11.1992 GB 9224629
(43) Date of publication of application: 07.12.1994
(73) Proprietor: JAMES BLACK FOUNDATION LIMITED, London SE24 9JE (GB)
(72) Inventor: KALINDJIAN, Sarkis, Barret, Banstead, Surrey SM7 2EJ (GB); LOW, Caroline, Minli Rachel, Croydon, Surrey CR0 5LU (GB); MCDONALD, Iain Mair, Paddock Wood, Kent TN12 6AE (GB); HULL, Robert Antony David, Tonbridge, Kent TN11 9AP (GB); SHANKLEY, Nigel Paul, Nr. Edenbridge, Kent TN8 6SD (GB); BUCK, Ildiko, Maria, London N19 3XU (GB); STEEL, Katherine, Isobel, Mary, Beckenham, Kent BR3 1QP (GB); DAVIES, Jonathan, Michael, Richard, Beckenham, Kent BR3 4HT (GB); DUNSTONE, David John, Plaistow, London E13 9LP (GB); HARPER, Elaine Anne, London SE13 7QX (GB); PETHER, Michael John, Carford, London SE26 2SF (GB); BODKIN, Michael James, Chiswick, London W4 5EU (GB)
(74) Representative: Fisher, Adrian John
(86) International application number: GB9300346
(87) International publication number: WO9316982

(56) References cited:
- EP-A- 0 405 537
- US-A- 3 577 366
- US-A- 3 950 407
- US-A- 4 306 063
- JOURNAL OF ORGANIC CHEMISTRY. vol. 53, no. 25, 9 December 1988, EASTON US pages 5831 - 9 E. WEBER ET. AL. 'Design of Roof-Shaped Clathrate Hosts. Inclusion Properties and X-ray Crystal Structures of a Free Host and of Inclusion Compounds with 1-BuOH and DMF'
- JOURNAL OF THE CHEMICAL SOCIETY, CHEMICAL COMMUNICATIONS vol. 1984, no. 23, December 1984, LETCHWORTH GB pages 1632 - 4 M. CZUGLER ET. AL. 'Selective Clathrate Formation with the New Host System cis-and trans-9,10-Dihydro-9,10-ethanoanthracene-1 1,12-dicarboxylic acid: Inclusion Properties and X-ray Structure of an Encapsulated Acetic Acid Dimer.'
- CHEMICAL ABSTRACTS, vol. 101, no. 25, 17 December 1984, Columbus, Ohio, US; abstract no. 229865n, A.K.SINGH ET. AL. 'PMR Spectral Studies of Diels-Alder adducts: Anthracene-crotonic acid, anthracene-fumaric acid and beta-naphthol-fumaric acid' page 712 ;column 1 ;
- CHEMICAL ABSTRACTS, vol. 73, no. 1, 6 July 1970, Columbus, Ohio, US; abstract no. 3689z, I. NANU ET. AL. 'Esters of 9,10-dihydroanthracene-endo-alpha-beta succinic acid as models for the study of large molecule plasticisers' page 314 ;column 2 ;
- JOURNAL OF THE AMERICAN CHEMICAL SOCIETY. vol. 94, no. 5, 8 March 1972, GASTON, PA US pages 1693 - 8 G.A. RUSSELL ET. AL. 'Aliphatic Semidiones. XIX. Polycyclic Derivatives of Cyclobutanesemidione'

## Description

This invention relates to bicyclo[2.2.2]octane derivatives, and more particularly to bicyclo[2.2.2]octane derivatives which bind to cholecystokinin and/or gastrin receptors. The invention also relates to methods for preparing such bicyclo[2.2.2]octane derivatives and to compounds which are useful as intermediates in such methods.

Gastrin and the CCK's are structurally-related neuropeptides which exist in gastrointestinal tissue and in the CNS (see Mutt V., Gastrointestinal Hormones, Glass G.B.J., ed., Raven Press, N.Y., p 169 and Nisson G., ibid, p. 127).

Gastrin is one of the three primary stimulants of gastric acid secretion. Several forms of gastrin are found including 34-, 17-, and 14-amino acid species with the minimum active fragment being the C-terminal tetrapeptide (TrpMetAspPhe-NH₂) which is reported in the literature to have full pharmacological activity (see Tracey H.J. and Gregory R.A., Nature (London), 1964, 204, 935). Much effort has been devoted to the synthesis of analogues of this tetrapeptide (and the N-protected derivative Boc-TrpMetAspPhe-NH₂) in an attempt to elucidate the relationship between structure and activity.

Natural cholecystokinin is a 33 amino acid peptide (CCK-33), the C-terminal 5 amino acids of which are identical to those of gastrin. Also found naturally is the C-terminal octapeptide (CCK-8) of CCK-33.

The cholecystokinins are reported to be important in the regulation of appetite. They stimulate intestinal motility, gall bladder contraction, pancreatic enzyme secretion, and are known to have a trophic action on the pancreas. They also inhibit gastric emptying and have various effects in the CNS.

Compounds which bind to cholecystokinin and/or gastrin receptors are important because of their potential pharmaceutical use as antagonists of the natural peptides.

A number of gastrin antagonists have been proposed for various therapeutic applications, including the prevention of gastrinrelated disorders, gastrointestinal ulcers, Zollinger-Ellison syndrome, antral G Cell hyperplasia and other conditions in which lowered gastrin activity is desirable. The hormone has also been shown to have a trophic action on cells in the stomach and so an antagonist may be expected to be useful in the treatment of cancers, particularly in the stomach.

Possible therapeutic uses for cholecystokinin antagonists include the control of appetite disorders such as anorexia nervosa, and the treatment of pancreatic inflammation, biliary tract disease and various psychiatric disorders. Other possible uses are in the potentiation of opiate (e.g. morphine) analgesia, and in the treatment of cancers, especially of the pancreas. Moreover, ligands for cholecystokinin receptors in the brain (so-called CCK₈ receptors) have been claimed to possess anxiolytic activity.

According to the present invention, there are provided compounds for use in therapy (and particularly for use as gastrin and/or CCK antagonists), such compounds being of the formula 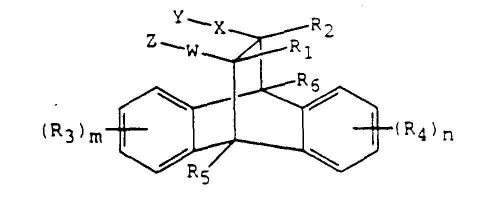 wherein
W is a carbonyl, sulphonyl or sulphinyl group, and X is a carbonyl, sulphonyl or sulphinyl group or -C(O)-CH₂- (in which the carbonyl group is bonded to Y), provided that at least one of W and X contains carbonyl,
Y is R₇-O- or R₇-N(R₅)- (wherein R- is H or C₁ to C₁₅ hydrocarbyl, up to two carbon atoms of the hydrocarbyl moiety optionally being replaced by a nitrogen, oxygen or sulphur atom provided that Y does not contain a -O-O-group, and R₈ is H, C₁ to C₃ alkyl, carboxymethyl or esterified carboxymethyl),
Z is selected from

   i) -O-R₉

   wherein R₉ is H, C₁ to C₅ alkyl, phenyl, substituted phenyl, benzyl or substituted benzyl; 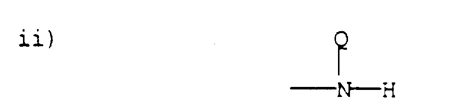
   wherein Q is H, C₁ to C₅ hydrocarbyl, or -R₁₀-U, wherein R₁₀ is a bond or C₁ to C₃ alkylene and U is aryl, substituted aryl, heterocyclic, or substituted heterocyclic, 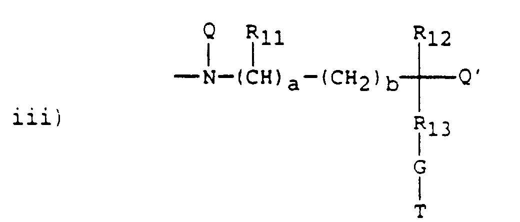
   wherein a is 0 or 1 and b is from 0 to 3,
      R₁₁ is H or methyl,
      R₁₂ is H or methyl; or R₁₂ is CH₂= and Q' is absent; or R₁₁ and R₁₂ are linked to form a 3- to 7-membered ring,
      R₁₃ is a bond or C₁ to C₃ hydrocarbylene,
      G is a bond, -CHOH- or -C(O)-
      C is as recited above for Q or -R₁₀-(C(O))_{d}-L-(C(O))ₑ-R₉ (wherein R₉ and R₁₀ are as defined above, L is O, S or -N(R₁₄)-, in which R₁₄ is as defined above for R₈, and d and e are 0 or 1, provided that d+e<2); or Q' and R₁₂, together with the carbon atom to which they are attached, form a 3- to 7-membered ring,
      Q is as defined above; or Q and R₁₂ together form a group of the formula -(CH₂)_{f}-V-(CH₂)_{g}- wherein V is -S-, -S(O)-, -S(O)₂-, -CH₂-, -CHOH- or -C(O)-, f is from 0 to 2 and g is from 0 to 3; or, when Q' is -R₁₀-U and U is an aromatic group, Q may additionally represent a methylene link to U, which link is *ortho* to the R₁₀ link to U,
      T is H, cyano, C₁ to C₄ alkyl, -CH₂OH, carboxy, esterified carboxy or amidated carboxy; or 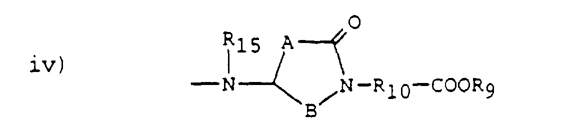
   wherein A and B are independently a bond or C₁ to C₃ alkylene, provided that A and B together provide from 2 to 4 carbon atoms in the ring, R₉ and R₁₀ are as defined above, and R₁₅ is as defined above for R₈
or Z is absent and W is H,
R₁ is H, methyl, halo, carboxy, esterified carboxy, amidated carboxy, carboxymethyl, esterified carboxymethyl or amidated carboxymethyl,
R₂ is selected from the groups recited above for R₁; or, when Z is absent and W is H, R₂ may additionally -C(O)-Z' wherein Z' is selected from the groups recited above for Z; or R₁ and R₂ together form a second bond between the carbon atoms to which they are attached,
R₃ and R₄ (or each R₃ and R₄ group, when m or n is 2 or more) are independently selected from halo, amino, nitro, cyano, sulphamoyl, C₁ to C₃ alkyl, C₁ to C₃ alkoxy, carboxy, esterified carboxy and amidated carboxy
R₅ and R₆ are independently selected from H and the groups recited above for R₃
m is from 0 to 4, provided that m is not more than 2 unless R₃ is exclusively halo,
n is from 0 to 4, provided that n is not more than 2 unless R₄ is exclusively halo,
or pharmaceutically acceptable salts thereof.

A number of compounds falling within the above definition are known from US-A-3950407; Weber *et al*, J. Org. Chem. 53, 5831-9 (1988); Czugler *et al*, J. Chem. Soc., Chem. Commun. (23), 1632-4 (1984); Russell *et al*, J. Am. Chem. Soc. 94(5), 1693-8 (1972); Chem. Abs. 73, no. 3689z (1970); and Singh *et al*, Ind. J. Chem. 23B, 631-4 (1984). However, compounds according to the above definition are believed to be novel *per se*, provided that
if one (but only one) of R₁ and R₂ is methyl, m and n are not both 0,
Z is not hydroxy or methoxy when Y is hydroxy or methoxy,
Z and Y are not *trans* to each other when Z is R₉-O- and Y is R₇-O- ,
-X-Y does not equal -W-Z when R₁=R₂=H and m=n=0, and
if Z is absent and R₁ and R₂ are both H, Y is not R₇-O-
and further provided that the compounds are not 7-(N,N-dimethylaminocarbonyl) -8-methyl-2,3,5,6-dibenzobicyclo[2.2.2]octane or 7-(N-methyl-N-phenylaminocarbonyl)-8-methyl-2,3,5,6-dibenzobicyclo[2.2.2]octane.

CCK inhibitors of different structures are disclosed in EP-A-0405537 and US-A-4 306 063.

The term "hydrocarbyl", as used herein, refers to monovalent groups consisting of carbon and hydrogen. Hydrocarbyl groups thus include alkyl, alkenyl, and alkynyl groups (in both straight and branched chain forms), cycloalkyl (including polycycloalkyl), cycloalkenyl, and aryl groups, and combinations of the foregoing, such as alkylaryl, alkenylaryl, alkynylaryl, cycloalkylaryl, and cycloalkenylaryl groups,

A "carbocyclic" group, as the term is used herein, comprises one or more closed chains or rings, which consist entirely of carbon atoms. Included in such groups are alicyclic groups (such as cyclopropyl, cyclobutyl, cyclopentyl, cyclohexyl and adamantyl), groups containing both alkyl and cycloalkyl moieties (such as methyl adamantyl), and aromatic groups (such as phenyl, naphthyl, indanyl, fluorenyl, (1,2,3,4)-tetrahydronaphthyl, indenyl and isoindenyl).

The term "aryl" is used herein to refer to aromatic carbocyclic groups, including those mentioned above.

A "heterocyclic" group comprises one or more closed chains or rings which have at least one atom other than carbon in the closed chain or ring. Examples include thienyl, furanyl, pyrrolyl, imidazolyl, pyrazolyl, thiazolyl, isothiazolyl, oxazolyl, pyrrolidinyl, pyrrolinyl, imidazolidinyl, imidazolinyl, pyrazolidinyl, tetrahydrofuranyl, pyranyl, pyronyl, pyridyl, pyrazinyl, pyridazinyl, piperidyl, piperazinyl, morpholinyl, thionaphthyl, benzofuranyl, isobenzofuryl, indolyl, oxyindolyl, isoindolyl, indazolyl, indolinyl, 7-azaindolyl, isoindazolyl, benzopyranyl, coumarinyl, isocoumarinyl, quinolyl, isoquinolyl, naphthridinyl, cinnolinyl, quinazolinyl, pyridopyridyl, benzoxazinyl, quinoxadinyl, chromenyl, chromanyl, isochromanyl and carbolinyl.

The term "halogen", as used herein, refers to any of fluorine, chlorine, bromine and iodine. Most usually, however, halogen substituents in the compounds of the invention are chlorine or fluorine substituents.

Preferably, m and n are both 0. However, when m and n are not both 0, R₃ and R₄ are preferably selected from halo, amino, nitro, cyano, sulphamoyl, C₁ to C₃ alkyl and C₁ to C₃ alkoxy. As mentioned above, when m or n is 2 or more, each R₃ and R₄ group is independent of the others. For example, the compounds of the invention may include two different R₃ groups.

Particularly preferred groups for R₅ and R₆ are hydrogen and the groups just recited for R₃, and especially hydrogen, methyl and fluoro.

When reference is made herein to a "substituted" aromatic group, the substituents will generally be from 1 to 3 in number (and more usually 1 or 2 in number), and selected from the groups recited above for R₃.

An "esterified" carboxy group, as the term is used herein, is preferably of the form -COOR₁₆, wherein R₁₆ is C₁ to C₅ alkyl, phenyl, substituted phenyl, benzyl, substituted benzyl, or one of the following:

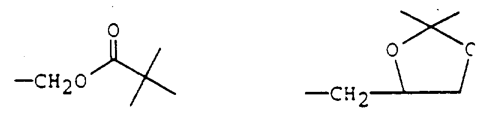

Most commonly, R₁₆ is C₁ to C₅ alkyl, benzyl or substituted benzyl, and particularly C₁ to C₅ alkyl. Similarly, an "amidated" carboxy group is preferably of the form -CONR₁₇R₁₈ wherein R₁₇ and R₁₈ are independently H, C₁ to C₅ alkyl, phenyl, substituted phenyl, benzyl or substituted benzyl.

In the case of the group T, preferred amidated carboxy groups take the form -CONR₁₇R₁₈ (wherein R₁₇ and R₁₈ are as defined above) or 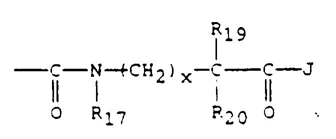 wherein R₁₇ is as defined above, R₁₉ and R₂₀ are independently H or methyl, or R₁₉ and R₂₀ (together with the carbon atom to which they are attached) form a 3- to 7-membered carbocyclic group, J is -OH, -O-R₁₆ or -NHR₁₈, wherein R₁₆ and R₁₈ are as defined above, and x is 0 to 3.

When R₁₁ and R₁₂ are linked to form a ring, such ring will generally be saturated, and usually also carbocyclic. Similarly, when Q' and R₁₂ are linked to form a ring, this will also usually be saturated and carbocyclic.

Exemplary carbocyclic and heterocyclic groups which may form the group U include: 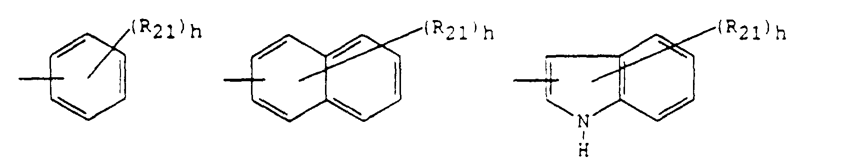 wherein R₂₁ is as defined above for R₃, and h is from 0 to 3, and 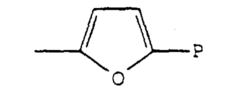 wherein P is H or -COOR₂₂, in which R₂₂ is as defined above for R₁₇.

Z is preferably -NH₂, -O-R₉ or 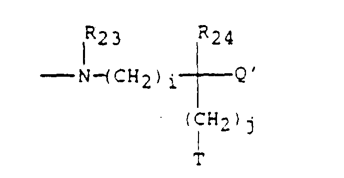 wherein i is from 0 to 4, j is from 0 to 3, R₂₃ and R₂₄ are independently H or methyl, or R₂₃ and R₂₄ together form a group of the formula -(CH₂)ₖ-V'-CH₂- (wherein V' is -CH₂-, -CHOH- or -C(O)-, and k is 0 to 2). Most commonly, i is 0 or 1 and j is 0 to 2.

When W is sulphinyl, Y is preferably R₂-NH-.

Preferably, R₇ is C₆ to C₈ straight or branched chain alkyl, or R₂₅-(CH₂)ₚ-, wherein R₂₅ is selected from phenyl, 1-naphthyl, 2-naphthyl, indolyl, norbornyl, adamantyl or cyclohexyl, and p is from 0 to 3.

Pharmaceutically acceptable salts of the acidic compounds of the invention include salts with alkali metals and alkaline earth metals, such as sodium, potassium, calcium and magnesium, and salts with organic bases. Suitable organic bases include amines such as N-methyl-D-glucamine.

Pharmaceutically acceptable salts of the basic compounds of the invention include salts derived from organic or inorganic acids. Suitable acids include hydrochloric acid, phosphoric acid, oxalic acid, maleic acid, succinic acid and citric acid.

The compounds of the invention exist in various enantiomeric and diastereomeric forms as a result of the asymmetric carbon atoms to which W and X are attached. It will be understood that the invention comprehends the different enantiomers and diastereomers in isolation from each other, as well as mixtures of enantiomers and diastereomers. Also, the structural formulae herein show the groups W and X arranged *cis* to each other, but it will be appreciated that the invention includes the corresponding *trans* isomers.

Compounds according to the present invention in which W is a carbonyl group, X is carbonyl or sulphonyl, and Z is OH may conveniently be made by the process depicted in Reaction Scheme A.

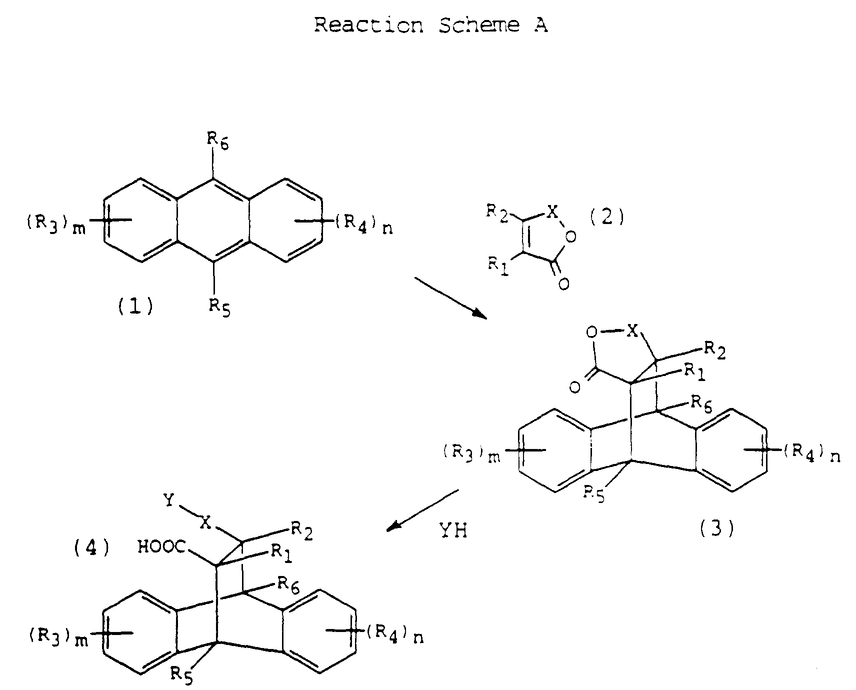

In this scheme, anthracene or an anthracene derivative (1) is reacted with the acid anhydride (2) in a Diels-Alder reaction. The reactants are conveniently refluxed together in a suitable solvent such as toluene to form the adduct (3). In some cases, it may be appropriate to conduct the reaction at elevated pressure and/or in the presence of a Lewis acid catalyst. The adduct (3) is then reacted with a compound of the formula YH (ie. either an alcohol or an amine) to form the acid compound (4). If YH is an amine, the reaction is suitably carried out in a solvent such as THF in the presence of a catalytic amount of DMAP. If YH is an alcohol, the reaction may be conducted in pyridine at elevated temperature.

The invention therefore also provides a method of making compounds wherein W is carbonyl and X is carbonyl or sulphonyl, said method including the step of reacting a compound of the formula 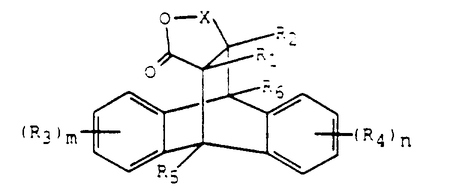 with a compound of formula YH.

The equivalent *trans* adducts can be prepared using a suitably differentiated fumaric acid (eg the mono methyl mono benzyl diester), which, after addition to anthracene or an anthracene derivative (1), allows independent elaboration of the two sidechains.

Compounds in which Z is other than OH may of course be made from the acid compound (4) by conventional esterification or amidation reactions. Suitable amidation methods are described in detail in "The Peptides, Vol. 1", Gross and Meinenhofer, Eds., Academic Press, N.Y., 1979. These include the carbodiimide method (using, for example, 1,3-dicyclohexylcarbodiimide [DCC] or 1-(3-dimethylaminopropyl)-3-ethylcarbodiimide hydrochloride [EDCI], and optionally an additive such as 1-hydroxybenzotriazole [HOBT] to prevent racemization), the azide method, the mixed anhydride method, the symmetrical anhydride method, the acid chloride method, the use of bis (2-oxo-3-oxazolidinyl) phosphinic chloride [BOP-C1], the use of PyBOP, the use of the isopropenylsuccinimido carbonate method and the active ester method (using, for example, N-hydroxysuccinimide esters, 4-nitrophenyl esters or 2,4,5-trichlorophenol esters).

The coupling reactions are generally conducted under an inert atmosphere, such as an atmosphere of nitrogen or argon. Suitable solvents for the reactants include methylene chloride, tetrahydrofuran [THF], dimethoxyethane [DME] and dimethylformamide [DMF].

A procedure analogous to that shown in reaction scheme A may also be used as the basis for preparing the compounds of the invention in which W is sulphonyl and Y is R₇-O-, as depicted in reaction scheme B below:

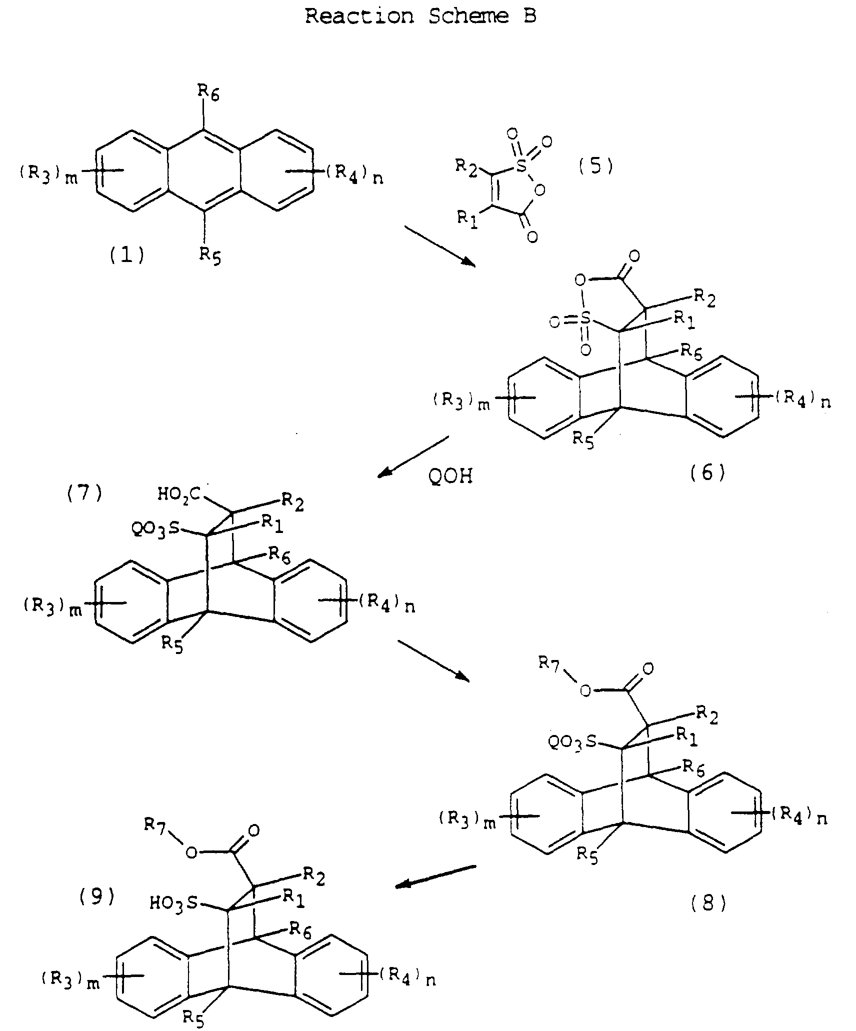

In this case, the Diels-Alder adduct (6) is opened with an alcohol such as benzyl alcohol (represented as QOH), so that product (7) is the corresponding sulphonyl ester. The free carboxylic acid group of this sulphonyl ester may then be esterified by conventional methods, followed by hydrogenolysis of the product (8) to yield the desired sulphonic acid carboxylic ester (9).

The compounds of the invention in which W is sulphonyl and Y is R₇-NH- may be prepared by analogous means, in which compound (7) is amidated (rather than esterified) prior to hydrogenolysis. Alternatively, a process such as is depicted in reaction scheme C may be employed:

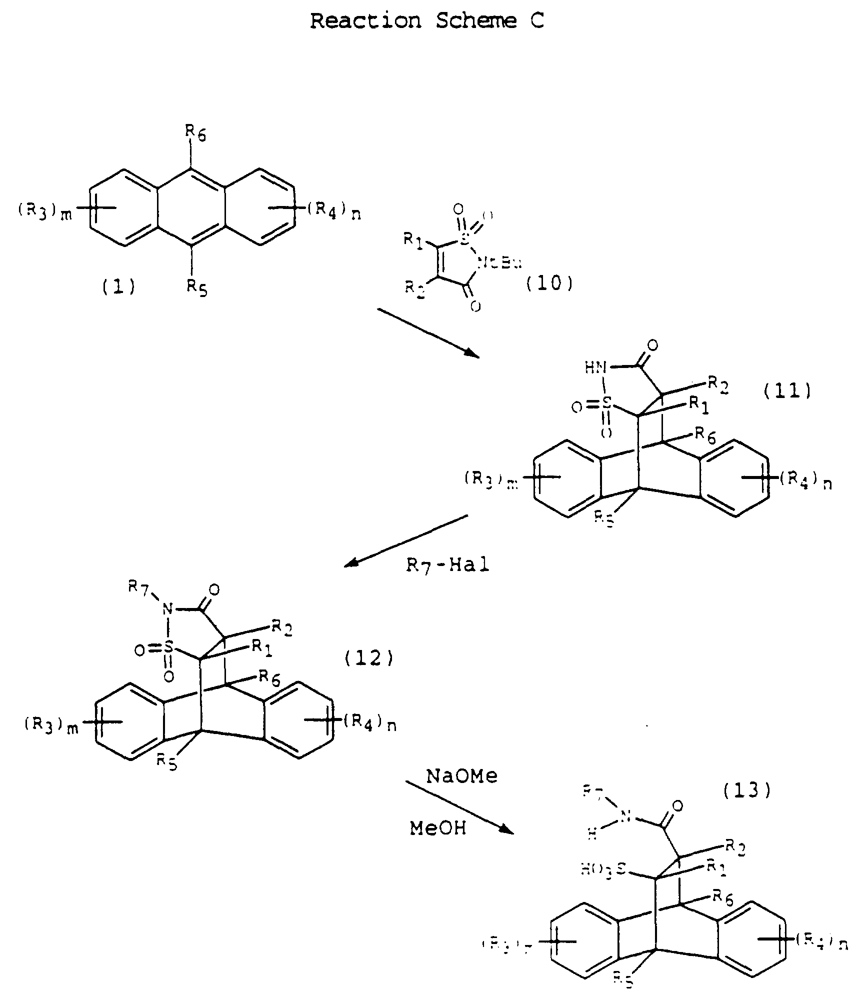

In this scheme, anthracene or an anthracene derivative (1) is reacted with the N-protected compound (10) in a Diels-Alder reaction analogous to that of the first step in reaction scheme A.

The deprotected product Diels-Alder adduct (11) is then reacted with a compound of the formula R₇-Hal (wherein Hal represents a halogen atom) to form compound (12). The N-containing ring may then be opened using an alkoxide (eg. sodium methoxide in methanol) to produce the target compound (13).

The invention therefore also provides a method of making compounds wherein W is sulphonyl and Y is R₇-NH-, said method comprising the step of reacting a compound of the formula 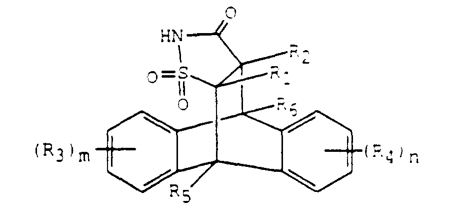 with a compound of the formula R₇-Hal, and then reacting the product with an alkoxide.

Compounds of the invention wherein W or X is a sulphinyl group may conveniently be prepared by the route shown in reaction scheme D:

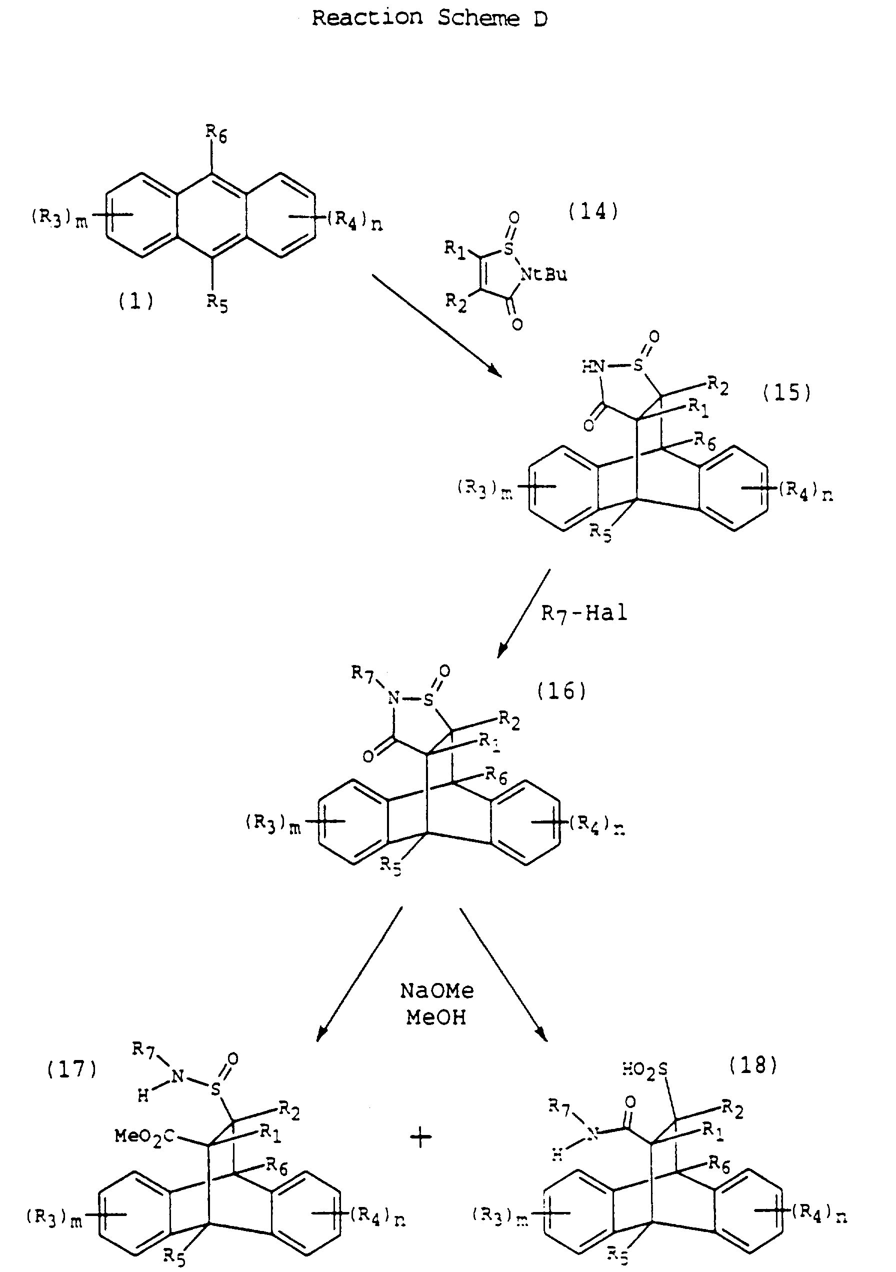

Reaction scheme D is analogous to reaction scheme C, except that the sulphinyl analogue of compound (10) is used in the Diels-Alder reaction, to yield the sulphinyl analogue of adduct (12). This can then be opened both ways to give on the one hand the sulphinamide acid alkyl ester (17), and on the other the sulphinic acid amide (18). The free sulphinamide acid can of course be obtained from the alkyl ester (12) by conventional methods.

Accordingly, the invention also provides a method of making compounds wherein W or X is sulphinyl, said method comprising the step of reacting a compound of the formula: 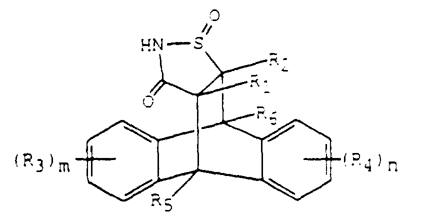 with a compound of the formula R₇-Hal, and then reacting the product with an alkoxide.

While reaction schemes C and D above lead to the free sulphonic or sulphinic acid compounds, it will be appreciated that the corresponding ester or amide derivatives can be prepared from the free acid compounds by conventional methods. Most usually, coupling of the sulphonic or sulphinic acid compounds will be *via* the corresponding sulphonic or sulphinic acid chlorides.

pharmaceutically acceptable salts of the acidic or basic compounds of the invention can of course be made by conventional procedures, such as by reacting the free base or acid with at least a stoichiometric amount of the desired salt-forming acid or base.

The compounds of the invention can be administered by oral or parenteral routes, including intravenous, intramuscular, intraperitoneal, subcutaneous, rectal and topical administration.

For oral administration, the compounds of the invention will generally be provided in the form of tablets or capsules or as an aqueous solution or suspension.

Tablets for oral use may include the active ingredient mixed with pharmaceutically acceptable excipients such as inert diluents, disintegrating agents, binding agents, lubricating agents, sweetening agents, flavouring agents, colouring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

Capsules for oral use include hard gelatin capsules in which the active ingredient is mixed with a solid diluent, and soft gelatin capsules wherein the active ingredient is mixed with water or an oil such as peanut oil, liquid paraffin or olive oil.

For intramuscular, intraperitoneal, subcutaneous and intravenous use, the compounds of the invention will generally be provided in sterile aqueous solutions or suspensions, buffered to an appropriate pH and isotonicity. Suitable aqueous vehicles include Ringer's solution and isotonic sodium chloride. Aqueous suspensions according to the invention may include suspending agents such as cellulose derivatives, sodium alginate, polyvinylpyrrolidone and gum tragacanth, and a wetting agent such as lecithin. Suitable preservatives for aqueous suspensions include ethyl and n-propyl p-hydroxybenzoate.

The invention is now further illustrated by means of the following examples.

### Example 1 Preparation of (±)-cis-8-(3-phenylpropylaminocarbonyl)-2,3,5,6-dibenzobicyclo[2.2.2]octane-7-carboxylic acid

### a. 2,3,5,6-dibenzobicyclo[2.2.2]octane-7,8-dicarboxylic acid anhydride

Anthracene (8.9 g, 0.05 mol) and maleic anhydride (4.9 g, 0.05 mol) were refluxed for 3h in toluene (200 ml). Upon cooling, the title compound was obtained as white crystals which were isolated by filtration (10.2g 74%).

### b. (±)-cis-8-(3-phenylpropylaminocarbonyl)-2,3,5,6-dibenzobicyclo[2.2.2]octane-7-carboxylic acid

2,3,5, 6-dibenzobicyclo[2.2.2]octane-7,8-dicarboxylic acid anhydride (107 mg, 0.39 mmol) and 1-phenyl-3-propylamine (55 mg, 0.4 mmol) were dissolved in dry THF (5 ml) and DMAP (2 mg) was introduced. The mixture was stirred at room temperature overnight during which time a thick white precipitate formed. The solid was filtered off, washed with THF and dried to give the title compound (100 mg 62%), mp 190-1°, found: C, 78.82; H, 5.99; N, 3.40. C₂₇H₂₅NO₃ requires C, 78.81; H, 6.12; N, 3.38%

### Example 2 Preparation of (±)-cis-8-(2-(3-indolyl)ethylaminocarbonyl)-2,3,5,6-dibenzobicyclo[2.2.2]octane-7-carboxylic acid

The compound was prepared essentially as in example 1 except that tryptamine was used instead of 1-phenyl-3-propylamine in step b. Yield 84%, m.p. 137-8°, found: C, 74.13; H, 6.12; N, 5.73. C₂₈H₂₄N₂O₃. 0.7 H₂O. 0.6 THF requires C, 74.16; H, 6.18; N, 5.69%

### Example 3 Preparation of (±)-cis-8-(phenylmethylaminocarbonyl)-2,3,5,6-dibenzobicyclo[2.2.2]octane-7-carboxylic acid

The compound was prepared essentially as in example 1 except that benzylamine was used instead of 1-phenyl-3-propylamine in step b. Yield 27%, m.p. 194-5°, found: C, 77.35; H, 5.97; N, 3.36. C₂₅H₂₁NO₃. 0.5 THF requires C, 77.30; H, 6.18; N, 3.34%

### Example 4 Preparation of (±)-cis-8-(1-naphthylmethylaminocarbonyl)-2,3,5,6-dibenzobicyclo[2.2.2]octane-7-carboxylic acid

The compound was prepared essentially as in example 1 except that 1-naphthylmethylamine was used instead of 1-phenyl-3-propylamine in step b. Yield 35%, m.p. 135-7°, found: C, 78.37; H, 6.07; N, 2.98. C₂₉H₂₃NO₃. 1.0 THF requires C, 78.39; H, 6.18; N, 2.77%

### Example 5 Preparation of (±)-cis-8-(2-naphthylmethylaminocarbonyl)-2,3,5,6-dibenzobicyclo[2.2.2]octane-7-carboxylic acid

The compound was prepared essentially as in example 1 except that 2-naphthylmethylamine was used instead of 1-phenyl-3-propylamine in step b. Yield 35%, m.p. 247-8°, found: C, 80.46; H, 5.03; N, 3.32. C₂₉H₂₃NO₃ requires C, 80.46; H, 5.03; N, 3.23%

### Example 6 Preparation of (±)-cis-8-(2-norbornylmethylaminocarbonyl)-2,3,5,6-dibenzobicyclo(2.2.2]octane-7-carboxylic acid

The compound was prepared essentially as in example 1 except that 2-norbornylmethylamine was used instead of 1-phenyl-3-propylamine in step b. Yield 24%, m.p. 127-9°, found: C, 74.93; H, 7.07; N, 3.76. C₂₆H₂₇NO₃. 0.75 H₂O requires C, 75.24; H, 6.92; N, 3.38%

### Example 7 Preparation of (±)-cis-8-(hexylaminocarbonyl)-2,3,5,6-dibenzobicyclo[2.2.2]octane-7-carboxylic acid

The compound was prepared essentially as in example 1 except that hexylamine (2eq) was used instead of 1-phenyl-3-propylamine in step b. and the product was precipitated with 2M HCl and then filtered and washed with water. Yield 91%, m.p. 174-6°, found: C, 76.52; H, 7.24; N, 3.98. C₂₄H₂₇NO₃ requires C, 76.36; H, 7.21; N, 3.71%

### Example 8 Preparation of (±)-cis-8-(octylaminocarbonyl)-2,3,5,6-dibenzobicyclo[2.2.2]octane-7-carboxylic acid

The compound was prepared essentially as in example 7 except that octylamine was used instead of hexylamine. Yield 89%, m.p. 116-8°, found: C, 76.83; H, 7.70; N, 3.58. C₂₆H₃₁NO₃ requires C, 77.01; H, 7.71; N, 3.45%

### Example 9 Preparation of (±)-cis-8-(cyclohexylmethylaminocarbonyl)-2,3,5,6-dibenzobicyclo[2.2.2]octane-7-carbocylic acid

The compound was prepared essentially as in example 7 except that cyclohexylmethylamine was used instead of hexylamine. Yield 93%, m.p. 185-7°, found: C, 76.88; h, 7.09; N, 3.69. C₂₅H₂₇NO₃ requires C, 77.09; H, 6.99; N, 3.60%

### Example 10 Preparation of (±)-cis-8-(3,3-dimethylbutylaminocarbonyl)-2,3,5,6-dibenzobicyclo[2.2.2]octane-7-carboxylic acid

The compound was prepared essentially as in example 7 except that 3,3-dimethylbutylamine was used instead of hexylamine. Yield 25%, m.p. 128-30°, found: C, 75.56; H, 7.22; N, 3.92. C₂₄H₂₇NO₃.0.2 H₂O requires C, 75.64; H, 7.25; N, 3.68%

### Example 11 Preparation of (±)-cis-8-(1-adamantylaminocarbonyl)-2,3,5,6-dibenzobicyclo[2.2.2]octane-7-carboxylic acid

2,3,5,6-dibenzobicyclo[2.2.2]octane-7,8-dicarboxylic acid anhydride (prepared in example 1 step a) (276 mg, 1.0 mmol) was dissolved in THF (5 ml) and 1-adamantamine ( 215 mg, 1.4 mmole) was added followed by triethylamine (0.16 ml). The solution was heated at a gentle reflux for 1.5 h and the clear solution on cooling was poured onto 2M HCl (20 ml). The resulting gummy solid was extracted with dichloromethane (10 ml) and the organic layer was dried, filtered and evaporated. The residue was taken up in methanol (5 ml) and diluted with water (5 ml) to precipitate a white solid.

This was filtered off and dried. The product (230 mg, 54%), m.p. 234-5°, found: C, 76.14; H, 6.72; N, 3.10. C₂₈H₂₉NO₃.0.75 H₂O requires C, 76.25; H, 6.97; N, 3.17%

### Example 12 Preparation of (±)-cis-8-(2-(1-adamantyl)ethylaminocarbonyl)-2,3,5,6-dibenzobicyclo[2.2.2]octane-7-carboxylic acid

The compound was prepared essentially as in example 11 except that 1-adamantylethylamine was used instead of 1-adamantylamine. Yield 26%, m.p. 138-40°, found: C, 77.31; H, 7.21; N, 2.70. C₃₀H₃₃NO₃.0.6 H₂O requires C, 77.26; H, 7.39; N, 3.00%

### Example 13 Preparation of (±)-cis-8-(-1-adamantylmethyloxycarbonyl)-2,3,5,6-dibenzobicyclo[2.2.2]octane-7-carboxylic acid

2,3,5,6-dibenzobicyclo[2.2.2]octane-7,8-dicarboxylic acid anhydride (prepared in example 1 step a) (276 mg, 1.0 mmol) and 1-adamantanemethanol (166 mg, 1.0 mmol) were heated together in pyridine (2 ml) at 100° for 4 h. After cooling the solution was poured onto 2M HCl and extracted with dichloromethane (20 ml). The solution was dried filtered and evaporated to leave a white residue which was further purified by column chromatography (silica dichloromethane\ethyl acetate\methanol 9:1:0.5 as eluent). The product was further triturated with hexane to leave the title compound (110 mg, 25%), m.p. 165°, found: C, 77.14; H, 6.88. C₂₉H₃₀O₄.0.5 H₂O requires C, 77.13; H, 6.91%

### Example 14 Preparation of (±)-cis-8-(1-adamantylmethylaminocarbonyl)-2,3,5,6-dibenzobicyclo(2.2.2]octane-7-carboxylic acid

2,3,5,6-dibenzobicyclo[2.2.2]octane-7,8-dicarboxylic acid anhydride (prepared in example 1 step a) (276 mg, 1.0 mmol) and 1-adamantanemethylamine (182 mg, 1.1 mmol) were dissolved in dry THF (5 ml) and refluxed for 1h. A thick white precipitate was formed and this was isolated by filtration and washed with THF to leave the title compound (320 mg, 72%), m.p. 237-9°, found: C,78.76; H, 7.18; N, 3.33. C₂₉H₃₁NO₃ requires C, 78.88; H, 7.08; N, 3.17% The compound was further characterised as the N-methyl-D-glucamine salt found: C, 63.48; H, 7.61; N, 3.79. C₃₆H₄₈N₂O₈. 2.5H₂O requires C, 63.42; H, 7.83; N, 4.11%

### Example 15 Preparation of (±)-cis-7-(methoxycarbonylmethylaminocarbonyl)-8-(1-adamantylmethylaminocarbonyl)-2,3,5,6-dibenzobicyclo[2.2.2]octane.

(±)-cis-8-(1-adamantylmethylaminocarbonyl)-2,3,5,6-dibenzobicyclo-[2.2.2]octane-7-carboxylic acid prepared as in example 14 (441 mg, 1.0 mmol) was dissolved in warm DMF (5 ml) and then the solution was cooled to 0°. N-hydroxysuccinimide (115 mg, 1.0 mmol) was added followed by DCCI (206 mg, 1.0 mmol). The reaction was allowed to warm to room temperature and stirred overnight. The white precipitate was removed by filtration. Triethylamine (0.2 ml) was added to the filtrate followed by glycine methyl ester hydrochloride (125 mg, 1 mmol) and the reaction mixture was stirred for a further 24h. The reaction mixture was poured onto a mixture of 2M HCl and ice. The white precipitate was isolated by filtration and washed well with water and dried. The solid was taken up in ethyl acetate (20 ml) and filtered through celite. The residue on evaporation was triturated with methanol leaving a white crystalline solid (125 mg, 24%), m.p. 209-11°, found: C,74.16; H, 7.14; N, 5.53 C₃₂H₃₆N₂O₄ requires C, 74.19; H, 7.12; N, 5.40%

### Example 16 Preparation of (±)-cis-7-(carboxymethylaminocarbonyl)-8-(1-adamantylmethylaminocarbonyl)-2,3,5,6-dibenzobicyclo[2.2.2]octane

### a. (±)-cis-7-(benzyloxycarbonylmethylaminocarbonyl)-8-(1-adamantylmethylaminocarbonyl)-2,3,5,6-dibenzobicyclo-[2.2.2]octane

(±)-Cis-8-(1-adamantylmethylaminocarbonyl)-2,3,5,6-dibenzobicyclo[2.2.2]octane-7-carboxyl acid (prepared as in example 14) (440 mg, 1 mmole) and PyBOP (520 mg, 1 mmole) were taken up in dry dichloromethane (15 ml) and Hunigs base (0.52 ml, 3 mmole) was added. The reaction mixture was stirred under an atmosphere of dry argon for 1h. glycine benzyl ester 4-toluenesulphonic acid salt (340 mg, 1 mmole) was added and the mixture stirred overnight. The organic layer was washed with 5% potassium hydrogensulphate (15 ml), sodium hydrogencarbonate (15 ml) and saturated brine (15 ml). It was then dried, filtered and evaporated to leave the crude title compound which was further purified by column chromatography on silica using 80% ethyl acetate and 20% hexane as eluent. The title compound (510 mg, 87%) was isolated as a white solid, m.p. 130-3°

### b. (±)-cis-7-(carboxymethylaminocarbonyl)-8-(1-adamantylmethylaminocarbonyl)-2,3,5,6-dibenzobicyclo[2.2.2]octane

The product of step a (350 mg, 0.59 mmole) was dissolved in methanol (20 ml) and 10% palladium on charcoal (100 mg) was added. The mixture was stirred under an atmosphere of hydrogen for 3h. The product was filtered through celite and on evaporation yielded the title compound ( 0.30 g, 100%). The product was characterised and tested as the N-methyl-D-glucamine salt, m.p. 110-2°, found: C, 63.42; H, 7.55; N, 5.66. C₃₈H₅₁N₃O₉. 1.5 H₂O requires C, 63.42; H, 7.68; N, 5.66%.

### Example 17 Preparation of methyl (±)-cis-8-(1-adamantylmethylaminocarbonyl)-2,3,5,6-dibenzobicyclo[2.2.2]octane-7-carboxylate

2,3,5,6-dibenzobicyclo[2.2.2]octane-7,8-dicarboxylic acid anhydride (prepared as in example 1 step a) (2.76 g, 6.0 mmol), methanol (0.41 ml) and DMAP (20 mg) were stirred in pyridine (5 ml). The solution was stirred and refluxed for 4h, poured onto 2M HCl (50 ml) and extracted with dichloromethane. The organic layer was washed with mor 2M HCl and then water. The solution was dried filtered and evaporated to yield the crude monoester (1.7 g) . This material (308 mg, 1mmol) and 1-adamantanemethylamine (181 mg, 1.0 mmol) were dissolved in dry dichloromethane (10 ml) and diisopropylethylamine (0.35 ml) was added followed by PyBOP (520 mg, 1 mmol). The solution was stirred at room temperature for 72h. It was then evaporated and the residue taken up in ethyl acetate and washed successively with 5% aqueous potassium hydrogensulphate (3x40 ml), saturated aqueous sodium hydrogencarbonate (40 ml) and brine (40 ml). The organic layer was dried, filtered and evaporated to leave a foam that was purified by column chromatography (silica eluent 90% dichloromethane and 10% ethyl acetate). Further purification was achieved by recrystallisation from methanol. Yield 200 mg, 44%, m.p. 227-30°, found: C,79.06; H, 7.54; N, 2.94. C₃₀H₃₃NO₃ requires C, 79.09; H, 7.30; N, 3.07%

### Example 18 Preparation of (±)-cis-8-(2-naphthylmethylaminocarbonyl)-2,3,5,6-dibenzobicyclo[2.2.2]octane-7-sulphonic acid

### a. Diels-Alder adduct of anthracene and 3-oxo-2,3-dihydroisothiazolone

N-t-butyl-3-oxo-2,3-dihydroisothiazolone ( prepared as in Helv.Chim. Acta., 1989, 72, 1416) (200 mg, 1.1 mmol) and anthracene (178 mg, 1 mmol) were suspended in dry toluene, (2 ml) and a catalytic amount of anhydrous aluminium chloride was added. The reaction mixture was stirred and refluxed overnight. On cooling a white solid separated which was filtered and washed successively with toluene and pentane and air dried. The solid was then taken up in ethyl acetate and washed with dilute HCl and brine and finally dried and evaporated to leave a white solid (185 mg, 62%)

### b. Alkylation of the Diels-Alder adduct

The product from step a (312 mg, 1 mmol), anhydrous potassium carbonate (138 mg, 1 mmol) and 2-bromomethylnaphthalene (225 mg, 1 mmol) were dissolved in dry DMF (3 ml) and stirred and heated to 100° for 4h. After cooling the solution was poured onto cold water (30 ml) and the resulting white solid filtered off and dried in an oven. The solid was triturated with hexane/toluene/ethanol 9:9:2 and the solid was recrystallised from ethanol (178 mg, 39%), m.p. 184-5°

### c. 8-(2-naphttylmethylaminocarbonyl)-2,3,5,6-dibenzobicyclo-[2.2.2]octane-7-sulphonic acid

Sodium (16 mg, 0.7 mmol) was dissolved in methanol (5 ml) and the product from step b was added (225 mg, 0.5 mmol). The reaction was stirred and refluxed for lh. The reaction mixture was cooled and acidified with concentrated HCl. The reaction mixture was then evaporated and the residue partitioned between water and ethyl acetate. The organic layer was dried and evaporated. The product was recrystallised from chloroform. Yield 152 mg, 32%, m.p. 245-7° found: C, 68.74; H, 4.88; N, 2.83. C₂₈,H₂₃NO₄S, 1.0 H₂O requires C, 68.97; H, 5.16; N, 2.87%

### Example 19 Preparation of (±)-cis-8-(phenylmethylaminocarbonyl)-2,3,5,6-dibenzobicyclo[2.2.2]octane-7-sulphonic acid

This was prepared essentially as in example 18 using benzyl bromide in step b instead of 2-bromomethylnaphthalene m.p. 245-7° found: C, 68.764; H, 4.81; N, 3.19. C₂₄H₂₁NO₄S requires C, 68.72; H, 5.05; N, 3.34%

### Example 20 Preparation of (±)-cis-8-(2-naphthylmethylaminosulphonyl)-2,3,5,6-dibenzobicyclo[2.2.2]octane-7-carboxylic acid

### a. Diels-Alder adduct of anthracene and β-sulphoacrylic anhydride

β-sulphoacrylic anhydride (prepared as in J.A.C.S. 1962, 84, 653) (184 mg, 1.0 mmol) and anthracene (178 mg, 1mmol) were suspended in dry toluene (6 ml) and refluxed under an atmosphere of dry nitrogen for 3h. The reaction mixture was decanted from a small amount of tarry residue and cooled in ice. White crystals that separated were filtered off and wasned with a little hexane and dried. Yield 163 mg, 52%

### b. (±)-cis-8-(2-naphthylmethylaminosulphonyl)-2,3,5,6-dibenzobicyclo[2.2.2]octane-7-carboxylic acid

The Adduct from step a (207 mg, 0.66 mmole) and 2-naphthylmethylamine (209 mg, 1.33 mmol) were dissolved in dry THF (5 ml) and DMAP (5 mg) was added. The solution was stirred at room temperature overnight and evaporated to dryness. The residue was taken up in methanol and water and stirred with Amberlite IR-120(plus) resin, filtered and evaporated. The residue was triturated with ether, to yield the title compound 215 mg, 68% m.p. 212-15 found: C, 69.78; H, 4.98; N, 2.98. C₂₈H₂₃NO₄S. 0.7 H₂O requires C, 69.75; H, 5.10; N, 2.91%

### Example 21 Preparation of (±)-cis-8-(2-(3-indolyl)ethylaminosulphonyl)-2,3,5,6-dibenzobicyclo[2.2.2]octane-7-carboxylic acid

This was prepared essentially as in example 20 but using tryptamine instead of 2-naphthylmethylamine in step b, m.p. >220° found: C, 68.87; H, 5.17; N, 6.07. C₂₇H₂₄N₂O₄S requires C, 68.63; H, 5.12; N, 5.92%

### Example 22 Preparation of (±)-cis-8-(1-adamantylmethylaminosulphonyl)-2,3,5,6-dibenzobicyclo[2.2.2]octane-7-carboxylic acid

This was prepared essentially as in example 21 but using 1-adamantylmethylamine instead of 2-naphthylmethylamine in step b, m.p. 135-40° found: C, 69.90; H, 6.49; N, 2.68. C₂₈H₃₁NO₄S. 0.2H₂O requires C, 69.89; H, 6.58; N, 2.91%

### Example 23 Preparation of cis-7-(1-R-carboxy-ethylaminocarbonyl)-8-(1-adamantylmethylaminocarbonyl)-2,3,5,6-dibenzobicyclo-[2.2.2]octane (mixture of diastereomers)

(±)-cis-8-(1-adamantylmethylaminocarbonyl)-2,3,5,6-dibenzobicyclo[2.2.2]octane-7-carboxylic acid (prepared as in example 14) (440 mg, 1 mmole) was dissolved by warming in dry DMF (10 ml). Isopropenylsuccinimido carbonate (200 mg, 1 mmole) was then added at room temperature. A catalytic amount of DMAP was added and the reagents stirred for 4h. Triethylamine (0.168 ml, 1.2 mmole) was added followed by D-alanine (100 mg, 1.1 mmole) and the reaction left to stir at room temperature for 60h. The reaction mixture was poured onto 2N HCl and the white precipitate so formed was isolated by filtration. The solid was further purified by column chromatography (silica, dichloromethane to 90% dichloromethane and 10% methanol) to leave the title compound (50 mg). The compound was characterised and tested as the N-methyl-D-glucamine salt m.p 128-30°, found: C, 61.98; H, 7.42; N, 5.85. C₃₉H₅₃N₃O₉. 2.4H₂O requires C, 62.32; H, 7.75; N, 5.59%

### Example 24 Preparation of cis-(±)-7-(2-methoxycarbonylethylaminocarbonyl)-8-(1-adamantylmethylaminocarbonyl)-2,3,5,6-dibenzobicyclo[2.2.2]octane

This was prepared essentially as in example 23 using beta-alanine methyl ester instead of D-alanine, m.p. 207°, found: C, 74.98; H, 7.46; N, 5.27. C₃₃H₃₈N₂O₄ requires C, 75.26; H, 7.27; N, 5.32%

### Example 25 Preparation of cis-7-(1-S-methoxycarbonyl-ethylaminocarbonyl)-8-(1-adamantylmethylaminocarbonyl)-2,3,5,6-dibenzobicyclo[2.2.2]octane (mixture of diastereomers)

(±)-cis-8-(1-adamantylmethylaminocarbonyl)-2,3,5,6-dibenzobicyclo[2.2.2]octane-7-carboxylic acid (prepared as in example 14) (440 mg, 1 mmole) and PyBOP (520 mg, 1 mmole) were taken up in dry dichloromethane (15 ml) and Hunigs base (0.52 ml, 3 mmole) was added. The reaction mixture was stirred under an atmosphere of dry argon for lh. L-alanine methyl ester hydrochloride (140 mg, 1 mmole) was added and the mixture stirred overnight. The organic layer was washed with 5% potassium hydrogensulphate (15 ml), sodium hydrogencarbonate (15 ml) and saturated brine (15 ml) . It was then dried, filtered and evaporated to leave the crude title compound which was further purified by column chromatography on silica using 80% ethyl acetate and 20% hexane as eluent. The title compound (300 mg, 57%) was isolated as a white solid, m.p. 107°, found: C, 75.33; H, 7.25; N, 5.16. C₃₃H₃₈N₂O₄ requires C, 75.26; H, 7.27; N, 5.32%

### Example 26 Preparation of cis-7-(1-S-methoxycarbonyl-ethylaminocarbonyl)-8-(1-adamantylmethylaminocarbonyl)-2,3,5,6-dibenzobicyclo[2.2.2]octane (diastereomer A)

The compound of example 25 was separated into its component diastereomers by preparative HPLC using a silica phase column and 50% ethyl acetate and 50% hexane as eluant. The title compound diastereomer A had a retention time of 18.4 minutes and was isolated as a white powder, m.p.95-100°, [α]^{D}= -10.5° (c= 1.66 in methanol), found: C, 75.32; H, 7.14; N, 5.33. C₃₃H₃₈N₂O₄ requires C, 75.26; H, 7.27; N, 5.32%

### Example 27 Preparation of cis-7-(1-S-methoxycarbonyl-ethylaminocarbonyl)-8-(1-adamantylmethylaminocarbonyl)-2,3,5,6-dibenzobicyclo[2.2.2]octane (diastereomer B)

The compound of this example was the second diastereomer isolated by the HPLC technique described in example 26. The title compound diastereomer B had a retention time of 21.7 minutes and was isolated as a white powder, m.p.75-85°, [α]^{D}= +3.8° (c= 1.57 in methanol), found: C, 73.41; H, 7.37; N, 5.20. C₃₃H₃₈N₂O₄. 0.73 H₂O requires C, 73.42; H, 7.37; N, 5.20%

### Example 28 Preparation of cis-7-(1-R-methoxycarbonyl-ethylaminocarbonyl)-8-(1-adamantylmethylaminocarbonyl)-2,3,5,6-dibenzobicyclo[2.2.2]octane (mixture of diastereomers)

The reaction was performed essentially as in example 25 but using D-alanine methyl ester hydrochloride instead of the L-isomer. The title compound (300 mg, 57%) was isolated as a white solid, m.p. 113-5°, found: C, 74.41; H, 7.42; N, 5.14. C₃₃H₃₈N₂O₄.0.33 H₂O requires C, 74.41; H, 7.32; N, 5.26%

### Example 29 Preparation of cis-(±)-7-(2-benzyloxycarbonyl-ethylaminocarbonyl)-8-(1-adamantylmethylaminocarbonyl)-2,3,5,6-dibenzobicyclo[2.2.2]octane

The reaction was performed essentially as in example 25 but using the benzyl ester of beta alanine instead of L-alanine methyl ester hydrochloride. Yield 70%, m.p. 77-8°, found: C, 76.67; H, 7.04; N, 4.52. C₃₉H₄₂N₂O₄.0.43 H₂O requires C, 76.73; H, 7.08; N, 4.59%

### Example 30 Preparation of cis-(±)-7-(2-carboxy-ethylaminocarbonyl)-8-(1-adamantylmethylaminocarbonyl)-2,3,5,6-dibenzobicyclo[2.2.2]octane

The product of example 29 (370 mg, 0.6 mmole) was dissolved in ethanol (20 ml) and 10% palladium on charcoal (100 mg) was added. The mixture was stirred under an atmosphere of hydrogen overnight. The product was filtered through celite and on evaporation yielded the title compound, 56%. The product was characterised and tested as the N-methyl-D-glucamine salt, m.p. 75-8°, found: C, 62.54; H, 7.94; N, 5.31. C₃₉H₅₃N₃O₉. 2.44 H₂O requires C, 62.31; H, 7.76; N, 5.59%.

### Example 31 Preparation of cis-7-(1-S-aminocarbonyl-ethylaminocarbonyl)-8-(1-adamantylmethylaminocarbonyl)-2,3,5,6-dibenzobicyclo[2.2.2]octane (mixture of diastereomers)

The reaction was performed essentially as in example 25 but using L-alaninamide hydrochloride instead of the L-alanine methyl ester hydrochloride. Yield 81%, m.p. 175-185°, [α]^{D}= -6.5° (c=1 in methanol), found: C, 73.13; H, 7.53; N, 7.95. C₃₂H₃₇N₃O₃. 0.76 H₂O requires C, 73.16; H, 7.39; N, 8.00%

### Example 32 Preparation of cis-7-(1-S-(hydroxymethy)-ethylaminocarbonyl)-8-(1-adamantylmethylaminocarbonyl)-2,3,5,6-dibenzobicyclo[2.2.2]octane (mixture of diastereomers)

The reaction was performed essentially as in example 25 but using L-alaninol instead of the L-alanine methyl ester hydrochloride. Yield 76%, m.p. 115-120°, [α]^{D}= -4.0° (c=1 in methanol), found: C, 73.09; H, 7.82; N, 5.32. C₃₂H₃₈N₂O₃. 1.5 H₂O requires C, 73.14; H, 7.86; N, 5.33%

### Example 33 Preparation of cis-7-(1-S-benzyloxycarbonyl-ethylaminocarbonyl)-8-(1-adamantylmethylaminocarbonyl)-2,3,5,6-dibenzobicyclo[2.2.2]octane (diastereomer A)

The reaction was performed essentially as in example 25 but using the L-alanine benzyl ester of instead of L-alanine methyl ester hydrochloride. Overall yield 62%, The two diastereomers were separated by column chromatography (silica eluant 93% dichloromethane and 7% ethyl acetate). The less polar isomer has been designated diastereomer A, the title compound, Retention time HPLC silica 50% hexane and 50% ethyl acetate 7.9 min, m.p. 92-4°, [α]^{D}= -5.0° (c=1 in chloroform), found: C, 75.49; H, 7.12; N, 4.40. C₃₉H₄₂N₂O₄.1.0 H₂O requires C, 75.39; H, 7.15; N, 4.51%

### Example 34 Preparation of cis-7-(1-S-benzyloxycarbonyl-ethylaminocarbonyl)-8-(1-adamantylmethylaminocarbonyl)-2,3,5,6-dibenzobicyclo[2.2.2]octane (diastereomer B)

The more polar isomer from the chromatographic separation outlined in example 33 was designated diastereomer B, Retention time HPLC silica 50% hexane and 50% ethyl acetate 10.9 min, m.p. 90-5°, [α]^{D}= -1.0° (c=1 in chloroform), found: C, 77.66; H, 7.24; N, 4.41. C₃₉H₄₂N₂O₄ requires C, 77.71; H, 7.02; N, 4.65%

### Example 35 Preparation of cis-7-(1-S-carboxyethylaminocarbonyl)-8-(1-adamantylmethylaminocarbonyl)-2,3,5,6-dibenzobicyclo[2.2.2]octane (diastereomer A)

The compound was prepared essentially as described in example 30 but using the product of example 33 instead of cis-(±)-7-(2-benzyloxycarbonyl-ethylaminocarbonyl)-8-(1-adamantylmethylaminocarbonyl)-2,3,5,6-dibenzobicyclo[2.2.2]octane (the product of example 29) as the substrate. Yield 87%, [α]^{D}= -16.0° (c=1 in methanol). The compound was further characterised and tested as the N-methyl-D-glucamine salt, m.p. 100-105°, found: C, 60.66; H, 7.82; N, 5.74. C₃₉H₅₃N₃O₉. 3.4 H₂O requires C, 60.93; H, 7.84; N, 5.47%.

### Example 3 Preparation of cis-7-(1-S-carboxyethylaminocarbonyl)-8-(1-adamantylmethylaminocarbonyl)-2,3,5,6-dibenzobicyclo[2.2.2]octane (diastereomer B)

The compound was prepared essentially as described in example 30 but using the product of example 34 instead of cis-(±)-7-(2-benzyloxycarbonyl-ethylaminocarbonyl)-8-(1-adamantylmethylaminocarbonyl)-2,3,5,6-dibenzobicyclo[2.2.2]octane (the product of example 29) as the substrate. Yield 99%, [α]^{D}= +3.5° (c=1 in methanol). The compound was further characterised and tested as the N-methyl-D-glucamine salt, m.p. 105-110°, found: C, 61.89; H, 7.67; N, 5.72. C₃₉H₅₃N₃O₉. 2.6 H₂O requires C, 62.03; H, 7.77; N, 5.57%.

### Example 37 Preparation of endo-cis-(±)-8-(1-adamantylmethylaminocarbonyl)-2,3-benzo-5,6-(2,5-dimethoxybenzo)bicyclo[2.2.2]octane-7-carboxylic acid

### a. Diels-Alder adduct of 1,4-dimethoxyanthracene

Maleic anhydride (0.21 g, 2.18 mmole) and 1,4-dimethoxyanthracene (0.52 g, 2.18 mmole) were dissolved in toluene (5 ml) and heated to reflux for 4h under an atmosphere of argon. The solvent was evaporated and the residue washed with dichloromethane affording a white powder which was recrystallised from acetone to yield the exo adduct (140 mg), used in the preparation of example 38. The endo adduct was obtained as a white solid on addition of hexane to the dichloromethane solution, which was filtered and dried (143 mg).

### b. endo-cis-(±)-8-(1-adamantylmethylaminocarbonyl)-2,3-benzo-5,6-(2,5-dimethoxybenzo)bicyclo[2.2.2]octane-7-carboxylic acid

The endo adduct (from step a) (132 mg, 0.39 mmole) was dissolved in THF (3 ml) and 1-adamantylmethylamine (70 mg, 0.39 mmole) was added. The reaction was stirred at room temperature under an atmosphere of argon for 15 min. The solution was evaporated and taken up in dichloromethane and precipitated with hexane. The solution was filtered and dried, dissolved in warm ether and decanted from insoluble material. Addition of hexane, cooling and filtration gave the title compound (93 mg, 48%). The compound was characterised and tested as the N-methyl-D-glucamine salt, m.p. 99-103°, found: C, 61.63; H, 7.73; N, 3.92. C₃₈H₅₂N₂O₁₀. 2.3 H₂O requires C, 61.76; H, 7.73; N, 3.79%.

### Example 38 Preparation of exo-cis-(±)-8-(1-adamantylmethylaminocarbonyl)-2,3-benzo-5,6-(2,5-dimethoxybenzo)bicyclo-[2.2.2]octane-7-carboxylic acid

The compound was prepared essentially as in example 37 step b but using the exo anhydride from example 37, step a, rather than the endo isomer. Yield 88%. The compound was characterised and tested as the N-methyl-D-glucamine salt, m.p. 109-112°, found: C, 63.07; H, 7.70; N, 3.71. C₃₈H₅₂N₂O₁₀. 1.5 H₂O requires C, 63.04; H, 7.66; N, 3.87%.

### Example 39 Preparation of cis-(±)-8-(2-adamantylmethylaminocarbonyl)-2,3-benzo-5,6-(2,5-dimethoxybenzo)bicyclo-[2.2.2]octane-7-carboxylic acid

The compound was prepared essentially as in example 1 step b using 2-adamantylmethylamine instead of 1-phenyl-3-propylamine. Yield 85%. The compound was characterised and tested as the N-methyl-D-glucamine salt, found: C, 67.73; H, 7.81; N, 4.41. C₃₆H₄₈N₂O₈ requires C, 67.90; H, 7.60; N, 4.40%.

### Example 40 Preparation of cis-7-(1-S-dimethylaminocarbonyl-ethylaminocarbonyl)-8-(1-adamantylmethylamino-carbonyl)-2,3,5,6-dibenzobicyclo[2.2.2]octane (mixture of diastereomers)

The reaction was performed essentially as in example 25 but using L-alanine-N,N-dimethylamide trifluoroacetate instead of the L-alanine methyl ester hydrochloride. Yield 79%, m.p. 130-5°, [α]^{D}= -14° (c=1 in methanol) found: C, 72.58; H, 7.86; N, 7.35. C₃₄H₄₁N₃O₃. 1.3 H₂O requires C, 72.49; H, 7.81; N, 7.46%

### Example 41 Preparation of cis-7-(1-S-methoxycarbonylethylaminocarbonyl)-8-(cyclohexylmethylaminocarbonyl)-2,3,5,6-di benzobicyclo[2.2.2]octane (mixture of diastereomers)

The reaction was performed essentially as in example 25 but using (±)-cis-8-(1-cyclohexylmethylaminocarbonyl)-2,3,5,6-dibenzobicyclo[2.2.2]octane-7-carboxylic acid (prepared in example 9) instead of (±)-cis-8-(1-adamantylmethylaminocarbonyl)-2,3,5,6-dibenzobicyclo[2.2.2]octane-7-carboxylic acid. Yield 59%, m.p. 80-2°, found: C, 73.10; H, 7.31; N, 5.78. C₂₉H₃₄N₂O₄ requires C, 73.39; H, 7.22; N, 5.90%

### Example 42 Preparation of cis-7-[methoxycarbonylmethyl-(N-methyl)-aminocarbonyl]-8-(1-adamantylmethylaminocarbonyl)-2,3,5,6-dibenzobicyclo[2.2.2]octane

The reaction was performed essentially as in example 25 but using the methyl ester of sarcosine hydrochloride instead of the L-alanine methyl ester hydrochloride. Yield 74%, m.p. 185-7°, found: C, 75.47; H, 7.33; N, 5.22. C₃₃H₃₈N₂O₄ requires C, 75.26; H, 7.27; N, 5.32%

### Example 43 Preparation of cis-7-[ethoxycarbonylmethyl-(N-methyl)-aminocarbonyl]-8-(1-adamantylmethylaminocarbonyl)-2,3,5,6-dibenzobicyclo[2.2.2]octane

The reaction was performed essentially as in example 25 but using the ethyl ester of sarcosine hydrochloride instead of the L-alanine methyl ester hydrochloride. Yield 57%, found: C, 75.40; H, 7.51; N, 5.03. C₃₄H₄₀N₂O₄ requires C, 75.53; H, 7.46; N, 5.18%

### Example 44 Preparation of (±)-trans-8-(1-adamantylmethylaminocarbonyl)-2,3,5,6-dibenzobicyclo[2.2.2]octane-7-carboxylic acid

### a. (±)-trans-8-ethoxycarbonyl-2,3,5,6-dibenzobicyclo-[2.2.2]octane-7-carboxylic acid

Anthracene (5 g, 28 mmol) and fumaric acid monoethyl ester (4.04 g, 28 mmol) were dissolved in dioxan (50 ml) and the solution heated at reflux for 3d. The reaction mixture was evaporated and the solid obtained recrystallised from hot toluene and dried (5.06 g, 56%).

### b. (±)-trans-ethyl-8-(1-adamantylmethylaminocarbonyl)-2,3,5,6-dibenzobicyclo[2.2.2]octane-7-carboxylate

The product of step a (0.2 g, 0.62 mmol) was stirred in anhydrous benzene (25 ml) and thionyl chloride (0.27 ml, 3.1 mmol) was added. The mixture was stirred at room temperature for 2h. The solution was evaporated in vacuo to leave a gum. This was taken up in dry dichloromethane (25 ml) and 1-adamantylmethylamine (0.103 g, 0.62 mmol) was added followed by triethylamine (0.095 0ml, 0.68 mmol) and the mixture stirred at room temperature for 1h. The dichloromethane solution was washed successively with 2M hydrochloric acid, water and saturated brine and dried, filtered and evaporated to afford a colourless solid (0.28 g, 96%).

### c. (±)-trans-8-(1-adamantylmethylaminocarbonyl)-2,3,5,6-dibenzobicyclo[2.2.2]octane-7-carboxylic acid

The product of step b (0.28 g, 0.6 mmol) was dissolved in ethanol (20 ml) and sodium hydroxide (48 Mg, 1.2 mmole) was added. The reaction mixture was heated to reflux for 2 min whereupon it was diluted with 2N hydrochloric acid, cooled to room temperature and filtered. The precipitated solid was washed successively with water, ethanol (2 ml), ether (10 ml) and dried (165 mg, 63%). The compound was characterised and tested as the N-methyl-D-glucamine salt, found: C, 67.73; H, 7.62; N, 4.22. C₃₆H₄₈N₂O₈ requires C, 67.90; H, 7.60; N, 4.40%.

### Example 45 Preparation of methyl cis-(±)-8-(1-adamantylmethyloxycarbonyl)-2,3,5,6-dibenzobicyclo[2.2.2]octane-7-carboxylate

cis-(±)-8-(1-adamantylmethyloxycarbonyl)-2,3,5,6-dibenzobicyclo[2.2.2]octane-7-carboxylic acid prepared as in example 13 (115 mg, 0.26 mmol) was dissolved in ether (10 ml) and a solution of diazomethane in ether was added dropwise until a yellow colour persisted in solution. After 20 min at room temperature the reaction was quenched by dropwise addition of acetic acid. The reaction mixture was diluted with ether and washed sequentially with 5% sodium hydrogencarbonate solution and brine. The organic layer was dried, filtered and evaporated to give a colourless glass. Trituration with hexane then gave the desired product as a white solid (65 mg, 55%), m.p. 186-7°, found: C, 78.86; H, 7.06. C₃₀H₃₂O₄ requires C, 78.92; H, 7.06%

### Example 46 Preparation of cis-7-(2-R-benzyloxycarbonylpyrrolidinocarbonyl)-8-(1-adamantylmethylaminocarbonyl)-2,3,5,6-dibenzobicyclo[2.2.2]octane (mixture of diastereomers)

(±)-cis-8-(1-adamantylmethylaminocarbonyl)-2,3,5,6-dibenzobicyclo[2.2.2]octane-7-carboxylic acid (prepared as in example 14) (440 mg, 1 mmole) and PyBOP (520 mg, 1 mmole) were taken up in dry dichloromethane (15 ml) and Hunigs base (0.52 ml, 3 mmole) was added. The reaction mixture was stirred under an atmosphere of dry argon for 1h. D-Proline benzyl ester hydrochloride (266 mg, 1.1 mmole) was added and the mixture stirred overnight. The organic layer was washed with 5% potassium hydrogensulphate (15 ml), sodium hydrogencarbonate (15 ml) and saturated brine (15 ml). It was then dried, filtered and evaporated to leave the crude title compound which was further purified by column chromatography on silica using a gradient elution starting with 50% ethyl acetate and 50% hexane going up to 80% ethyl acetate and 20% hexane. The title compound (580 mg, 92%) was isolated, m.p. 89-90°, found: C, 78.14; H, 7.13; N, 4.41. C₄₁H₄₄N₂O₄ requires C, 78.31; H, 7.05; N, 4.45%

### Example 47 Preparation of cis-7-(2-S-benzyloxycarbonylpyrrolidinocarbonyl)-8-(1-adamantylmethylaminocarbonyl)-2,3,5,6-dibenzobicyclo[2.2.2]octane (mixture of diastereomers)

This compound was prepared essentially as in example 46 using L-proline benzyl ester hydrochloride instead of D-proline benzyl ester hydrochloride. m.p. 91-2°, found: C, 77.03; H, 7.12; N, 4.22. C₄₁H₄₄N₂O₄. 0.6 H₂O requires C, 76.99; H, 7.12; N, 4.38%

### Example 48 Preparation of cis-7-(2-R-carboxy-pyrrolidinocarbonyl)-8-(1-adamantylmethylaminocarbonyl)-2,3,5,6-dibenzobicyclo[2.2.2]octane (mixture of diastereomers)

The product of example 46 ( 520 mg, 0.83 mmol) was dissolved in ethanol (20 ml) and 10% palladium on charcoal (100 mg) was added. The reaction mixture was stirred overnight under an atmosphere of hydrogen and then filtered through celite and evaporated to yield the title compound (380 mg, 86%). The compound was characterised and tested as the N-methyl-D-glucamine salt m.p. 124-7°, found: C, 64.58; H, 7.92; N, 5.38. C₄₁H₅₅N₃O₉. 1.71 H₂O requires C, 64.40; H, 7.70; N, 5.45%

### Example 49 Preparation of cis-7-(2-S-carboxy-pyrrolidinocarbonyl)-8-(1-adamantylmethylaminocarbonyl)-2,3,5,6-dibenzobicyclo[2.2.2]octane (mixture of diastereomers)

This compound was prepared essentially as in example 48 but using the product of example 47 as substrate rather than the product of example 46. Yield 60% The compound was characterised and tested as the N-methyl-D-glucamine salt m.p. 98-101°, found: C, 61.12; H, 8.11; N, 5.08. C₄₁H₅₅N₃O₉. 3.88 H₂O requires C, 61.26; H, 7.87; N, 5.23%

### Example 50 Preparation of cis-7-(2-methoxycarbonyl-pyrrolidinocarbonyl)-8-(1-adamantylmethylaminocarbonyl)-2,3,5,6-dibenzobicyclo[2.2.2]octane (mixture of diastereomers)

The compound of example 46 (320 mg, 0.59 mmol) was dissolved in dioxan (10 ml) and a solution of diazomethane in ether was added dropwise until the colour persisted. After stirring for lh at room temperature acetic acid was added to quench the reaction and the solution was evaporated and taken up in ethyl acetate. The product was then washed with saturated sodium hydrogencarbonate solution and saturated brine. The organic phase was dried filtered and evaporated and the title compound purified on silica using 50% ethyl acetate and 50% hexane as eluent. Yield (120 mg, 37%), m.p. 112-5°, found: C, 75.86; H, 7.43; N, 4.96. C₃₅H₄₀N₂O₄ requires C, 75.86; H, 7.29; N, 5.07%

### Example 51 Preparation of cis-7-(2-S-methoxycarbonylpyrrolidinocarbonyl)-8-(1-adamanthylmethylaminocarbonyl)-2,3,5,6-dibenzobicyclo[2.2.2]octane (mixture of diastereomers)

This compound was prepared essentially as in example 50 using the compound of example 47 as substrate instead of the compound of example 46. Yield 43%, m.p. 104-6°, found: C, 74.55; H, 7.43; N, 4.88. C₃₅H₄₀N₂O₄. 0.6 H₂O requires C, 74.57; H, 7.37; N, 4.97%

### Example 52 Preparation of (±)-cis-7-(3-indolylethylaminocarbonyl)-8-(1-adamantylmethylaminocarbonyl)-2,3,5,6-dibenzobicyclo[2.2.2]octane

(±)-cis-8-(1-adamantylmethylaminocarbonyl)-2,3,5,6-dibenzobicyclo[2.2.2]octane-7-carboxylic acid (prepared as in example 14) (440 mg, 1 mmole) and PyBOP (520 mg, 1 mmole) were taken up in dry dichloromethane (15 ml) and Hunigs base (0.52 ml, 3 mmole) was added. The reaction mixture was stirred under an atmosphere of dry argon for 1h. Tryptamine hydrochloride (197 mg, 1 mmole) was added and the mixture stirred overnight. The organic layer was washed with 5% potassium hydrogensulphate (15 ml), sodium hydrogencarbonate (15 ml) and saturated brine (15 ml). It was then dried, filtered and evaporated to leave the crude title compound which was further purified by column chromatography on silica using 15% ethyl acetate and 85% dichloromethane as eluent. The title compound (432 mg, 74%) was isolated as a white solid, m.p. 130-40°, found: C, 75.92; H, 6.98; N, 7.19. C₃₉H₄₁N₃O₂ requires C, 76.26; H, 7.28; N, 6.84%

### Example 53 Preparation of cis-7-[R-2-(3-indolyl)-1-methoxycarbonyl-ethylaminocarbonyl]-8-(1-adamantylmethylaminocarbonyl)-2,3,5,6-dibenzobicyclo[2.2.2]octane (mixture of diastereomers)

This compound was prepared essentially as in example 52 using D-tryptophan methyl ester hydrochloride instead of tryptamine hydrochloride. Yield 78%, m.p. 135-460°; found: C, 75.64; H, 6.81; N, 6.09. C₄₁H₄₃N₃O₄. 0.65 H₂O requires C, 75.35; H, 6.83; N, 6.43%

### Example 54 Preparation of cis-7-[2-S-(3-indolyl)-1-methoxycarbonyl-ethylaminocarbonyl]-8-(1-adamantylmethylaminocarbonyl)-2,3,5,6-dibenzobicyclo[2.2.2]octane (mixture of diastereomers)

This compound was prepared essentially as in example 52 using L-tryptophan methyl ester hydrochloride instead of tryptamine hydrochloride. Yield 80%, m.p. 135-42°, found: C, 76.55; H, 6.95; N, 6.77. C₄₁H₄₃N₃O₄ requires C, 76.63; H, 6.75; N, 6.55%

### Example 55 Preparation of cis-7-[2-R-(3-indolyl)-1-carboxyethylaminocarbonyl]-8-(1-adamantylmethylaminocarbonyl)-2,3,5,6-dibenzobicyclo[2.2.2]octane (diastereomer 1)

### a. cis-7-[2-R-(3-indolyl)-1-benzyloxycarbonyl-ethylaminocarbonyl]-8-(1-adamantylmethylaminocarbonyl)-2,3,5,6-dibenzobicyclo[2.2.2]octane and separation of diastereomers

The mixture of diastereomers was prepared essentially as in example 52 using D-tryptophan benzyl ester trifluoroacetate salt instead of tryptamine hydrochloride. The diastereomers were separated by column chromatography (silica 15% ethyl acetate and 85% dichloromethane) to give a 35% yield of each component.

### b. cis-7-[2-R-(3-indolyl)-1-carboxy-ethylaminocarbonyl]-8-(1-adamantylmethylaminocarbonyl)-2,3,5,6-dibenzobicyclo-[2.2.2]octane (diastereomer 1)

### Diastereomer 1 (from step a) (0.23 g, 0.32 mmol) was dissolved in methanol (10 ml) and a catalytic amount of 10% palladium on charcoal was added. The mixure was stirred under an atmosphere of hydrogen overnight, filtered and evaporated to leave the title compound (0.21 g, 100%). The compound was characterised and tested as the N-methyl-D-glucamine salt, m.p. 130-5°, [α]^{D}= -18.5° (c=1 in methanol), found: C, 67.17; H, 7.36; N, 6.49. C₄₇H₅₈N₄O₉. H₂O requires C, 67.09; H, 7.19; N, 6.65%

### Example 56 Preparation of cis-7-[2-R-(3-indolyl)-1-carboxy-ethylaminocarbonyl]-8-(1-adamantylmethylaminocarbonyl)-2,3,5,6-dibenzobicyclo[2.2.2]octane (diastereomer 2)

The compound was prepared essentially as in example 4 but using diastereomer 2 (isolated in example 4 step a) instead of diastereomer 1 in step b. Yield 90% The compound was further characterised and tested as the N-methyl-D-glucamine salt, m.p. 140-5°, [α]^{D}= -22.0° (c=1 in methanol), found: C, 67.16; H, 7.18; N, 6.68. C₄₇H₅₈N₄O₉. H₂O requires C, 67.09; H, 7.19; N, 6.65%

### Example 57 Preparation of cis-7-[2-S-(3-indolyl)-1-carboxy-ethylaminocarbonyl]-8-(1-adamantylmethylaminocarbonyl)-2,3,5,6-dibenzobicyclo[2.2.2]octane (diastereomer 1)

This was prepared essentially as in example 55 but using L-tryptophan benzyl ester trifluoracetate instead of the D-isomer in step a. Separation of diastereomers was achieved at the benzyl ester stage as indicated in example 55 step a. and diastereomer 1 used in step b. was again the isomer with the higher R_{f}. Overall yield 22% based on starting racemic carboxylic acid.

The compound was further characterised and tested as the N-methyl-D-glucamine salt, m.p. 119-24°, [α]^{D}= -5.7° (c=0.7 in methanol), found: C, 65.07; H, 7.25; N, 6.44. C₄₇H₅₈N₄O₉ requires C, 65.03; H, 7.32; N, 6.45%

### Example 58 Preparation of cis-7-[2-S-(3-indolyl)-1-carboxy-ethylaminocarbonyl]-8-(1-adamantylmethylaminocarbonyl)-2,3,5,6-dibenzobicyclo[2.2.2]octane Diastereomer 2

The compound was prepared essentially as in example 55 but using diastereomer 2 (isolated in example 57 step a) instead of diastereomer 1 in step b. Overall yield 26% based on starting racemic carboxylic acid. The compound was characterised and tested as the N-methyl-D-glucamine salt, m.p. 133-8°, [α]^{D}= +7.6° (c=0.66 in methanol), found: C, 64.42; H, 7.17; N, 6.41. C₄₇H₅₈N₄O₉.3H₂O requires C, 64.37; H, 7.35; N, 6.39%

### Example 59 Preparation of cis-(±)-7-(2-Furanylmethylaminocarbonyl)-8-(1-adamantylmethylaminocarbonyl)-2, 3, 5, 6-dibenzobicyclo[2.2.2]octane

The reaction was performed essentially as in Example 25 but using furfurylamine instead of L-alanine methyl ester hydrochloride. Yield 65%, m.p. 300°, found: C, 78.15; H, 6.99; N, 5.42. C₃₄H₃₆N₂O₃ requires C, 78.43; H, 6.97; N, 5.38%

### Example 60 Preparation of cis-(±)-7-[2-(5-methyloxycarbonyl)-furanylaminocarbonyl]-8-(1-adamantylmethylaminocarbonyl)-2,3,5,6-dibenzobicyclo[2.2.2]octane

The reaction was performed essentially as in Example 25 but using 5-methoxycarbonyl-2-aminofuran instead of L-alanine methyl ester hydrochloride. Yield 65%, m.p. 225°, found: C, 74.31; H. 6.54; N, 4.91. C₃₅H₃₆N₂O₅ requires C, 74.45; H, 6.43; N, 4.96%

### Example 61 Preparation of cis-7-(1-S-benzyloxycarbonyl-2-methylpropylaminocarbonyl)-8-(1-adamantylmethylaminocarbonyl)-2,3,5,6-dibenzobicyclo[2.2.2]octane (mixture of diastereomers)

The reaction was performed essentially as in example 46 but using the p-toluenesulphonate salt of the benzyl ester of L-valine instead of D-proline benzyl ester hydrochloride. m.p 85-87°. Found: C, 77.99; H, 7.52; N, 4.17. C₄₁H₄₄N₂O₄. 0.1H₂O requires C, 77.86; H, 7.36; N, 4.42%

### Example 62 Preparation of cis-7-(1-S-carboxy-2-methylpropylaminocarbonyl)-8-(1-adamantylmethylaminocarbonyl)-2,3,5,6-dibenzobicyclo-[2.2.2]octane (mixture of diastereomers)

The reaction was performed essentially as in example 48 but using the product of example 61 as substrate instead of the product of example 46. The compound was characterised and tested as the N-methyl-D-glucamine salt m.p 105-8°. Found: C, 64.00; H, 8.06; N, 5.37. C₄₁H₅₇N₃O₉. 1.8H₂O requires C, 64.09; H, 7.95; N, 5.46%

### Example 63 Preparation of cis-7-(1-S-methoxycarbonyl-2-methylpropylaminocarbonyl)-8-(1-adamantylmethylaminocarbonyl)-2,3,5,6-dibenzobicyclo-[2.2.2]octane (mixture of diastereomers)

cis-7-(1-S-carboxy-2-methylpropylaminocarbonyl)-8-(1-adamantylmethylaminocarbonyl)-2,3,5,6-dibenzobicyclo-[2.2.2]octane (mixture of diastereomers) (150 mg) prepared as in example 62 was dissolved in ethyl acetate (5ml) and a solution of diazomethane in diethyl ether was added until a yellow colour persisted in solution. After stirring the rection mixture at room temperature for 10 min, glacial acetic acid was added and the organic layer was washed with saturated sodium hydrogencarbonate solution, dried over magnesium sulphate, filtered and evaporated. Yield 130 mg, 85%, m.p. 103-5°. Found: C, 75.51; H, 7.67; N, 5.06. C₃₅H₄₂N₂O₄ requires C, 75.78; H, 7.63; N, 5.05%

### Example 64 Preparation of cis-7-(1-S-2-dicarboxyethylaminocarbonyl)-8-(1-adamantylmethylaminocarbonyl)-2,3,5,6-dibenzobicyclo[2.2.2]octane (mixture of diastereomers)

### a. cis-7-(1-S-2-dibenzyloxycarbonylethylaminocarbonyl)-8-(1-adamantylmethylaminocarbonyl)-2,3,5,6-dibenzobicyclo-[2.2.2]octane (mixture of diastereomers)

The reaction was performed essentially as in example 46 but using the dibenzyl ester of L-aspartic acid instead of D-proline benzyl ester hydrochloride. The product was used directly in step b.

### b. cis-7-(1-S-2-dicarboxyethylaminocarbonyl)-8-(1-adamantylmethylaminocarbonyl)-2,3,5,6-dibenzobicyclo-[2.2.2]octane (mixture of diastereomers)

The reaction was performed essentially as in example 48 but using the product of step a. as substrate instead of the product of example 46. The compound was characterised and tested as the mono-N-methyl-D-glucamine salt, m.p 115-7°. Found: C, 62.84; H, 7.03; N, 5.35. C₄₀H₅₃N₃O₁₁. 0.62H₂O requires C, 62.54; H, 7.03; N, 5.61%

### Example 65 Preparation of 7-(1-S-carboxy-2-phenyl-ethylaminocarbonyl)-8-(1-adamantylmethylaminocarbonyl)-2,3,5,6-dibenzobicyclo[2.2.2]octane (mixture of diastereomers)

The reaction was performed essentially as in example 64 but using the benzyl ester of phenylalanine in step a. instead of the dibenzyl ester of aspartic acid. The compound was characterised and tested as the N-methyl-D-glucamine salt, m.p 101-3°. Found: C, 66.13; H, 7.64; N, 5.05 C₄₅H₅₇N₃O₄. 1.9H₂O requires C, 66.06; H, 7.49; N, 5.14%

### Example 66 Preparation of (±)-cis-7-(1-methoxycarbonyl-1-methylethylaminocarbonyl)-8-(1-adamantylmethylaminocarbonyl)-2,3,5,6-dibenzobicyclo[2.2.2]octane

The reaction was performed essentially as in example 46 but using the trifluoromethylacetate salt of the methyl ester of aminoisobutyric acid instead of D-proline benzyl ester hydrochloride. m.p 120-2°. Found: C, 75.51; H, 7.43; N, 4.90. C₃₄H₄₀N₂O₄ requires C, 75.53; H, 7.46; N, 5.18%

### Example 67 Preparation of (±)-cis-7-(1-carboxy-1-methylethylaminocarbonyl)-8-(1-adamantylmethylaminocarbonyl)-2,3,5,6-dibenzobicyclo[2.2.2]octane

The reaction was performed essentially as in example 64 but using the benzyl ester of aminoisobutyric acid in step a. instead of the dibenzyl ester of aspartic acid. The compound was characterised and tested as the N-methyl-D-glucamine salt, m.p 115-25°. Found: C, 64.71; H, 7.74; N, 5.92. C₄₀H₅₅N₃O₉ requires C, 64.93; H, 7.76; N, 5.68%

### Example 68 Preparation of cis-7-(2-R-carboxy-4-R-hydroxypyrrolidinocarbonyl)-8-(1-adamantylmethylaminocarbonyl)-2,3,5,6-dibenzobicyclo[2.2.2]octane (mixture of diastereomers)

The reaction was performed essentially as in example 64 but using the benzyl ester of cis hydroxy-D-proline in step a. instead of the dibenzyl ester of aspartic acid. The compound was characterised and tested as the N-methyl-D-glucamine salt, m.p 118-21°. Found: C, 58.59; H, 7.48; N, 5.10. C₄₁H₅₅N₃O₁₀. 4.8 mol H₂O requires C, 58.89; H, 7.78; N, 5.02%

### Example 69 Preparation of cis-7-(2-R-carboxy-4-R-hydroxypyrrolidinocarbonyl)-8-(1-adamantylmethylaminocarbonyl)-2,3,5,6-dibenzobicyclo[2.2.2]octane (diastereomer 1)

The cis-7-(2-R-benzyloxycarbonyl-4-R-hydroxy-pyrrolidino-carbonyl)-8-(1-adamantylmethylaminocarbonyl)-2,3,5,6-dibenzobicyclo[2.2.2]octane mixture of diastereomers prepared as referenced in example 68 was separated into its two diastereomeric components by repeated recrystallisation from ethyl acetate. The insoluble isomer was designated diastereomer 1. The soluble material isolated by evaporation was designated diastereomer 2. Diastereomer 1 was converted to the title compound essentially as in example 48 using it as substrate instead of the product of example 46. The compound was characterised and tested as the N-methyl-D-glucamine salt, m.p 112-4°. Found: C, 60.81; H, 7.86; N, 4.96. C₄₁H₅₅N₃O₁₀. 4.4 mol H₂O requires C, 60.70; H, 7.68; N, 5.18%

### Example 70 Preparation of cis-7-(2-R-carboxy-4-R-hydroxy-pyrrolidinocarbonyl)-8-(1-adamantylmethylaminocarbonyl)-2,3,5,6-dibenzobicyclo[2.2.2]octane (diastereomer 2)

Diastereomer 2 prepared as described in example 69 was converted to the title compound essentially as in example 48 using it as substrate instead of the product of example 46. The compound was characterised and tested as the N-methyl-D-glucamine salt, m.p 132-35°. Found: C, 63.69; H, 7.51; N, 5.04. C₄₁H₅₅N₃O₁₀. 1.5 mol H₂O requires C, 63.40; H, 7.52; N, 5.41%

### Example 71 Preparation of cis-7-(2-R-methoxycarbonyl-4-R-hydroxy-pyrrolidinocarbonyl)-8-(1-adamantylmethylaminocarbonyl)-2,3,5,6-dibenzobicyclo[2.2.2]octane (diastereomer 1)

The compound was prepared essentially as in example 63 but using the product of example 69 as substrate instead of cis-7-(1-S-carboxy-2-methylpropylaminocarbonyl)-8-(1-adamantylmethylaminocarbonyl)-2,3,5,6-dibenzobicyclo[2.2.2]octane (mixture of diastereomers), m.p 133-35°. Found: C, 72.81; H, 7.21; N, 4.88. C₃₅H₄₀N₂O₅. 0.5 mol H₂O requires C, 72.81; H, 7.15; N, 4.85%

### Example 72 Preparation of cis-7-(2-R-methoxycarbonyl-4-R-hydroxy-pyrrolidinocarbonyl)-8-(1-adamantylmethylaminocarbonyl)-2,3,5,6-dibenzobicyclo[2.2.2]octane (diastereomer 2)

The compound was prepared essentially as in example 63 but using the product of example 70 as substrate instead of cis-7-(1-S-carboxyl-2-methylpropylaminocarbonyl)-8-(1-adamantylmethylaminocarbonyl)-2,3,5,6-dibenzobicyclo[2.2.2]octane (mixture of diastereomers), m.p 133-35°. Found: C, 70.28; H, 7.14; N, 4.81. C₃₅H₄₀N₂O₅. 1.5 mol H₂O requires C, 70.42; H, 7.28; N, 4.69%

### Example 73 Preparation of (±)-cis-7-(3-(±)-carboxy-piperidinocarbonyl)-8-(1-adamantylmethylaminocarbonyl)-2,3,5,6-dibenzobicyclo[2.2.2]octane

The reaction was performed essentially as in example 46 but using the trifluoromethylacetate salt of the benzyl ester of racemic nipecotic acid instead of D-proline benzyl ester hydrochloride. The compound was characterised and tested as the N-methyl-D-glucamine salt. Found: C, 65.54; H, 8.12; N, 5.23. C₄₂H₅₇N₃O₉. 1.4 H₂O requires C, 65.24; H, 7.80; N, 5.43%

### Example 74 Preparation of (±)-cis-7-(3-(±)-methoxycarbonylpiperidinocarbonyl)-8-(1-adamantylmethylaminocarbonyl)-2,3,5,6-dibenzobicyclo[2.2.2]octane

The compound was prepared essentially as in example 63 but using the product of example 73 as substrate instead of cis-7-(1-S-carboxy-2-methylpropylaminocarbonyl)-8-(1-adamantylmethylaminocarbonyl)-2,3,5,6-dibenzobicyclo[2.2.2]octane (mixture of diastereomers). Found: C, 74.24; H, 7.30; N, 4.65. C₃₆H₄₂N₂O₄. 0.8 H₂O requires C, 74.40; H, 7.56; N, 4.82%

### Example 75 Preparation of cis-7-(1-S-cyanoethylaminocarbonyl)-8-(1-adamantylmethylaminocarbonyl)-2,3,5,6-dibenzobicyclo[2.2.2]octane (mixture of diastereomers)

The compound of example 31 (0.33 g, 0.59 mmol) was dissolved in pyridine (5 ml) and cooled to 0° under an atmosphere of dry argon. Tosyl chloride (0.13 g, 0.70 mmol) was added and the reaction allowed to warm to room temperature and was then stirred overnight. The solvent was removed by evaporation and the residue partitioned between ethyl acetate and water. The organic phase was washed successively with 1M hydrochloric acid and saturated sodium hydrogen carbonate solution, dried, filtered and evaporated to leave the crude product. This material was purified by column chromatography (silica, 90% dichloromethane and 10% ethyl acetate) to leave the title compound (150 mg), m.p. 125-8°. Found: C, 76.30; H, 7.23; N, 8.21. C₃₂H₃₅N₃O₂. 0.6 H₂O requires C, 76.19; H, 7.23; N, 8.32%

### Example 76 Preparation of cis-7-(1-S-methylcarbonylethylaminocarbonyl)-8-(1-adamantylmethylaminocarbonyl)-2,3,5,6-dibenzobicyclo[2.2.2]octane (mixture of diastereomers)

The compound of example 75 (0.25 g, 0.5 mmol) was dissolved in THF (3 ml) and cooled to 0° under an atmosphere of dry argon. Methyl magnesium bromide solution (1.4M in THF 1.4 ml, 2.0 mmol) was added. The solution was stirred at 0° for lh. 2M hydrochloric acid (2ml) was added followed by saturated ammonium chloride solution. (20 ml). The product was extracted with ethyl acetate (2 x 20 ml), dried, filtered and evaporated (0.25 ml). The crude product was purified by column chromatography (silica 90% dichloromethane and 10% ethyl acetate) to leave the title compound (130 mg), m.p. 105-10°. Found: C, 76.61; H, 7.40; N, 5.25. C33H38N2O3. 0.4 H2O requires C, 76.54; H, 7.55; N, 5.41%

### Example 77 Preparation of cis-7-(1-S-propyloxycarbonylethylaminocarbonyl)-8-(1-adamantylmethylaminocarbonyl)-2,3,5,6-dibenzobicyclo[2.2.2]octane (mixture of diastereomers)

The reaction was performed essentially as in example 46 but using the trifluoromethylacetate salt of the propyl ester of L-alanine (prepared from alklation of the caesium salt of BOC-L-alanine with propyl bromide followed by treatment with trifluoroacetic acid) instead of D-proline benzyl ester hydrochloride. m.p 90-3°. Found: C, 75.33; H, 7.74; N, 4.81. C₃₅H₄₂N₂O₄. 0.25 H₂O requires C, 75.33; H, 7.66; N, 5.01%

### Example 78 Preparation of cis-7-(2-R-carboxy-pyrrolidinocarbonyl)-8-(1-adamantylmethylaminocarbonyl)-2,3,5,6-dibenzobicyclo[2.2.2]octane (diastereomer 1)

The compound of example 48 was separated into its constituent diastereomers by reverse phase HPLC (silica C8 column 60% acetonitrile, 40% water and 0.1% acetic acid modifier). The first compound eluted was designated diastereomer 1, the title compound. The compound was characterised and tested as the N-methyl-D-glucamine salt. Found: C, 64.23; H, 7.86; N, 5.38. C₄₁H₅₅N₃O₁₀. 1.8 H₂O requires C, 64.22; H, 7.71; N, 5.48%

### Example 79 Preparation of cis-7-(2-R-carboxy-pyrrolidinocarbonyl)-8-(1-adamantylmethylaminocarbonyl)-2,3,5,6-dibenzobicyclo[2.2.2]octane (diastereomer 2)

The second compound eluted during the HPLC separation referred to in example 78 was designated diastereomer 2. The compound was characterised and tested as the N-methyl-D-glucamine salt. Found: C, 64.23; H, 7.86; N, 5.38. C₄₁H₅₅N₃O₁₀. 1.8 H₂O requires C, 64.22; H, 7.71; N, 5.48%

### Example 80 Preparation of cis-7-(2-S-methoxycarbonylpyrrolidinocarbonyl)-8-(1-adamantylmethylaminocarbonyl)-2,3,5,6-dibenzobicyclo[2.2.2]octane (diastereomer 1)

The compound of example 51 was separated into its constituent diastereomers by repeated recrystallisation from 80% ethyl acetate and 20% hexane. The crystals isolated were designated diastereomer 1, the title compound, m.p. 256°. Found: C, 76.01; H, 7.31; N, 4.98. C₃₅H₄₀N₂O₄ requires C, 76.06; H, 7.29; N, 5.07%

### Example 81 Preparation of cis-7-(2-S-methoxycarbonylpyrrolidinocarbonyl)-8-(1-adamantylmethylaminocarbonyl)-2,3,5,6-dibenzobicyclo[2.2.2]octane (diastereomer 2)

The mother liquors from the recrystallisation described in example 80 were concentrated to yield the other pure isomer designated diastereomer 2, m.p. 94-6°. Found: C, 74.88; H, 7.31; N, 5.03. C₃₅H₄₀N₃O₄ . 0.5 H₂O requires C, 74.91; H, 7.35; N, 4.99%

### Example 82 Preparation of cis-7-(1-S-carboxy-2-hydroxyethylaminocarbonyl-8-(1-adamantylmethylaminocarbonyl)-2,3,5,6-dibenzobicyclo[2.2.2]octane (diastereomer 1)

### Step a. cis-7-(1-S-Benzyloxycarbonyl-2-hydroxyethylaminocarbonyl)-8-(1-adamantylmethylaminocarbonyl)-2,3,5,6-dibenzobicyclo[2.2.2]octane (diastereomer 1 and 2)

The reaction was performed essentially as in example 46 but using L-serine benzyl ester hydrochloride instead of D-proline benzyl ester hydrochloride. The compound was separated into its component diastereomers by column chromatography (silica 25% ethyl acetate and 75% dichloromethane). The less polar material was designated diastereomer 1 and the more polar diastereomer 2.

### Step b. cis-7-(1-S-carboxy-2-hydroxyethylaminocarbonyl)-8-(1-adamantylmethylaminocarbonyl)-2,3,5,6-dibenzobicyclo[2.2.2]octane (diastereomer 1)

The reaction was performed essentially as in example 48 but using the diastereomer 1 from step a. above as substrate instead of the product of example 46. The compound was characterised and tested as the N-methyl-D-glucamine salt, m.p 102-5° found: C, 61.24; H, 7.78; N, 5.22. C₃₉H₅₃N₃O₁₀. 2.4 H₂O requires C, 61.08; H, 7.59; N, 5.48%

### Example 83 Preparation of cis-7-(1-S-carboxy-2-hydroxyethylaminocarbonyl)-8-(1-adamantylmethylaminocarbonyl)-2,3,5,6-dibenzobicyclo[2.2.2]octane (diastereomer 2)

The reaction was performed essentially as in example 48 but using the diastereomer 2 from example 82 step a. as substrate instead of the product of example 46. The compound was characterised and tested and tested as the N-methyl-D-glucamine salt, m.p 107-10° found: C, 61.27; H, 7.69; N, 5.29. C₃₉H₅₃N₃O₅. 2.3 H₂O requires C, 61.19; H, 7.59; N, 5.49%

### Example 84 Preparation of cis-7-(1-S-methoxycarbonyl-2-hydroxyethylaminocarbonyl)-8-(1-adamantylmethylaminocarbonyl-2,3,5,6-dibenzobicyclo[2.2.2]octane (diastereomer 1)

### Step a. cis-7-(1-S-methoxycarbonyl-2-hydroxyethylaminocarbonyl)-8-(1-adamantylmethylaminocarbonyl)-2,3,5,6-dibenzobicyclo[2.2.2]octane (mixture of diastereomers)

The reaction was performed essentially as in example 46 but using L-serine methyl ester hydrochloride instead of D-proline benzyl ester hydrochloride.

### Step b. cis-7-(1-S-methoxycarbonyl-2-hydroxyethylaminocarbonyl)-8-(1-adamantylmethylaminocarbonyl)-2,3,5,6-dibenzobicyclo-[2.2.2]octane (diastereomer 1)

The compound prepared in step a. was separated into its component diastereomers by column chromatography (silica 30% ethyl acetate and 70% dichloromethane). The less polar material was designated diastereomer 1, the title compound, m.p 115-20° found: C, 68.94; H, 6.95; N, 4.91. C₃₃H₃₈N₂O₅. 1.6 H₂O requires C, 69.26; H, 7.27; N, 4.90%

### Example 85 Preparation of cis-7-(1-S-methoxycarbonyl-2-hydroxyethylaminocarbonyl)-8-(1-adamantylmethylaminocarbonyl-2,3,5,6-dibenzobicyclo[2.2.2]octane (diastereomer 2)

The compound of this example was the more polar diastereomer isolated from the column chromatography described in example 84 step b, m.p 100-10° found: C, 56.92; H, 6.03; N, 3.49. C₃₃H₃₈N₂O₅. 2.4 DCM requires C, 56.91; H, 5.78; N, 3.75%

### Example 86 Preparation of (±)-cis-7-(1-methoxycarbonyl-1-ethyleneaminocarbonyl)-8-(1-adamantylmethylaminocarbonyl)-2,3,5,6-dibenzobicyclo[2.2.2]octane

The product of example 64 step a. (270 mg, 0.5 mmol) was dissolved in THF (2 ml) and N,N-carbonyldiimidazole (80 mg, 0.5 mmol) was added followed by triethylamine (0.07 ml). The solution was stirred at room temperature overnight under an atmosphere of dry argon. The solvent was evaporated and the crude material purified by column chromatography (silica 10% ethyl acetate and 90% dichloromethane) to give the title compound as a solid (50 mg), m.p 98-108° found: C, 73.59; H, 7.05; N, 5.06. C₃₃H₃₆N₂O₄. 0.8 H₂O requires C, 73.55; H, 7.03; N, 5.20%

### Example 87 Preparation of cis-7-(1-S-methoxycarbonyl-2-carboxyethylaminocarbonyl)-8-(1-adamantylmethylaminocarbonyl)-2,3,5,6-dibenzobicyclo[2.2.2]octane (mixture of diastereomers)

The reaction was performed essentially as in example 64 but using the alpha methyl beta benzyl diester of aspartic acid in step a. instead of the dibenzyl ester. The compound was characterised and tested as the N-methyl-D-glucamine salt, m.p 103-5° found: C, 56.56; H, 7.70; N, 4.71. C₄₁H₅₅N₃O₁₁. 5.9 H₂O requires C, 56.48; H, 7.72; N, 4.82%

### Example 88 Preparation of cis-7-(2-R-carboxypiperidinocarbonyl)-8-(1-adamantylmethylaminocarbonyl)-2,3,5,6-dibenzobicyclo[2.2.2]octane (mixture of diastereomers)

The reaction was performed essentially as in example 64 but using the benzyl ester of D-pipecolinic acid in step a. instead of the dibenzyl ester of aspartic acid. The compound was characterised and tested as the N-methyl-D-glucamine salt. Found: C, 65.13; H, 8.07; N, 5.64. C₄₂H₅₇N₃O₉. 1.5 H₂O requires C, 65.10; H, 7.81; N, 5.42%

### Example 89 Preparation of cis-7-(2-R-methoxycarbonylpiperidinocarbonyl)-8-(1-adamantylmethylaminocarbonyl)-2,3,5,6-dibenzobicyclo[2.2.2]octane (mixture of diastereomers)

The compound was prepared essentially as in example 63 but using the product of example 88 as substrate instead of cis-7-(1-S-carboxy-2-methylpropylaminocarbonyl)-8-(1-adamantylmethylaminocarbonyl)-2,3,5,6-dibenzobicyclo[2.2.2]octane (mixture of diastereomers). Found: C, 70.00; H, 7.18; N, 4.70. C₃₆H₄₂N₂O₄. 0.5 CHCl₃ requires C, 70.04; H, 6.78; N, 4.40%

### Example 90 Preparation of cis-7-(2-S-methoxycarbonyl-4-S-hydroxypyrrolidinocarbonyl)-8-(1-adamantylmethylaminocarbonyl)-2,3,5,6-dibenzobicyclo[2.2.2]octane (mixture of diastereomers)

The reaction was performed essentially as in example 46 but using the trifluoroacetate salt of the methyl ester of cis-hydroxy-L-proline instead of D-proline benzyl ester hydrochloride. Found: C, 76.36; H, 7.24; N, 4.38. C₃₅H₄₀N₂O₅. 0.9 toluene requires C, 76.25; H, 7.31; N, 4.26%

### Example 91 Preparation of cis-7-(2-S-methoxycarbonyl-4-R-hydroxypyrrolidinocarbonyl)-8-(1-adamantylmethylaminocarbonyl)-2,3,5,6-dibenzobicyclo[2.2.2]octane (mixture of diastereomers)

The reaction was performed essentially as in example 46 but using the trifluoroacetate salt of the methyl ester of trans-hydroxy-L-proline instead of D-proline benzyl ester hydrochloride. Found: C, 75.16; H, 7.32; N, 4.19. C₃₅H₄₀N₂O₉. 0.6 toluene requires C, 75.45; H, 7.24; N, 4.49%

### Example 92 Preparation of cis-7-(1-S-methoxycarbonyl-2-benzylsulphenylethylaminocarbonyl)-8-(1-adamantylmethylaminocarbonyl)-2,3,5,6-dibenzobicyclo[2.2.2]octane (diastereomer 1)

### Step a. cis-7-(1-S-methoxycarbonyl-2-benzylsulphenylethylaminocarbonyl)-8-(1-adamantylmethylaminocarbonyl)-2,3,5,6-dibenzobicyclo[2.2.2]octane (mixture of diastereomers)

The reaction was performed essentially as in example 46 but using the benzylthioether of L-cysteine methyl ester hydrochloride instead of D-proline benzyl ester hydrochloride.

### Step b. cis-7-(1-S-methoxycarbonyl-2-benzylsulphenylethylaminocarbonyl)-8-(1-adamantylmethylaminocarbonyl)-2,3,5,6-dibenzobicyclo[2.2.2]octane (diastereomer 1)

The compound prepared in step a. was separated into its component diastereomers by column chromatography (silica 15% ethyl acetate and 85% dichloromethane). The more polar material (R_{f} 0.4) was designated diastereomer 1, the title compound, m.p. 80-1° found: C, 72.93; H, 6.88; N, 4.08. C₄₀H₄₄N₂O₄S. 0.5 H₂O requires C, 73.04; H, 6.89; N, 4.25%

### Example 93 Preparation of cis-7-(1-S-methoxycarbonyl-2-benzylsulphenylethylaminocarbonyl)-8-(1-adamantylmethylaminocarbonyl)-2,3,5,6-dibenzobicyclo[2.2.2]octane (diastereomer 2)

The less polar material isolated by the chromatography (R_{f} 0.6) described in example 92 step b. was designated as diastereomer 2, m.p. 85° found: C, 74.03; H, 7.01; N, 4.38. C₄₀H₄₄N₂O₄S requires C, 74.04; H, 6.64; N, 4.32%

### Example 94 Preparation of cis-7-(1-S-carboxyethyl-(N-methyl)-aminocarbonyl)-8-(1-adamantylmethylaminocarbonyl)-2,3,5,6-dibenzobicyclo[2.2.2]octane (mixture of diastereomers)

The reaction was performed essentially as in example 64 but using the trifluoroacetate salt of the benzyl ester of N-methyl-L-alanine in step a. instead of the dibenzyl ester of aspartic acid. The compound was characterised and tested as the N-methyl-D-glucamine salt, m.p. 100-10° found: C, 64.12; H, 7.89; N, 5.71. C₄₀H₄₅N₃O₉. 1.5 H₂O requires C, 64.21; H, 7.80; N, 5.62%

### Example 95 Preparation of cis-7-(1-R-carboxyethyl-(N-methyl)-aminocarbonyl)-8-(1-adamantylmethylaminocarbonyl)-2,3,5,6-dibenzobicyclo[2.2.2]octane (mixture of diastereomers)

The reaction was performed essentially as in example 64 but using the trifluoroacetate salt of the benzyl ester of N-methyl-D-alanine in step a. instead of the dibenzyl ester of aspartic acid. The compound was characterised and tested as the N-methyl-D-glucamine salt, m.p. 105-15° found: C, 62.06; H, 7.81; N, 5.55. C₄₀H₅₅N₃O₉. 2.8 H₂O requires C, 62.18; H, 7.91; N, 5.44%

### Example 96 Preparation of cis-7-(1-S-methoxycarbonylethyl-(N-methyl)-aminocarbonyl)-8-(1-adamantylmethylaminocarbonyl)-2,3,5,6-dibenzobicyclo[2.2.2]octane (mixture of diastereomers)

The reaction was performed essentially as in example 63 but using the product of example 94 as substrate instead of cis-7-(1-S-carboxy-2-methylpropylaminocarbonyl)-8-(1-adamantylmethylaminocarbonyl)-2,3,5,6-dibenzobicyclo[2.2.2]octane (mixture of diastereomers), m.p. 96-8° found: C, 75.55; H, 7.68; N, 5.10. C₃₄H₄₀N₂O₄ requires C, 75.53; H, 7.46; N, 5.18%

### Example 97 Preparation of cis-7-(1-R-methoxycarbonylethyl-(N-methyl)-aminocarbonyl)-8-(1-adamantylmethylaminocarbonyl)-2,3,5,6-dibenzobicyclo[2.2.2]octane (mixture of diastereomers)

The reaction was performed essentially as in example 63 but using the product of example 95 as substrate instead of cis-7-(1-S-carboxy-2-methylpropylaminocarbonyl)-8-(1-adamantylmethylaminocarbonyl)-2,3,5,6-dibenzobicyclo[2.2.2]octane (mixture of diastereomers), m.p. 95-105° found: C, 75.49; H, 7.53; N, 5.24. C₃₄H₄₀N₂O₄ requires C, 75.53; H, 7.46; N, 5.18%

### Example 98 Preparation of (±)-cis-7-(pyrrolidinocarbonyl)-8-(1-adamantylmethylaminocarbonyl)-2,3,5,6-dibenzobicyclo[2.2.2]octane

The reaction was performed essentially as in example 64 but using pyrrolidine in step a. instead of the dibenzyl ester of aspartic acid, m.p. 205-7° found: C, 80.15; H, 7.77; N, 5.78. C₃₃H₃₈N₂O₂ requires C, 80.12; H, 7.74; N, 5.66%

### Example 99 Preparation of (±)-cis-7-(methylaminocarbonyl)-8-(1-adamantylmethylaminocarbonyl)-2,3,5,6-dibenzobicyclo[2.2.2]octane

The compound of example 14 (440 mg, 1 mmol) was dissolved in dichloromethane (15 ml) and diisopropylethylamine (0.52 ml) and PyBOP (520 mg) were added. The solution was stirred for 5-10 min until a clear solution was obtained. Dry methylamine gas was bubbled through the solution for 5 min until this was saturated. The solution was stirred for lh and then evaporated. The residue was taken up in ethyl acetate and washed successively with 5% aqueous potassium hydrogensulphate solution (2 x 20 ml) saturated sodium hydrogencarbonate solution (20 ml), brine (20 ml), dried, filtered and evaporated to leave a crude product which was purified by column chromatography (silica 70% dichloromethane and 30% ethyl acetate). The title compound was a white solid (240 mg), m.p. 192-3° found: C, 79.15; H, 7.72; N, 5.91. C₃₀H₃₄N₂O₂ requires C, 79.26; H, 7.53; N, 6.16%

### Example 100 Preparation of (±)-cis-7-(dimethylaminocarbonyl)-8-(1-adamantylmethylaminocarbonyl)-2,3,5,6-dibenzobicyclo[2.2.2]octane

The compound was prepared essentially as in example 99 but using dimethylamine instead of methylamine, m.p. 253-5° found: C, 78.86; H, 7.78; N, 5.67. C₃₁H₃₆N₂O₂ requires C, 78.69; H, 7.8 N, 5.92%

### Example 101 Preparation of (±)-cis-7-(ethylaminocarbonyl)-8-(1-adamantylmethylaminocarbonyl)-2,3,5,6-dibenzobicyclo[2.2.2]octane

The compound was prepared essentially as in example 99 but using ethylamine instead of methylamine, m.p. 200-1° found: C, 79.82; H, 7.67; N, 5.94. C₃₁H₃₆N₂O₂ requires C, 79.45; H, 7.74 N, 5.98%

### Example 102 Preparation of (±)-cis-7-(1-methylethylaminocarbonyl)-8-(1-adamantylmethylaminocarbonyl)-2,3,5,6-dibenzobicyclo[2.2.2]octane

The compound was prepared essentially as in example 99 but using isopropylamine instead of methylamine, m.p. 122-4° found: C, 74.80; H, 7.21; N, 5.38. C₃₂H₃₈N₂O₂. 0.4 DCM. 0.1 ethyl acetate requires C, 74.94; H, 7.56 N, 5.33%

### Example 103 Preparation of (±)-cis-7-aminocarbonyl-8-(1-adamantylmethylaminocarbonyl)-2,3,5,6-dibenzobicyclo[2.2.2]octane

The compound was prepared essentially as in example 99 but using ammonia instead of methylamine, m.p. 238-40° found: C, 78.78; H, 7.45; N, 6.41. C₂₉H₃₂N₂O₂ requires C, 79.06; H, 7.32 N, 6.36%

### Example 104 Preparation of (±)-cis-7-(2-benzyloxycarbonylethylaminocarbonyl)-8-(1-adamantylmethylaminocarbonyl)-1-cyano-2,3,5,6-dibenzobicyclo[2.2.2]octane (regioisomer 1)

The mixture of regioisomers was prepared essentially as in example 29 but using (±)-cis-8-(1-adamantylmethylaminocarbonyl)-1-cyano-2,3,5,6-dibenzobicyclo[2.2.2]octane-7-carboxylic acid instead of (±)-cis-8-(1-adamantylmethylaminocarbonyl)-2,3,5,6-dibenzobicyclo[2.2.2]octane-7-carboxylic acid. This in turn was made by reaction of 1-adamantanemethylamine with 1-cyano-2,3,5,6-dibenzobicyclo[2.2.2]octane-7,8-dicarboxylic acid anhydride essentially as in example 14. The anhydride was prepared by reaction of maleic anhydride with 9-cyanoanthracene in refluxing toluene. The regioisomers were separated by preparative HPLC (silica, ethyl acetate 15% and dichloromethane 85%). The less polar regioisomer was designated regioisomer 1, the title compound, m.p. 205-8° found: C, 76.55; H, 6.61; N, 6.68. C₄₀H₄₁N₃O₄ requires C, 76.53; H, 6.58; N, 6.69%

### Example 105 Preparation of (±)-cis-7-(2-benzyloxycarbonylethylaminocarbonyl)-8-(1-adamantylmethylaminocarbonyl)-1-cyano-2,3,5,6-dibenzobicyclo[2.2.2]octane (regioisomer 2)

The more polar regioisomer from the HPLC separation described in example 104 was designated regioisomer 2, the title compound, m.p. 104-7° found: C, 76.48; H, 6.65; N, 6.59. C₄₀H₄₁N₃O₄ requires C, 76.53; H, 6.58; N, 6.69%

### Example 106 Preparation of cis-7-(1-S-methoxycarbonylethylaminocarbonyl)-8-(neopentylaminocarbonyl)-2,3,5,6-dibenzobicyclo[2.2.2]octane (mixture of diastereomers)

### a. (±)-8-(neopentylaminocarbonyl)-2,3,5,6-dibenzobicyclo-[2.2.2]octane-7-carboxylic acid

The material was prepared essentially as in example 14 but using neopentylamine hydrochloride as substrate instead of 1-adamantylamine.

### b. cis-7-(1-S-methoxycarbonylethylaminocarbonyl)-8-(neopentylaminocarbonyl)-2,3,5,6-dibenzobicyclo[2.2.2]octane (mixture of diastereomers)

The material was prepared essentially as in example 25 but using the compound prepared in step a. above instead of (±)-cis-8-(1-adamantylmethylaminocarbonyl)-2,3,5,6-dibenzobicyclo[2.2.2]octane-7-carboxylic acid as substrate. found: C, 72.14; H, 7.32; N, 6.29. C₂₇H₃₂N₂O₄ requires C, 72.30; H, 7.19; N, 6.25%

### Example 107 Preparation of cis-7-(2-R-carboxy-pyrrolidinocarbonyl)-8-(neopentylaminocarbonyl)-2,3,5,6-dibenzobicyclo[2.2.2]octane (mixture of diastereomers)

### a. cis-7-(2-R-benzyloxycarbonylpyrrolidinocarbonyl)-8-(neopentylaminocarbonyl)-2,3,5,6-dibenzobicyclo[2.2.2]octane (mixture of diastereomers) The compound was prepared essentially as in example 46 but using the product of example 106 step a. as substrate instead of (±)-cis-8-(1-adamantylmethylaninocarbonyl)-2,3,5,6-dibenzobicyclo[2.2.2]octane-7-carboxylic acid.

### b. cis-7-(2-R-carboxypyrrolidinocarbonyl)-8-(neopentylaminocarbonyl)-2,3,5,6-dibenzobicyclo[2.2.2]octane (mixture of diastereomers)

The reaction was performed essentially as in example 48 but using the product of example 107 step a. as substrate instead of the product of example 46. The compound was characterised and tested as the N-methyl-D-glucamine salt, m.p. 105-15° found: C, 57.98; H, 7.89; N, 6.07. C₃₅H₄₉N₃O₉. 4.0 H₂O requires C, 57.75; H, 7.89; N, 5.77%

### Example 108 Preparation of 7-(1-adamantylmethylaminocarbonyl)-2,3,5,6-dibenzobicyclo[2.2.2]oct-7,8-ene

### a. 7-carboxy-2,3,5,6-dibenzobicyclo[2.2.2]oct-7,8-ene

7-(methoxycarbonyl)-2,3,5,6-dibenzabicyclo[2.2.2]act-7,8-ene (prepared as in J.C.S. Perkin I, 1984, 779) (0.5 g, 1.9 mmol) was dissolved in ethanol (20 ml) and sodium hydroxide (0.5 g) was added along with water (2 ml). The solution was stirred and refluxed for 1.5 h. The hot solution was poured onto 2M HCl (50 ml). The white precipitate formed was filtered off, washed with water and dried at 50° in vacuo. This compound was used without further purification.

### b. 7-(1-adamantylmethylaminocarbonyl)-2,3,5,6-dibenzobicyclo-[2.2.2]oct-7,8-ene

The acid prepared in step a. above (248 mg, 1 mmol) was dissolved in dry dichloromethane (10 ml) and diisopropylamine (0.52 g, 3 mmol) and PyBOP (0.52 g, 1 mmol) were added. After stirring at room temperature for 5 min, 1-adamantylmethylamine (180 mg) was added. After stirring for a further 30 min whereupon the reaction mixture was evaporated. The residue was taken up in ethyl acetate and washed successively with 5% aqueous potassium hydrogensulphate solution (2 x 20 ml), saturated sodium hydrogencarbonate solution (20 ml), brine (20 ml), dried, filtered and evaporated to leave a crude product which was recrystallised from toluene. The white solid was dried in vacuo, m.p. 254-5° found: C, 81.28; H, 7.81; N, 3.40. C₂₈H₂₉NO. H₂O requires C, 81.32; H, 7.50; N, 3.39%

### Example 109 Preparation of (±)-7-(1-adamantylmethylaminocarbonyl)-2,3,5,6-dibenzobicyclo[2.2.2]octane

### a. (±)-2,3,5,6-dibenzobicyclo[2.2.2]octane-7-carboxylic acid

7-(methoxycarbonyl)-2,3,5,6-dibenzobicyclo[2.2.2]octane (prepared as in US patent 5,055,468) (20 g, 80 mmol) was dissolved in methanol (220 ml) and potassium hydroxide (40 g) was added along with water (40 ml). The solution was stirred and refluxed for 4h. The solution was cooled, filtered through charcoal and treated with concentrated HCl. The buff precipitate formed was filtered off, washed with water and recrystallised from hot benzene. This compound was used without further purification.

### b. (±)-7-(1-adamantylmethylaminocarbonyl)-2,3,5,6-dibenzobicyclo[2.2.2]octane

(±)-2,3,5,6-dibenzobicyclo[2.2.2]octane-7-carboxylic acid (prepared in step a. above) (1.0 g) was heated with thionyl chloride (5 ml) and DMF (2 drops) at reflux for 30 min. On cooling and evaporation the pale yellow acid chloride was isolated.

The acid chloride (267 mg, 1.0 mmol) was dissolved in dry dichloromethane (5 ml) and and added with stirring to a solution of 1-adamantanemethylamine (182 mg, 1.1 mmol) and triethylamine (0.3 ml). After 30 min the solution was washed successively with 2M HCl and brine, dried, filtered and evaporated to leave a solid which was recrystallised from toluene (253 mg), m.p. 220-1° found: C, 84.88; H, 7.81; N, 3.39. C₂₈H₃₁NO requires C, 84.81; H, 7.63; N, 3.39%

### Example 110 Preparation of (±)-7-(1-adamantylmethoxycarbonyl)-2,3,5,6-dibenzobicyclo[2.2.2]octane

The material was prepared essentially as in example 109 except that 1-adamantanemethanol was used instead of 1-adamantanemethylamine in step b, m.p. 152-3° found: C, 84.42; H, 7.65. C₂₈H₃₀O₂ requires C, 84.38; H, 7.59%

### Example 111 Preparation of cis-7-(1-S-methoxycarbonylethylaminocarbonyl)-8-(1-adamantylmethoxycarbonyl)-2,3,5,6-dibenzobicyclo[2.2.2]octane (mixture of diastereomers)

The material was prepared essentially as in example 25 but using the compound prepared in example 13 instead of (±)-cis-8-(1-adamantylmethylaminocarbonyl)-2,3,5,6-dibenzobicyclo[2.2.2]octane-7-carboxylic acid as substrate, m.p. 75-7° found: C, 74.27; H, 7.36; N, 2.80. C₃₃H₃₇NO₅. 0.06 mol DCM requires C, 74.53; H, 7.02; N, 2.62%

### Example 112 Preparation of cis-7-(1-R-methoxycarbonylethylaminocarbonyl)-8-(1-adamantylmethoxycarbonyl)-2,3,5,6-dibenzobicyclo[2.2.2]octane (mixture of diastereomers)

The material was prepared essentially as in example 111 but using D-alanine methyl ester hydrochloride instead of L-alanine methyl ester hydrochloride as substrate, m.p. 85-7° found: C, 74.38; H, 7.29; N, 2.75. C₃₃H₃₇NO₅. 0.05 mol DCM requires C, 74.63; H, 7.03; N, 2.63%

### Example 113 Preparation of (±)-cis-7-(2-benzyloxycarbonylethylaminocarbonyl)-8-(1-adamantylmethylaminocarbonyl)-1,4-dimethyl-2,3,5,6-dibenzobicyclo[2.2.2]octane

The compound was prepared essentially as in example 29 but using (±)-cis-8-(1-adamantylmethylaminocarbonyl)-1,4-dimethyl-2,3,5,6-dibenzobicyclo[2.2.2]octane-7-carboxylic acid instead of (±)-cis-8-(1-adamantylmethylaminocarbonyl)-2,3,5,6-dibenzobicyclo-[2.2.2]octane-7-carboxylic acid. This in turn was made by reaction of 1-adamantanemethylamine with 1,4-dimethyl-2,3,5,6-dibenzobicyclo[2.2.2]octane-7,8-dicarboxylic acid anhydride essentially as in example 14. The anhydride was prepared by reaction of maleic anhydride with 9,10-dimethylanthracene in refluxing toluene. m.p. 170-3° found: C, 77.89; H, 7.49; N, 4.49. C₄₁H₄₆N₂O₄ requires C, 78.06; H, 7.49; N, 4.44%

### Example 114 Preparation of (±)-cis-7-(1-S-methoxycarbonylethylaminocarbonyl)-8-(1-adamantylmethylaminocarbonyl)-1-nitro-2,3,5,6-dibenzobicyclo[2.2.2]octane (diastereomer 1)

The compound was prepared essentially as in example 25 but using (±)-cis-8-(1-adamantylmethylaminocarbonyl)-1-nitro-2,3,5,6-dibenzobicyclo[2.2.2]octane-7-carboxylic acid instead of (±)-cis-8-(1-adamantylmethylaminocarbonyl)-2,3,5,6-dibenzo-bicyclo[2.2.2]octane-7-carboxylic acid . This in turn was made by reaction of 1-adamantanemethylamine with l-nitro-2,3,5,6-dibenzobicyclo-[2.2.2]octane-7,8-dicarboxylic acid anhydride essentially as in example 14. The anhydride was prepared by reaction of maleic anhydride with 9-nitroanthracene in refluxing toluene. The final mixture of diastereomers was separated into three components by HPLC (silica gradient elution of 5% ethyl acetate and 95% dichloromethane to 15% ethyl acetate and 85% dichloromethane). The least polar fraction was designated diastereomer 1. Found: C, 68.99; H, 6.78; N, 7.24. C₃₃H₃₇N₃O₆ requires C, 69.33; H, 6.52; N, 7.35%

### Example 115 Preparation of (±)-cis-7-(1-S-methoxycarbonylethylaminocarbonyl)-8-(1-adamantylmethylaminocarbonyl)-1-nitro-2,3,5,6-dibenzobicyclo[2.2.2]octane (diastereomer 2)

The second least polar fraction from the HPLC separation described in example 114 was designated diastereomer 2. Found: C, 69.43; H, 6.69; N, 7.37. C₃₃H₃₇N₃O₆ requires C, 69.33; H, 6.52; N, 7.35%

### Example 116 Preparation of (±)-cis-7-(1-S-methoxycarbonylethylaminocarbonyl)-8-(1-adamantylmethylaminocarbonyl)-1-nitro-2,3,5,6-dibenzobicyclo[2.2.2]octane (diastereomer 3)

The most polar fraction from the HPLC separation described in example 114 was designated diastereomer 3,
found: C, 69.21; H, 6.80; N, 7.22. C₃₃H₅₇N₃O₆ requires C, 69.33; H, 6.52; N, 7.35%

### Example 117 Preparation of cis-7-(1-S-methoxycarbonyl-2-(3-indolyl)ethylaminocarbonyl)-8-(neopentylaminocarbonyl)-2,3,5,6-dibenzobicyclo[2.2.2]octane (mixture of diastereomers)

The compound was prepared essentially as in example 54 but using the product of example 106 step a. as substrate instead of (±)-cis-8-(1-adamantylmethylaminocarbonyl)-2,3,5,6-dibenzobicyclo[2.2.2]octane-7-carboxylic acid. Found: C, 73.81; H, 6.49; N, 7.23. C₃₅H₃₇N₃O₄. 0.25 H₂O requires C, 73.98; H, 6.65; N, 7.39%

### Example 118 Preparation of cis-7-(1-S-carboxy-2-(3-indolyl)ethylaminocarbonyl)-8-(neopentylaminocarbonyl)-2,3,5,6-dibenzobicyclo[2.2.2]octane (mixture of diastereomers)

The compound was prepared essentially as in example 107 but using the benzyl ester of L-tryptophan as substrate in step a. as substrate instead of the benzyl ester of D-proline. The compound was characterised and tested as the N-methyl-D-glucamine salt Found: C, 65.91; H, 7.01; N, 7.31. C₄₁H₅₂N₄O₉ requires C, 66.11; H, 7.04; N, 7.52% ,

### Example 119 Preparation of cis-7-(2-R-(carboxymethylaminocarbonyl)pyrrolidinocarbonyl)-8-(1-adamantylmethylaminocarbonyl)-2,3,5,6-dibenzobicyclo[2.2.2]octane (mixture of diastereomers)

The reaction was performed essentially as in example 64 but using the benzyl ester of D-prolylglycine in step a. instead of the dibenzyl ester of aspartic acid. The compound was characterised and tested as the N-methyl-D-glucamine salt. Found: C, 61.52; H, 7.52; N, 6.81. C₄₃H₅₈N₄O₁₀. 2.6 H₂O requires C, 61.65; H, 7.60; N, 6.69%

### Example 120 Preparation of cis-7-(2-R-(carboxymethylaminocarbonyl)pyrrolidinocarbonyl)-8-(1-adamantylmethylaminocarbonyl)-2,3,5,6-dibenzobicyclo[2.2.2]octane (diastereomer 1)

The benzyl este- intermediate isolated after example 119 step a. was separated into its constituent diastereomers by recrystallization from ethyl acetate. The mother liquors on concentration gave a benzyl ester which on hydrogenolysis gave the diastereomer of this example. The compound was characterised and tested as the N-methyl-D-glucamine salt. Found: C, 65.12; H, 7.40; N, 6.95. C₄₃H₅₈N₄O₁₀ requires C, 65.30; H, 7.39; N, 7.08%

### Example 121 Preparation of cis-7-(2-R-(carboxymethylaminocarbonyl)pyrrolidinocarbonyl)-8-(1-adamantylmethylaminocarbonyl)-2,3,5,6-dibenzobicyclo[2.2.2]octane (diastereomer 2)

The crystalline material from the recrystallisation described in example 120 on hydrogenolysis gave the diastereomer of this example. The compound was characterised and tested as the N-methyl-D-glucamine salt. Found: C, 61.54; H, 7.75; N, 6.34. C₄₃H₅₈N₄O₁₀. 2.9 H₂O requires C, 61.29; H, 7.62; N, 6.64%

### Example 122 Preparation of cis-7-(2-R-(carboxyethylaminocarbonyl)pyrrolidinocarbonyl)-8-(1-adamantylmethylaminocarbonyl)-2,3,5,6-dibenzobicyclo[2.2.2]octane (mixture of diastereomers)

The reaction was performed essentially as in example 64 but using the benzyl ester of D-prolyl-beta-alanine in step a. instead of the dibenzyl ester of aspartic acid. The compound was characterised and tested as the N-methyl-D-glucamine salt. Found: C, 63.14; H, 7.78; N, 6.60. C₄₄H₆₀N₄O₁₀. 1.9 H₂O requires C, 63.02; H, 7.66; N, 6.68%

### Example 123 Preparation of cis-7-(2-R-(methoxycarbonylmethylaminocarbonyl)pyrrolidinocarbonyl)-8-(1-adamantylmethylaminocarbonyl)-2,3,5,6-dibenzobicyclo[2.2.2]octane (mixture of diastereomers)

The compound was prepared essentially as in example 63 but using the compound of example 119 as substrate instead of the compound of example 62. Found: C, 69.77; H, 7.08; N, 6.58. C₃₇H₄₃N₃O₅. 1.4 H₂O requires C, 69.97; H, 7.27; N, 6.62%

### Example 124 Preparation of cis-7-(2-R-(methoxycarbonylethylaminocarbonyl)pyrrolidinocarbonyl)-8-(1-adamantylmethylaminocarbonyl)-2,3,5,6-dibenzobicyclo[2.2.2]octane (mixture of diastereomers)

The compound was prepared essentially as in example 63 but using the compound of example 122 as substrate instead of the compound of example 62. Found: C, 71.15; H, 7.40; N, 6.56. C₃₈H₄₅N₃O₅. H₂O requires C, 71.13; H, 7.38; N, 6.54%

### Example 125 Preparation of cis-7-(1-S-(carboxyethylaminocarbonyl)ethylaminocarbonyl)-8-(1-adamantylmethylaminocarbonyl)-2,3,5,6-dibenzobicyclo[2.2.2]octane (mixture of diastereomers)

The reaction was performed essentially as in example 64 but using the benzyl ester of L-alanyl-beta-alanine in step a. instead of the dibenzyl ester of aspartic acid. The compound was characterised and tested as the N-methyl-D-glucamine salt. Found: C, 60.56; H, 7.74; N, 6.53. C₄₂H₅₈N₄O₁₀. 3.1 H₂O requires C, 60.40; H, 7.75; N, 6.71%

### Example 126 Preparation of cis-7-(1-S-(methoxycarbonylethylaminocarbonyl)ethylaminocarbonyl)-8-(1-adamantylmethylaminocarbonyl)-2,3,5,6-dibenzobicyclo[2.2.2]octane (mixture of diastereomers)

The compound was prepared essentially as in example 63 but using the compound of example 125 as substrate instead of the compound of example 62. Found: C, 69.51; H, 7.39; N, 6.49. C₃₆H₄₃N₃O₅. 1.4 H₂O requires C, 69.35; H, 7.41; N, 6.73%

### Example 127 Preparation of cis-7-(1-S-(methylaminocarbonyl)-ethylaminocarbonyl)-8-(1-adamantylmethylaminocarbonyl)-2,3,5,6-dibenzobicyclo[2.2.2]octane (mixture of diastereomers)

The compound was prepared essentially as in example 25 but using the trifluoroacetate salt of N-methyl-L-alaninamide instead of L-alanine methyl ester hydrochloride. Found: C, 72.12; H, 7.37; N, 7.50. C₃₃H₃₉N₃O₃. 1.2 H₂O requires C, 72.37; H, 7.63; N, 7.67%

### Example 128 Preparation of cis-7-(1-S-(methoxycarbonyl)-ethylaminocarbonyl)-8-(1-RS-(1-adamantyl)ethylaminocarbonyl)-2,3,5,6-dibenzobicyclo[2.2.2]octane (mixture of diastereomers A)

### a. 8-(1-(1-adamantyl)ethylaminocarbonyl)-2,3,5,6-dibenzobicyclo[2.2.2]octane-7-carboxylic acid (mixture of diastereomers)

This compound was prepared essentially as in example 14 but using 1-RS-(1-adamantyl)ethylamine (prepared as described in EP 178668) as substrate instead of 1-adamantanemethylamine.

### b. cis-7-(1-S-(methoxycarbonyl)ethylaminocarbonyl)-8-(1-RS-(1-adamantyl)ethylaminocarbonyl)-2,3,5,6-dibenzobicyclo[2.2.2]octane (mixture of diastereomers A)

This was prepared essentially as in example 25 but using the product of step a. instead of the product of example 14. The mixture of four compounds thus produced was separated into two pairs by use of preparative MPLC (silica 35% ethyl acetate and 65% hexane). The least polar pair were designated as the product of this example. found: C, 74.26; H, 7.60; N, 4.86. C₃₄H₄₀N₂O₄. 0.5 H₂O requires C, 74.29; H, 7.52; N, 5.10%

### Example 129 Preparation of cis-7-(1-S-(methoxycarbonyl)-ethylaminocarbonyl)-8-(1-RS-(1-adamantyl)ethylaminocarbonyl)-2,3,5,6-dibenzobicyclo[2.2.2]octane (mixture of diastereomers B)

The most polar pair of compounds isolated after the MPLC procedure described in example 128 were designated mixture B, the compounds of this example. Found: C, 74.01; H, 7.69; N, 4.90. C₃₄H₄₀N₂O₄. 0.5 H₂O requires C, 74.29; H, 7.52; N, 5.10%

### Example 130 Preparation of cis-7-(1-S-(ethylcarbonyl)ethylaminocarbonyl)-8-(1-adamantylmethylaminocarbonyl)-2,3,5,6-dibenzobicyclo[2.2.2]octane (mixture of diastereomers)

The compound of example 75 (0.37 g, 0.74 mmol) was dissolved in THF under an atmosphere of argon and cooled to 0°. A 1M solution of ethyl magnesium bromide in THF (3 ml) was added and the solution stirred for a further 2h before being allowed to warm to room temperature for overnight stirring. The reaction was quenched with 2M hydrochloric acid and after evaporation the the product was dissolved in ethyl acetate and washed succesively with saturated aqueous sodium hydrogencarbonate and with brine. The organic layer was dried, filtered and evaporated to leave the crude product which was purified by column chromatography (silica 90% dichloromethane and 10% ethyl acetate). Yield 0.19 g, 50%, m.p 105-8°. Found: C, 77.87; H, 7.61; N, 5.22. C₃₄H₄₀N₂O₃ requires C, 77.83; H, 7.68; N, 5.34%

### Example 131 Preparation of (±)-7-(methoxycarbonylmethyl)-cis-7-carboxy-8-(1-adamantylmethylaminocarbonyl)-2,3,5,6-dibenzobicyclo[2.2.2]octane

### a. Diels-Alder adduct of the methyl ester of aconitic anhydride and anthracene

The methyl ester of aconitic anhydride (10.2 g, 60 mmol) was dissolved in dry dichloromenthane (200 ml) and anthracene (7.12 g, 40 mmol) was added followed by anhydrous aluminium chloride (9.0 g, 60 mmol). The solution was stirred at room temperature overnight and then poured onto a mixture of ice and hydrochloric acid. The organic layer was separated and dried, filtered and evaporated to leave an orange oil Which was recrystallised from toluene after treatment with activated charcoal. The product, a buff solid, was dried in air (9.01 g) and used in the next step.

### b . (±)-7-(methoxycarbonylmethyl)-cis-7-carboxy-8-(1-adamantylmethylaminocarbonyl)-2,3,5,6-dibenzobicyclo[2.2.2]octane

The material was prepared essentially as in example 14 but using the material prepared in step a. above instead of (±)-2,3,5,6-dibenzobicyclo[2.2.2]octane-7,8-dicarboxylic anhydride. The compound was characterised and tested as the N-methyl-D-glucamine found: C, 65.72; H, 7.61; N, 3.83. C₃₉H₅₂N₂O₁₀ requires C, 66.08; H, 7.39; N, 3.95%

### Example 132 Preparation of cis-7-(1-S-(methoxycarbonyl)-ethylaminocarbonyl)-8-(2-adamantylmethylaminocarbonyl)-2,3,5,6-dibenzobicyclo[2.2.2]octane (mixture of diastereomers)

The compound was prepared essentially as in example 25 but using the compound of example 39 instead of 8-(1-adamantylmethylaminocarbonyl)-2,3,5,6-dibenzobicyclo[2.2.2]octane-7-carboxylic acid as substrate, m.p. 105-110°. Found: C, 74.13; H, 7.17; N, 4.85. C₃₃H₃₈N₂O₄ .0.5 H₂O requires C, 73.99; H, 7.33; N, 5.23%

### Example 133 Preparation of cis-7-(2-R-carboxypyrrolidinocarbonyl)-8-(2-adamantylmethylaminocarbonyl)-2,3,6-dibenzobicyclo-[2.2.2]octane (mixture of diastereomers)

The benzyl ester of the compound was prepared essentially as in example 46 but using the compound of example 39 instead of 8-(1-adamantylmethylaminocarbonyl)-2,3,5,6-dibenzobicyclo[2.2.2joctane-7-carboxylic acid as substrate. The hydrogenolysis was performed as described in example 48. m.p. 105-110°. The compound was further characterised and tested as its N-methyl-D-glucamine salt found: C, 64.86; H, 7.79; N, 5.19. C₄₁H₅₅N₃O₄ .1.6 H₂O requires C, 64.56; H, 7.69; N, 5.51%

### Example 134 Preparation of 8-(1-adamantylmethylaminocarbonyl)-2,3,5,6-dibenzobicyclo[2.2.2]oct-7-ene-7-carboxylic acid

### a. dimethyl-2,3,5,6,-dibenzobicyclo[2.2.2.]oct-7-ene-7,8-dicarboxylate

The Diels-Alder reaction between anthracene (10g, 0.06 mol) and dimethyl acetylenedicarboxylate (8.3 ml, 0.07 mol) was performed essentially as in step a. of example 1 with the exeption that the reactants were refluxed for 24 hours.

### b. 2,3,5,6,-dibenzobicyclo[2.2.2.]oct-7-ene-7,8-dicarboxylic acid

To a solution of potassium hydroxide (1.05 g, 18.8 mmol) in water (30 ml) was added the solution of the product of step a. (2.0 g, 6.2 mmol) in dioxan (10 ml). The reaction mixture was heated to reflux for 20 mins, cooled to room temperature and diluted with 2N hydrochloric acid. The precipitated solid was filtered, washed with water and dried (1.42 g, 78%).

### c. 2,3,5,6,-dibenzobicyclo[2.2.2.]oct-7-ene-7,8-dicarboxylic acid anhydride

A mixture of the product of step b (1.4 g, 4.8 mmol and acetic anhydride (36 ml) was heated at reflux for 45 mins. The solvent was evaporated and the residue was triturated with diethyl ether to afford white solid (0.73 g, 55%).

### d. 8-(1-adamantylmethylaminocarbonyl)-2,3,5,6-dibenzobicyclo[2.2.2]oct-7-ene-7-carboxylic acid

This was performed essentially as in example 14 using the product of step c above as substrate instead of 2,3,5,6-dibenzobicyclo[2.2.2]oct-7,8-dicarboxylic anhydride. The compound was characterised and tested as the N-methyl-D-glucamine salt. Found: C, 67.79; H, 7.51; N, 4.33. C₃₆H₄₆N₂O₈ requires C, 68.12; H, 7.30; N, 4.41%

### Example 135 Preparation of cis-7-(1-S-methoxycarbonyl-ethylaminocarbonyl)-8-(1-adamantylmethylaminocarbonyl)-2,3,5,6-dibenzobicyclo[2.2.2]oct-7-ene

This was performed essentially as in example 25 using the product of example 134 as substrate instead of (±)-cis-8-(1-adamantylmethylaminocarbonyl)-2,3,5,6-dibenzobicyclo[2.2.2]oct-7-carboxylic acid found: C, 75.45; H, 6.79; N, 5.26. C₃₃H₃₆N₂O₄ requires C, 75.55; H, 6.92; N, 5.34%

### Example 136 Preparation of cis-7-(2-R-(carboxymethylaminocarbonyl)pyrrolidinocarbonyl)-8-(1-adamantylmethylaminocarbonyl)-2,3,5,6-dibenzobicyclo[2.2.2]oct-7-ene

This was performed essentially as in example 119 using the product of example 134 as substrate instead of 8-(1-adamantylmethylaminocarbonyl)-2,3,5,6-dibenzobicyclo[2.2.2]-7-carboxylic acid. The compound was tested as the N-methyl-D-glucamine salt.

### Example 137 Preparation of cis-7-(2-R-carboxy-pyrrolidinocarbonyl)-8-(1-adamantylmethylaminocarbonyl)-1,4-difluoro-2,3,5,6-dibenzobicyclo[2.2.2]octane (mixture of diastereomers)

### a. Diels alder reaction

This was performed essentially as in step a. of example 1 using 9,10-difluoroanthracene (prepared as in J.Org.Chem., 1989, 54, 1018) as substrate instead of anthracene.

### b. (±)-cis-8-(1-adamantylmethylaninocarbonyl)-1,4-difluoro-2,3,5,6-dibenzobicyclo[2.2.2]octane-7-carboxylic acid

This was performed essentially as in example 14 using the product of step a. above as substrate instead of 2,3,5,6-dibenzobicyclo[2.2.2]oct-7,8-dicarboxylic anhydride

### c. cis-7-(2-R-benzyloxycarbonylpyrrolidinocarbonyl)-8-(1-adamantylmethylaminocarbonyl)-1,4-difluoro-2,3,5,6-dibenzobicyclo[2.2.2]octane (mixture of diastereomers)

This was performed essentially as in example 46 using the product of step b above as substrate instead of (±)-cis-8-(1-adamantylmethylaminocarbonyl)-2,3,5,6-dibenzobicyclo[2.2.2]oct-7-carboxylic acid

### d. cis-7-(2-R-carboxy-pyrrolidinocarbonyl)-8-(1-adamantylmethylaminocarbonyl)-1,4-difluoro-2,3,5,6-dibenzobicyclo[2.2.2]octane (mixture of diastereomers)

This was performed essentially as in example 48 using the product of step c above as substrate instead of the product of example 46.

The compound was characterised and tested as the N-methyl-D-glucamine salt. Found: C, 57.48; H, 7.43; N, 5.28. C₄₁H₅₃F₂N₃O₉. 4.5H₂O requires C, 57.82; H, 7.35; N, 4.93%

### Example 138 Preparation of cis-7-(2-R-carboxy-4-R-hydroxypyrrolidinocarbonyl)-8-(2-adamantylaminocarbonyl)-2,3,5,6-dibenzobicyclo[2.2.2]octane (mixture of diastereomers)

### a. (±)-cis-8-(2-adamantylaminocarbonyl)-2,3,5,6-dibenzobicyclo[2.2.2]octane-7-carboxylic acid

This was prepared essentially as in example 11 using 2-adamantamine as substrate instead of 1-adamantamine.

### b. cis-7-(2-R-benzyloxycarbonyl-4-R-hydroxy-pyrrolidinocarbonyl)-8-(2-adamantylaminocarbonyl)-2,3,5,6-dibenzobicyclo[2.2.2]octane (mixture of diastereomers)

This was performed essentially as in example 46 using the product of step a. above as substrate instead of cis-8-(1-adamantylmethylaminocarbonyl)-2,3,5,6-dibenzobicyclo[2.2.2]oct-7-carboxylic acid

### c. 7-(2-R-carboxy-4-R-hydroxy-pyrrolidinocarbonyl)-8-(2-adamantylaminocarbonyl)-2,3,5,6-dibenzobicyclo[2.2.2]octane (mixture of diastereomers)

This was performed essentially as in example 48 using the product of step b above as substrate instead of the product of example 46. The compound was characterised and tested as the N-methyl-D-glucamine salt. Found: C, 60.86; H, 7.40; N, 5.31. C₄₀H₅₃N₃O₁₀. 2.9H₂O requires C, 60.97; H, 7.52; N, 5.33%

### Example 139 Preparation of cis-7-(2-S-carboxypiperidinocarbonyl)-8-(1-adamantylmethylaminocarbonyl)-2,3,5,6 6-dibenzobicyclo[2.2.2]octane (mixture of diastereomers)

The reaction was performed essentially as in example 64 but using the benzyl ester of L-pipecolinic acid in step a. instead of the dibenzyl ester of aspartic acid. The compound was further characterised and tested as the N-methyl-D-glucamine salt. Found: C, 64.39; H, 7.97; N, 5.28. C₄₂H₅₇N₃O₉. 2.0H₂O requires C, 64.35; H, 7.84; N, 5.36%

### Example 140 Preparation of cis-7-(4-carboxypiperidinocarbonyl)-8-(1-adamantylmethylaminocarbonyl)-2,3,5,6-dibenzobicyclo[2.2.2]octane (mixture of diastereomers)

the reaction was performed essentially as in example 64 but using the benzyl ester of 4-carboxypiperidine in step a. instead of the dibenzyl ester of aspartic acid. The compound was further characterised and tested as the N-methyl-D-glucamine salt. Found: C, 64.29; H, 8.05; N, 5.14. C₄₂H₅₇N₃O₉. 2.0H₂O requires C, 64.35; H, 7.84; N, 5.36%

### Example 141 Preparation of cis-7-(2-S-(carboxymethyl)pyrrolidinocarbonyl)-8-(1-adamantylmethylaminocarbonyl)-2,3,5,6-dibenzobicyclo[2.2.2]octane (mixture of diastereomers)

The reaction was performed essentially as in example 64 but using the benzyl ester of 2-S-carboxymethylpyrrolidine (prepared as in WO 92/00295) in step a. instead of the dibenzyl ester of aspartic acid, [α]^{D}= -22.0° (c= 1.0 in methanol) . The compound was further characterised and tested as the N-methyl-D-glucamine salt. Found: C, 61.76; H, 7.89; N, 5.31. C₄₂H₅₇N₃O₉. 3.7H₂O requires C, 61.91; H, 7.97; N, 5.16%

### Example 142 Preparation of cis-7-(2-R-(carboxymethyl)pyrrolidinocarbonyl)-8-(1-adamantylmethylaminocarbonyl)-2,3,5,6-dibenzobicyclo[2.2.2]octane (mixture of diastereomers)

The reaction was performed essentially as in example 64 but using the benzyl ester of 2-R-carboxymethylpyrrrolidine (prepared as in WO 92/00295) in step a. instead of the dibenzyl ester of aspartic acid, [α]^{D}= +18.0° (c= 1.0 in methanol) . The compound was further characterised and tested as the N-methyl-D-glucamine salt. Found: C, 59.01; H, 8.12; N, 5.49. C₄₂H₅₇N₃O₉. 5.9H₂O requires C, 59.07; H, 8.12; N, 4.92%

### Example 143 Preparation of cis-7-(2-S-methoxycarbonylmethyl)pyrrolidinocarbonyl)-8-(1-adamantylmethylaminocarbonyl)-2,3,5,6-dibenzobicyclo[2.2.2]octane (mixture of diastereomers)

The compound was prepared essentially as in example 63 using the product of example 141 as substrate instead of cis-7-(1-S-carboxyl-2-methylpropylaminocarbonyl)-8-(1-adamantylmethylaminocarbonyl)-2,3,5,6-dibenzobicyclo[2.2.2]octane, [α]^{D}= -21.0° (c=2.0 in CHCl3). Found: C, 75.80; H, 7.49; N, 4.62. C₃₆H₄₂N₂O₄. 0.3 H₂O requires C, 75.62; H, 7.50; N, 4.90%

### Example 144 Preparation of cis-7-(2-R-(methoxycarbonylmethyl)pyrrolidinocarbonyl)-8-(1-adamantylmethylaminocarbonyl)-2,3,5,6-dibenzobicyclo[2.2.2]octane (mixture of diastereomers)

The compound was prepared essentially as in example 63 using the product of example 142 as substrate instead of cis-7-(1-S-carboxyl-2-methylpropylaminocarbonyl)-8-(1-adamantylmethylaminocarbonyl)-2,3,5,6-dibenzobicyclo[2.2.2]octane, [α]^{D}= +18.0° (c=2.0 in CHCl₃). Found: C, 76.48; H, 7.46; N, 5.05. C₃₆H₄₂N₂O₄ requires C, 76.30; H, 7.47; N, 4.94%

### Example 145 Preparation of cis-7-(2R-(1S-carboxyethylaminocarbonyl)pyrrolidinocarbonyl)-8-(1-adamantylmethylaminocarbonyl)-2,3,5,6-dibenzobicyclo[2.2.2]octane (diastereomer 1)

The compound was prepared essentially as in example 64 but using the benzyl ester of D-prolyl-L-alanine in step a. instead of the dibenzyl ester of aspartic acid. The diastereomers were separated at the benzyl ester stage by column chromatography (silica 60% dichloromethane and 40% ethyl acetate). The compound with the higher R_{f} was converted to the title compound by hydrogenation. The compound was tested as the N-methyl-D-glucamine salt.

### Example 146 Preparation of cis-7-(2R-(1S-carboxyethylaminocarbonylmethyl)pyrrolidinocarbonyl)-8-(1-adamantylmethylaminocarbonyl)-2,3,5,6-dibenzobicyclo[2.2.2]octane (diastereomer 2)

The compound was prepared essentially as in example 145 but using the compound with lower R_{f} after diastereomer separation in the final hydrogenation step. The compound was tested as the N-methyl-D-glucamine salt.

### Example 147 Preparation of cis-7-(2R-(1R-carboxyethylaminocarbonyl)pyrrolidinocarbonyl)-8-(1-adamantylmethylaminocarbonyl)-2,3,5,6-dibenzobicyclo[2.2.2]octane (diastereomer 1)

The compound was prepared essentially as in example 64 but using the benzyl ester of D-prolyl-D-alanine in step a. instead of the dibenzyl ester of aspartic acid. The diastereomers were separated at the benzyl ester stage by column chromatography (silica 60% dichloromethane and 40% ethyl acetate). The compound with the higher R_{f} was converted to the title compound by hydrogenation. The compound was further characterised and tested as the N-methyl-D-glucamine salt. Found: C, 59.62; H, 8.06; N, 6.23. C₄₄H₆₀N₄O₁₀ 4.7H₂O requires C, 59.45; H, 7.86; N, 6.30%

### Example 148 Preparation of cis-7-(2R-(lR-carboxyethylaminocarbonyl)pyrrolidinocarbonyl)-8-(1-adamantylmethylaminocarbonyl)-2,3,5,6-dibenzobicyclo[2.2.2]octane (diastereomer 2)

The compound was prepared essentially as in example 147 but using the compound with lower R_{f} after diastereomer separation in the final hydrogenation step. The compound was tested as the N-methyl-D-glucamine salt.

### Example 149 Preparation of cis-7-(2R-carboxy-4-S-hydroxypyrrolidinocarbonyl)-8-(1-adamantylmethylaminocarbonyl)-2,3,5,6-dibenzobicyclo[2.2.2]octane (mixture of diastereomers)

The compound was prepared essentially as in example 64 but using the benzyl ester of trans hydroxy-D-proline in step a. instead of the dibenzyl ester of aspartic acid. The compound was further characterised and tested as the N-methyl-D-glucamine salt. Found: C, 65.63; H, 7.48; N, 5.38. C₄₁H₅₅N₃O₁₀ requires C, 65.67; H, 7.39; N, 5.38%

### Example 150 Preparation of cis-7-(3-carboxy-pyrrolidinocarbonyl)-8-(1-adamantylmethylaminocarbonyl)-2,3,5,6-dibenzobicyclo[2.2.2]octane (mixture of diastereomers)

The compound was prepared essentially as in example 64 but using the benzyl ester of 3-carboxypyrrolidine (prepared as in WO 92/00295) in step a. instead of the dibenzyl ester of aspartic acid. The compound was further characterised and tested as the N-methyl-D-glucamine salt. Found: C, 65.33; H, 7.67; N, 5.48. C₄₁H₅₅N₃O₉. 1.4 H₂O requires C, 65.27; H, 7.65; N, 5.57%

### Example 151 Preparation of cis-7-(3-methoxycarbonylpyrrolidinocarbonyl)-8-(1-adamantylmethylaminocarbonyl)-2,3,5,6-dibenzobicyclo[2.2.2]octane (mixture of diastereomers)

The compound was prepared essentially as in example 63 using the product of example 150 as substrate instead of cis-7-(1-S-carboxyl-2-methylpropylaminocarbonyl)-8-(1-adamantylmethylaminocarbonyl)-2,3,5,6-dibenzobicyclo[2.2.2]octane. Found: C, 75.83; H, 7.41; N, 5.08. C₃₅H₄₀N₂O₄ requires C, 76.06; H, 7.29; N, 5.07%

### Example 152 Preparation of cis-7-(3-(+)-ethoxycarbonylpiperidinocarbonyl)-8-(1-adamantylmethylaminocarbonyl)-2,3,5,6-dibenzobicyclo[2.2.2]octane (mixture of diastereomers)

The compound was prepared essentially as in example 64 but using (+)ethyl nipecotate (prepared as described in J. Neurochem., 1976, 26, 1029) in step a. instead of the dibenzyl ester of aspartic acid to give the title compound directly without the need for subsequent deprotection. Found: C, 76.69; H, 7.64; N, 4.81. C₃₇H₄₄N₂O₄ requires C, 76.52; H, 7.63; N, 4.82%

### Example 153 Preparation of cis-7-(3-(-)-ethoxycarbonylpiperidinocarbonyl)-8-(1-adamantylmethylaminocarbonyl)-2,3,5,6-dibenzobicyclo[2.2.2]octane (mixture of diastereomers)

The compound was prepared essentially as in example 64 but using (-)ethyl nipecotate in step a. instead of the dibenzyl ester of aspartic acid to give the title compound directly without the need for subsequent deprotection. Found: C, 76.29; H, 7.61; N, 4.68. C₃₇H₄₄N₂O₄ requires C, 76.52; H, 7.63; N, 4.82%

### Example 154 Preparation of cis-7-(3-(+)-methoxycarbonylpiperidinocarbonyl)-8-(1-adamantylmethylaminocarbonyl)-2,3,5,6-dibenzobicyclo[2.2.2]octane (diastereomer 1)

### a. cis-7-(3-(+)-carboxy-piperidinocarbonyl)-8-(1-adamantylmethylaminocarbonyl)-2,3,5,6-dibenzobicyclo[2.2.2]octane (separated diastereomers)

The compound was prepared essentially as in example 64 but using (+)benzyl nipecotate (prepared by standard means from (+) ethyl nipecotate) in step a. instead of the dibenzyl ester of aspartic acid to give the carboxylic acid as a mixture of diastereomers. The two diastereomers were separated by chromatography (silica 90% dichloromethane and 10% ethyl acetate).

### b. cis-7-(3-(+)-methoxycarbonyl-piperidinocarbonyl)-8-(1-adamantylmethylaminocarbonyl)-2,3,5,6-dibenzobicyclo[2.2.2]octane diastereomer 1

The compound was prepared essentially as in example 63 using the less polar (higher R_{f} material) isolated in step a. above as substrate instead of cis-7-(1-S-carboxyl-2-methylpropylaminocarbonyl)-8-(1-adamantylmethylaminocarbonyl)-2,3,5,6-dibenzobicyclo[2.2.2]octane.

### Example 155 Preparation of cis-7-(3-(+)-methoxycarbonylpiperidinocarbonyl)-8-(1-adamantylmethylaninocarbonyl)-2,3,5,6-dibenzobicyclo[2.2.2]octane (diastereomer 2)

The compound was prepared essentially as in example 63 using the more polar (lower R_{f} material) isolated in step a. of example 154 as substrate instead of cis-7-(1-S-carboxyl-2-methylpropylaminocarbonyl)-8-(1-adamantylmethylaminocarbonyl)-2,3,5,6-dibenzobicyclo[2.2.2]octane.

### Example 156 Preparation of cis-7-(3-(-)-methoxycarbonylpiperidinocarbonyl)-8-(1-adamantylmethylaminocarbonyl)-2,3,5,6-dibenzobicyclo[2.2.2]octane (diastereomer 1)

The compound was prepared essentially as in example 154 but using (-) benzyl nipecotate as substrate in step a. rather than (+) benzyl nipecotate. As in that example the less polar material was converted to the title compound in step b.

### Example 157 Preparation of cis-7-(3-(-)-methoxycarbonylpiperidinocarbonyl)-8-(1-adamantylmethylaminocarbonyl)-2,3,5,6-dibenzobicyclo[2.2.2]octane (diastereomer 2)

The compound was prepared essentially as in example 63 using the more polar (lower R_{f} material) isolated in step a. of example 156 as substrate instead of cis-7-(1-S-carboxyl-2-methylpropylaminocarbonyl)-8-(1-adamantylmethylaminocarbonyl)-2,3,5,6-dibenzobicyclo[2.2.2]octane.

### Example 158 Preparation of cis-7-(2R-(1methyl-1-carboxy-cyclopropylaminocarbonyl)pyrrolidinocarbonyl)-8-(1-adamantylmethylaminocarbonyl)-2,3,5,6-dibenzobicyclo[2.2.2]octane (diastereomer 1)

The compound was prepared essentially as in example 64 but using the benzyl ester of D-prolyl-cyclopropylalanine (prepared by standard means) in step a. instead of the dibenzyl ester of aspartic acid. The diastereomers were separated at the benzyl ester stage by column chromatography (silica 60% dichloromethane and 40% ethyl acetate). The compound with the higher R_{f} was converted to the title compound by hydrogenation, [α]^{D}= +10.0° (c= 1.0 in methanol). The compound was further characterised and tested as the N-methyl-D-glucamine salt. Found: C, 58.54; H, 7.44; N, 6.03. C₄₅H₆₀N₄O₁₀ 5.6H₂O requires C, 58.92; H, 7.81; N, 6.11%

### Example 159 Preparation of cis-7-(2R-(1methyl-1-carboxy-cyclopropylaminocarbonyl)pyrrolidinocarbonyl)-8-(1-adamantylmethylaminocarbonyl)-2,3,5,6-dibenzobicyclo[2.2.2]octane (diastereomer 2)

The compound was prepared essentially as in example 158 but using the compound with lower R_{f} after diastereomer separation in the final hydrogenation step. The compound was tested as the N-methyl-D-glucamine salt.

### Example 160 Preparation of cis-7-(2-R-carboxypyrrolidinocarbonyl)-8-(1-adamantylmethylaminocarbonyl)-2,3-(2-fluorobenzo)-5,6-benzobicyclo[2.2.2]octane (mixture of isomers 1)

### a. 2,3-(2-fluorobenzo)-5,6-benzobicyclo[2.2.2]octane-7,8-dicarboxylic anhydride

This was performed essentially as described in example 1 step a. except that 2-fluoroanthracene was used as reactant instead of anthracene.

### b. 8-(1-adamantylmethylaminocarbonyl)-2,3-(2-fluorobenzo)-5,6-benzobicyclo[2.2.2]octane-7-carboxylic acid.

The reaction was performed essentially as described in example 14 but using the compound described in step a. above rather than 2,3,5,6-dibenzobicyclo[2.2.2]octane-7,8-dicarboxylic anhydride.

### c. cis-7-(2-R-benzyloxycarbonylpyrrolidinocarbonyl)-8-(1-adamantylmethylaminocarbonyl)-2,3-(2-fluorobenzo)-5,6-benzobicyclo[2.2.2]octane.

The reaction was performed essentially as in example 46 but using the compound prepared in step b above, rather than (+)cis-8-(1-adamantylmethylaminocarbonyl)-2,3,5,6-dibenzobicyclo[2.2.2]octane-7-carboxylic acid.

### d. cis-7-(2-R-carboxypyrrolidinocarbonyl)-8-(1-adamantylmethylaminocarbonyl)-2,3-(2-fluorobenzo)-5,6-benzobicyclo[2.2.2]octane (mixture of isomers 1)

The reaction was performed essentially as in example 48 but using the compound prepared in step c above, rather than cis-7-(2-R-benzyloxycarbonylpyrrolidinocarbonyl)-8-(1-adamantylmethylaminocarbonyl)-2,3,5,6-dibenzobicyclo[2.2.2]octane. The material which by this stage was a mixture of eight compounds was separated by HPLC (C8 column 60% acetonitrile, 40% water and 0.1% acetic acid) into three components. The material with a retention time of 15 min was designated the compound of this example. The compound was further characterised and tested as the N-methyl-D-glucamine salt found: C, 65.22; H, 7.38; N, 5.42. C₄₁H₅₄FN₃O₉ requires C, 65.49; H, 7.24; N, 5.59%

### Example 161 Preparation of cis-7-(2-R-carboxypyrrolidinocarbonyl)-8-(1-adamantylmethylaminocarbonyl)-2,3-(2-fluorobenzo)-5,6-benzobicyclo[2.2.2]octane (mixture of isomers 2)

The mixture from example 160 step d with a retention time of 16 min was designated the compound of this example. The compound was further characterised and tested as the N-methyl-D-glucamine salt found: C, 64.99; H, 7.29; N, 5.59. C₄₁H₅₄FN₃O₉ requires C, 65.49; H, 7.24; N, 5.59%

### Example 162 Preparation of cis-7-(2-R-carboxypyrrolidinocarbonyl)-8-(1-adamantylmethylaminocarbonyl)-2,3-(2-fluorobenzol-5,6-benzobicyclo[2.2.2]octane (mixture of isomers 3)

The mixture from example 160 step d with a retention time of 22 min was designated the compound of this example. The compound was further characterised and tested as the N-methyl-D-glucamine salt found: C, 65.29; H, 7.42; N, 5.65. C₄₁H₅₄FN₃O₉ requires C, 65.49; H, 7.24; N, 5.59%

### Example 163 Preparation of cis-7-(2R-(1-carboxy-1-methylethylaminocarbonyl)pyrrolidinocarbonyl)-8-(1-adamantylmethylaminocarbonyl)-2,3,5,6-dibenzobicyclo[2.2.2]octane (diastereomer 1)

The compound was prepared essentially as in example 64 but using the benzyl ester of D-prolyl-alpha-aminobutyric acid in step a. instead of the dibenzyl ester of aspartic acid. The diastereomers were separated at the benzyl ester stage by recrystallisation from ethyl acetate and column chromatography (silica 50% hexane and 50% ethyl acetate). The compound with the lower R_{f} was converted to the title compound by hydrogenation. The compound was further characterised and tested as the N-methyl-D-glucamine salt. Found: C, 65.91; H, 7.65; N, 6.60. C₄₅H₆₂N₄O₁₀ requires C, 65.99; H, 7.63; N, 6.84%

### Example 164 Preparation of cis-7-(2R-(1-carboxy-1-methylethylaminocarbonyl)pyrrolidinocarbonyl)-8-(1-adamantylmethylaminocarbonyl)-2,3,5,6-dibenzobicyclo[2.2.2]octane (diastereomer 2)

The compound was prepared essentially as in example 147 but using the compound with higher R_{f} after diastereomer separation in the final hydrogenation step. The compound was tested as the N-methyl-D-glucamine salt.

### Example 165 Preparation of cis-7-(2-R-carboxypyrrolidinocarbonyl)-8-(1-adamantylmethylaminocarbonyl)-8-fluoro-2,3,5,6-dibenzobicyclo[2.2.2]octane (mixture of diastereomers)

The compound was prepared essentially as described in example 160 but performing the reaction in step a. with anthracene and 2-fluoromaleic anhydride (prepared as in J.Am.Chem.Soc., 1959, 81, 2678) instead of the reagents stated. No attempt was made at separation of the diastereomers at any stage. The compound was further characterised and tested as the N-methyl-D-glucamine salt found: C, 65.59; H, 7.18; N, 5.61. C₄₁H₅₄FN₃O₉ requires C, 65.49; H, 7.24; N, 5.59%

### Example 166 Preparation of cis-7-(-N-(carboxymethyl)-N-(methoxycarbonylmethyl)aminocarbonyl)-8-(1-adamantylmethylaminocarbonyl)-2,3,5,6-dibenzobicyclo[2.2.2]octane

The reaction was performed essentially as in example 25 but using methyl N-(carboxymethyl)glycine (prepared as in Tetrahedron, 1984, 40, 1151) as substrate instead of L-alanine methyl ester. The compound was further characterised and tested as the N-methyl-D-glucamine salt found: C, 64.21; H, 7.25; N, 5.22. C₄₁H₅₅N₃O₁₁requires C, 64.30; H, 7.24; N, 5.49%

### Example 167 Preparation of cis-7-(-N-(4-(2-oxo-N-(carboxymethyl)pyrrolidine))aminocarbonyl)-8-(1-adamantylmethylaminocarbonyl)-2,3,5,6-dibenzobicyclo[2.2.2]octane (diastereomers 1 and 2)

The reaction was performed essentially as in example 64 but using the benzyl ester of N-carboxymethyl-2-oxo-4-aminopyrrolidine (prepared as in Peptide Research 1991, 4, 171) in step a. instead of the dibenzyl ester of aspartic acid. The compound was separated at the end of step a. into two pairs of diastereoisomers by column chromatography the higher R_{f} components being hydrogenated at step b to give the title compounds of this example. The compound was further characterised and tested as the N-methyl-D-glucamine salt. Found: C, 57.23; H, 7.41; N, 6.13. C₄₂H₅₆N₄O₁₀ 5.5H₂O requires C, 57.54; H, 7.71; N, 6.39%

### Example 168 Preparation of cis-7-(-N-(4-(2-oxo-N-(carboxymethyl)pyrrolidine))aminocarbonyl)-8-(1-adamantylmethylaminocarbonyl)-2,3,5,6-dibenzobicyclo[2.2.2]octane (diastereomers 3 and 4)

The reaction was performed essentially as in example 167 except that the lower R_{f} components were used in the hydrogenation step b. The compound was further characterised and tested as the N-methyl-D-glucamine salt. Found: C, 56.07; H, 7.39; N, 6.56. C₄₂H₅₆N₄O₁₀ 6.5H₂O requires C, 56.46; H, 7.78; N, 6.27%

### Example 169 Preparation of cis-7-(-N-(4-(2-oxo-N-(methoxycarbonylmethyl)pyrrolidine))aminocarbonyl)-8-(1-adamantylmethylaminocarbonyl)-2,3,5,6-dibenzobicyclo[2.2.2]octane (diastereomers 1 and 2)

The compound was prepared essentially as in example 63 using the compound of example 167 as substrate instead of cis-7-(1-S-carboxyl-2-methylpropylaminocarbonyl)-8-(1-adamantylmethylaminocarbonyl)-2,3,5,6-dibenzobicyclo[2.2.2]octane. Found: C, 66.83; H, 7.21; N, 6.15. C₃₆H₄₁N₃O₅. 1.7CH₃OH and 0.4 CH₂Cl₂ requires C, 66.89; H, 7.21; N, 6.15%

### Example 170 Preparation of cis-7-(-N-(4-(2-oxo-N-(methoxycarbonylmethyl)pyrrolidine))aminocarbonyl)-8-(1-adamantylmethylaminocarbonyl)-2,3,5,6-dibenzobicyclo[2.2.2]octane (diastereomers 3 and 4)

The compound was prepared essentially as in example 63 using the compound of example 168 as substrate instead of cis-7-(1-S-carboxyl-2-methylpropylaminocarbonyl)-8-(1-adamantylmethylaminocarbonyl)-2,3,5,6-dibenzobicyclo[2.2.2]octane. Found: C, 64.05; H, 6.71; N, 5.82. C₃₆H₄₁N₃O₅. 1.0CH₃OH and 1.0 CH₂Cl₂ requires C, 64.04; H, 6.65; N, 5.90%

### Example 171 Preparation of cis-7-(1S-(carboxymethylaminocarbonyl)-2-phenylethylaminocarbonyl)-8-(1-adamantylmethylaminocarbonyl)-2,3,5,6-dibenzobicyclo[2.2.2]octane (mixture of diastereomers)

The reaction was performed essentially as in example 64 but using the benzyl ester of L-phenylalanylglycine in step a. instead of the dibenzyl ester of aspartic acid. The compound was further characterised and tested as the N-methyl-D-glucamine salt. Found: C, 64.11; H, 7.62; N, 6.35. C₄₇H₆₀N₄O₁₀. 2.3H₂O requires C, 63.95; H, 7.38; N, 6.35%

### Example 172 Preparation of cis-7-(1S-(1R-carboxyethylaminocarbonyl)-2-phenylethylaminocarbonyl)-8-(1-adamantylmethylaminocarbonyl)-2,3,5,6-dibenzobicyclo[2.2.2]octane (mixture of diastereomers)

The reaction was performed essentially as in example 64 but using the benzyl ester of L-phenylalanyl-D-alanine in step a. instead of the dibenzyl ester of aspartic acid. The compound was tested as the mono-N-methyl-D-glucamine salt.

### Example 173 Preparation of cis-7-(1R-(carboxymethylaminocarbonyl)-2-phenylethylaminocarbonyl)-8-(1-adamantylmethylaminocarbonyl)-2,3,5,6-dibenzobicyclo[2.2.2]octane (mixture of diastereomers)

The reaction was performed essentially as in example 64 but using the benzyl ester of D-phenylalanylglycine in step a. instead of the dibenzyl ester of aspartic acid. The compound was further characterised and tested as the mono-N-methyl-D-glucamine salt. Found: C, 62.18; H, 7.48; N, 5.74. C₄₇H₆₀N₄O₁₀. 4.0H₂O requires C, 61.86; H, 7.51; N, 6.14%

### Example 174 Preparation of cis-7-(1S-(carboxymethylaminocarbonyl)-2-(3-indolyl)phenylethylaminocarbonyl)-3-(1-adamantylmethylaminocarbonyl)-2,3,5,6-dibenzobicyclo[2.2.2]octane (mixture of diastereomers)

The reaction was performed essentially as in example 64 but using the benzyl ester of L-tryptophanylglycine in step a. instead of the dibenzyl ester of aspartic acid. The compound was further characterised and tested as the mono-N-methyl-D-glucamine salt. Found: C, 58.88; H, 7.07; N, 6.62. C₄₉H₆₁N₅O₁₀. 3.2H₂O and 1.0 CH₂Cl₂ requires C, 58.73; H, 6.84; N, 6.85%

### Example 175 Preparation of cis-7-(2R-(1-S-carboxy-2-carboxyethylaminocarbonyl)pyrrolidinocarbonyl)-8-(1-adamantylmethylaminocarbonyl)-2,3,5,6-dibenzobicyclo[2.2.2]octane (diastereomer 1)

The compound was prepared essentially as in example 64 but using the dibenzyl ester of D-prolyl-L-aspartic acid in step a. instead of the dibenzyl ester of aspartic acid. The diastereomers were separated at the benzyl ester stage by column chromatography (silica 60% dichloromethane and 40% ethyl acetate). The compound with the higher R_{f} was converted to the title compound by hydrogenation. Found: C, 64.46; H, 6.97; N, 5.30. C₃₈H₄₃N₃O₇. 3.0 H₂O requires C, 64.48; H, 6.97; N, 5.63% ¹H NMR

### Example 176 Preparation of cis-7-(2R-(1-S-carboxy-2-carboxyethylaminocarbonyl)pyrrolidinocarbonyl)-8-(1-adamantylmethylaminocarbonyl)-2,3,5,6-dibenzobicyclo[2.2.2]octane (diastereomer 2)

The compound was prepared essentially as in example 175 but using the compound with lower R_{f} after diastereomer separation in the final hydrogenation step. Found: C, 69.21; H, 6.73; N, 5.93. C₃₈H₄₃N₃O₇. 0.5 H₂O requires C, 68.86; H, 6.69; N, 6.34%

### Example 177 Preparation of (±)-cis-8-(6-undecylaminocarbonyl)-2,3,5,6-dibenzobicyclo[2.2.2]octane-7-carboxylic acid

The compound was prepared essentially as in example 14 but using 6-undecylamine as substrate instead of 1-adamantylmethylamine. The compound was further characterised and tested as the N-methyl-D-glucamine salt. Found: C, 65.38; H, 8.53; N, 5.33. C₃₆H₅₄N₂O₈. 1.0H₂O requires C, 65.43; H, 8.54; N, 4.23%

### Example 178 Preparation of cis-7-(-N-(3S-(2-oxo-N-(carboxymethyl)pyrrolidine))aminocarbonyl)-8-(1-adamantylmethylaminocarbonyl)-2,3,5,6-dibenzobicyclo[2.2.2]octane

The reaction was performed essentially as in example 64 but using the benzyl ester of N-carboxymethyl-2-oxo-3S-aminopyrrolidine (prepared as in J. Org. Chem. 1982, 47, 105) in step a. instead of the dibenzyl ester of aspartic acid. The compound was tested as the N-methyl-D-glucamine salt.

### Example 179 Preparation of cis-7-(-N-(3R-(2-oxo-N-(carboxymethyl)pyrrolidine))aminocarbonyl)-8-(1-adamantylmethylaminocarbonyl)-2,3,5,6-dibenzobicyclo[2.2.2]octane

The reaction was performed essentially as in example 64 but using the benzyl ester of N-carboxymethyl-2-oxo-3R-aminopyrrolidine (prepared as in J. Org. Chem. 1982, 47, 105) in step a. instead of the dibenzyl ester of aspartic acid. The compound was tested as the N-methyl-D-glucamine salt.

### Example 180 Preparation of cis-7-(2R-(carboxymethylaminocarbonyl)-2S-methyl-pyrrolidinocarbonyl)-8-(1-adamantylmethylaminocarbonyl)-2,3,5,6-dibenzobicyclo[2.2.2]octane

The compound was prepared essentially as in example 64 but using the benzyl ester of α-methyl-D-prolyle-glycine (prepared as in J. Am. Chem. Soc., 1983, 105, 5390) in step a. instead of the dibenzyl ester of aspartic acid. The compound was tested as the N-methyl-D-glucamine salt.

### Example 181 Preparation of cis-7-(2R-(aminocarbonylmethylaminocarbonyl)-pyrrolidinocarbonyl)-8-(1-adamantylmethylaminocarbonyl)-2,3,5,6-dibenzobicyclo[2.2.2]octane

The compound was prepared essentially as in example 64 but using D-prolyl-glycinamide in step a. instead of the dibenzyl ester of aspartic acid to give the title compound directly. Obviously there was no need for a hydrogenation step. Found: C, 67.41; H, 7.01; N, 8.54. C₃₆H₄₂N₄O₄. 2.4H₂O requires C, 67.79; H, 7.39; N, 8.78%

### Example 182 Preparation of cis-7-(1R-(aminocarbonylmethylaminocarbonyl)-phenethylaminocarbonyl)-8-(1-adamantylmethylaminocarbonyl)-2,3,5,6-dibenzobicyclo[2.2.2]octane

The compound was prepared essentially as in example 64 but using D-phenylalanyl-glycinamide in step a. instead of the dibenzyl ester of aspartic acid to give the title compound directly. Obviously there was no need for a hydrogenation step. Found: C, 71.76; H, 7.01; N, 8.09. C₄₀H₄₄N₄O₄. 1.5H₂O requires C, 71.51; H, 7.05; N, 8.33%

### Example 183 Preparation of cis-7-(2R-(carboxymethylaminocarbonyl)-4R-hydroxy-pyrrolidinocarbonyl)-8-(1-adamantylmethylaminocarbonyl)-2,3,5,6-dibenzobicyclo[2.2.2]octane

The compound was prepared essentially as in example 64 but using the benzyl ester of cis-4-hydroxy-D-prolylglycine in step a. instead of the dibenzyl ester of aspartic acid. The compound was further characterised and tested as the N-methyl-D-glucamine salt. Found: C, 56.39; H, 7.65; N, 7.96. C₄₃H₅₈N₄O₁₁. 6.2H₂O and 1.6 CH₃CN requires C, 56.37; H, 7.70; N, 7.97%

### Example 184 Preparation of cis-7-(2S-(1S-carboxyethylaminocarbonylmethyl)pyrrolidinocarbonyl)-8-(1-adamantylmethylaminocarbonyl)-2,3,5,6-dibenzobicyclo[2.2.2]octane (mixture of diastereomers)

### a. cis-7-(2S-(1S-benzyloxycarbonylethylaminocarbonylmethyl)-pyrrolidinocarbonyl)-8-(1-adamantylmethylaminocarbonyl)-2,3,5,6-dibenzobicyclo[2.2.2]octane (mixture of diastereomers)

The compound of example 141 (250 mg, 0.45 mmol) was dissolved in dichloromethane (30 ml) and L-alanine benzyl ester p-toluenesulphonate salt (160 mg, 0.45 mmol) was added followed by PyBOP (235 mg, 0.45 mmol) and Hunigs base (240 ml, 1.35 mmol). The mixture was stirred at room temperature for 42 h and then washed with 5% potassium hydrogensulphate solution (15 ml), sodium hydrogencarbonate solution (15 ml) and saturated brine (15 ml). The solution was then dried and the product purified by chromatography (silica and ethyl acetate) to yield a colourless solid.

### b. cis-7-(2S-(1S-carboxyethylaminocarbonylmethyl)-pyrrolidinocarbonyl)-8-(1-adamantylmethylaminocarbonyl)-2,3,5,6-dibenzobicyclo[2.2.2]octane (mixture of diastereomers)

The reaction was performed essentially as in example 48 but using the product of step a. as substrate instead of the product of example 46. The compound was tested as the N-methyl-D-glucamine salt.

### Example 185 Preparation of cis-7-(2S-(1R-carboxyethylaminocarbonylmethyl)pyrrolidinocarbonyl)-8-(1-adamantylmethylaminocarbonyl)-2,3,5,6-dibenzobicyclo[2.2.2]octane (mixture of diastereomers).

The reaction was performed essentially as in example 184 but using D-alanine benzyl ester p-toluenesulphonate salt in step a. rather than L-alanine benzyl ester p-toluenesulphonate salt. The compound was further characterised and tested as the N-methyl-D-glucamine salt. Found: C, 59.20; H, 7.64; N, 6.11. C₄₅H₆₂N₄O₁₁. 4.9H₂O requires C, 59.54; H, 7.98; N, 6.17%

### Example 186 Preparation of cis-7-(2S-(1R-carboxyethylaminocarbonylmethyl)pyrrolidinocarbonyl)-8-(1-adamantylmethylaminocarbonyl)-2,3,5,6-dibenzobicyclo[2.2.2]octane (diastereomer 1)

### a . cis-7-(2S-carboxymethylpyrrolidinocarbonyl)-8-(1-adamantylmethylaminocarbonyl)-2,3,5,6-dibenzobicyclo[2.2.2]octane (diastereomer 1)

The compound of example 141 was separated into its constituent diastereomers by recrystallisation from dichloromethane. The crystals isolated were the title compound. In addition the other diastereomer was isolated by concentration of the mother liquors.

### b. cis-7-(2S-(1R-carboxyethylaminocarbonylmethyl)pyrrolidinocarbonyl)-8-(1-adamantylmethylaminocarbonyl)-2,3,5,6-dibenzobicyclo[2.2.2]octane (diastereomer 1)

This was prepared in two steps as described in example 185 but using the product from step a. above rather than the compound of example 141 as the substrate in step a. The compound was tested as the N-methyl-D-glucamine salt.

### Example 187 Preparation of cis-7-(2S-(1R-carboxyethylaminocarbonylmethyl)pyrrolidinocarbonyl)-8-(1-adamantylmethylaminocarbonyl)-2,3,5,6-dibenzobicyclo[2.2.2]octane (diastereomer 2)

This was prepared essentially as in example 185 but using the dichloromethane soluble diastereomer described in example 186 step a. as substrate in step a. rather than the compound of example 141. The compound was tested as the N-methyl-D-glucamine salt.

### Example 188 Preparation of cis-7-(2R-(methoxycarbonylmethylaminocarbonyl)pyrrolidinocarbonyl)-8-(1-adamantylmethylaminocarbonyl)-2,3,5,6-dibenzobicyclo[2.2.2]octane (diastereomer 1)

The compound of example 123 was separated into its constituent diastereomers by chromatography (silica 30% ethyl acetate and 70% dichloromethane). The less polar material was designated the compound of this example. Found: C, 64.59; H, 6.76; N, 5.94. C₃₇H₄₃N₃O₅.0.3 EtOAc and 1.1 CH₂Cl₂ requires C, 64.70; H, 6.58; N, 5.76%

### Example 189 Preparation of cis-7-(2R-(methoxycarbonylmethylaminocarbonyl)pyrrolidinocarbonyl)-8-(1-adamantylmethylaminocarbonyl)-2,3,5,6-dibenzobicyclo[2.2.2]octane.

The more polar material isolated in example 188 was designated the compound of this example. Found: C, 70.27; H, 7.70; N, 6.63. C₃₇H₄₃N₃O₅ requires C, 69.97; H, 7.27; N, 6.62%

### Example 190 Preparation of cis-7-(2R-(carboxymethyl(N-methyl)-aminocarbonyl)pyrrolidinocarbonyl)-8-(1-adamantylmethylaminocarbonyl)-2,3,5,6-dibenzobicyclo[2.2.2]octane (mixture of diastereomers)

The compound was prepared essentially as in example 64 but using the benzyl ester of D-prolyl-sarcosine in step a. Instead of the dibenzyl ester of aspartic acid. The compound was tested as the N-methyl-D-glucamine salt.

### Example 191 Preparation of cis-7-(-N-(3R-(2-oxo-N-(methoxycarbonylmethyl)pyrrolidine))aminocarbonyl)-8-(1-adamantylmethylaminocarbonyl)-2,3,5,6-dibenzobicyclo[2.2.2]octane

The reaction was performed essentially as in example 63 using the compound of example 179 as substrate instead of cis-7-(1-S-carboxyl-2-methylpropylaminocarbonyl)-8-(1-adamantylmethylaminocarbonyl)-2,3,5,6-dibenzobicyclo[2.2.2]octane. The compound was tested as the N-methyl-D-glucamine salt.

### Example 192 Preparation of cis-7-(2-R-carboxy-pyrrolidinocarbonyl)-8-(1-adamantylmethylaminocarbonyl)-1,4-dimethyl-2,3,5,6-dibenzobicyclo[2.2.2]octane (diastereomer 1)

The compound was prepared essentially as in example 137 but using 9,10-dinethylanthracene as substrate in step a. instead of 9,10-difluoroanthracene. In addition the mixcure of diastereomers isolated after step c was separated into its constituent isomers using chromatography (silica 10% ethyl acetate and 90% dichloromethane). The less polar material was taken through to the title compound. The compound was further characterised and tested as the N-methyl-D-glucamine salt found: C, 64.71; H, 7.88; N, 5.06. C₄₃H₅₉N₃O₉. 2.0H₂O requires C, 64.65; H, 7.96; N, 5.26%

### Example 193 Preparation of cis-7-(2-R-carboxy-pyrrolidinocarbonyl)-8-(1-adamantylmethylaminocarbonyl)-1,4-dimethyl-2,3,5,6-dibenzobicyclo[2.2.2]octane (diastereomer 2)

The more polar compound described in example 192 was hydrogenated to give the compound of this example. The compound was further characterised and tested as the N-methyl-D-glucamine salt. Found: C, 61.67; H, 7.88; N, 4.82. C₄₃H₅₉N₃O₉. 4.0H₂O requires C, 61.88; H, 8.10; N, 5.04

### Example 194 Preparation of cis-7-(2S-(carboxymethylaminocarbonylmethyl)pyrrolidinocarbonyl)-8-(1-adamantylmethylaminocarbonyl)-2,3,5,6-dibenzobicyclo[2.2.2]octane (mixture of diastereomers)

The reaction was performed essentially as in example 184 but using the benzyl ester of glycine in step a. rather than L-alanine benzyl ester p-toluenesulphonate salt. The compound was further characterised and tested as the N-methyl-D-glucamine salt, HPLC; R_{T}=16.2 mins, C8 column, CH₃CN 50%, H₂O 50%, 0.1% CH₃COOH.

### Example 195 Preparation of cis-7-(2R-(1R-(2,2-dimethyl-1,3-dioxolane-4-methoxycarbonyl)-ethylaminocarbonyl)pyrrolidinocarbonyl)-8-(1-adamantylmethylaminocarbonyl)-2,3,5,6-dibenzobicyclo[2.2.2]octane

The compound of example 147 (305 mg, 0.5 mmol) was dissolved in dry dichloromethane (3 ml) and solketal (66 ml, 0.5 mmol) was added. DMAP (2 mg) and DCCI (103 mg, 0.5 mmol) were added and the reaction mixture stirred at room temperature for lh. After filtration the solution was evaporated to leave a foam which was purified by column chromatography (silica 95% dichloromethane and 5% methanol) to leave the title compound (195 mg). Found: C, 71.42; H, 7.45; N, 5.84. C₄₃H₅₃N₃O₇ requires C, 71.35; H, 7.38; N, 5.81%

### Example 196 Preparation of cis-7-(2R-(1R-(pivaloyloxymethoxycarbonyl)-ethylaminocarbonyl)pyrrolidinocarbonyl)-8-(1-adamantylmethylaminocarbonyl)-2,3,5,6-dibenzobicyclo[2.2.2]octane

The compound of example 147 (305 mg, 0.5 mmol) was dissolved in DMF (2 ml) and pivaloyloxymethyl chloride (72 ml, 0.55 mmol) and caesium carbonate (82 mg, 0.5 mmol) was added. After gentle warming the reaction was stirred at room temperature for 1h. The reaction mixture was poured onto brine (30 ml) and extracted with ethyl acetate (30 ml). The organic layer was washed with brine (2 x 30 ml) dried and evaporated. The material was completely purified by passage through a silica pad eluting with a 1:1 mixture of dichloromethane and ethyl acetate to leave the title compound (160 mg). Found: C, 71.21; H, 7.48; N, 5.62. C₄₃H₅₃N₃O₇ requires C, 71.35; H, 7.38; N, 5.81%

### Example 197 Preparation of cis-7-(2R-aminocarbonyl)-pyrrolidinocarbonyl)-8-(1-adamantylmethylaminocarbonyl)-2,3,5,6-dibenzobicyclo[2.2.2]octane (mixture of diastereomers)

The compound was prepared essentially as in example 25 but using D-prolinamide instead of the methyl ester of l-alanine.

### Example 198 Preparation of cis-7-(2R-(carboxymethylaminocarbonylmethyl)pyrrolidinocarbonyl)-8-(1-adamantylmethylaminocarbonyl)-2,3,5,6-dibenzobicyclo[2.2.2]octane (diastereomer 1)

### a. cis-7-(2R-carboxymethylpyrrolidinocarbonyl)-8-(1-adamantylmethylaminocarbonyl)-2,3,5,6-dibenzobicyclo[2.2.2]octane (separation of diastereomers)

The compound of example 142 was treated with dichloromethane. The dichloromethane insoluble material was designated diastereomer 1 and the soluble isomer designated diastereomer 2.

### b. cis-7-(2R-(carboxymethylaminocarbonylmethyl)-pyrrolidinocarbonyl)-8-(1-adamantylmethylaminocarbonyl)-2,3,5,6-dibenzobicyclo[2.2.2]octane (diastereomer 1)

The compound was prepared essentially as in example 194 except that the diastereomer 1 from step a. above was used as substrate instead of the compound of example 141. The compound was tested as the N-methyl-D-glucamine salt.

### Example 199 Preparation of cis-7-(2R-(carboxymethylaminocarbonylmethyl)pyrrolidinocarbonyl)-8-(1-adamantylmethylaninocarbonyl)-2,3,5,6-dibenzobicyclo[2.2.2]octane (diastereomer 2)

The compound was prepared essentially as in example 194 except that the diastereomer 2 from example 198 step a. was used as substrate instead of the compound of example 141. The compound was tested as the N-methyl-D-glucamine salt.

### Example 200 Preparation of cis-7-(2R-(1S-carboxyethylaminocarbonylmethyl)pyrrolidinocarbonyl)-8-(1-adamantylmethylaminocarbonyl)-2,3,5,6-dibenzobicyclo[2.2.2]octane (diastereomer 1)

The compound was prepared essentially as in example 184 except that the diastereomer 1 from example 198 step a. was used as substrate instead of the compound of example 141. The compound was tested as the N-methyl-D-glucamine salt.

### Example 201 Preparation of cis-7-(2R-(1S-carboxyethylaminocarbonylmethyl)pyrrolidinocarbonyl)-8-(1-adamantylmethylaminocarbonyl)-2,3,5,6-dibenzobicyclo[2.2.2]octane (diastereomer 2)

The compound was prepared essentially as in example 184 except that the diastereomer 2 from example 198 step a. was used as substrate instead of the compound of example 141. The compound was tested as the N-methyl-D-glucamine salt.

### Example 202 Preparation of cis-7-(2R-(1R-carboxyethylaminocarbonylmethyl)pyrrolidinocarbonyl)-8-(1-adamantylmethylaminocarbonyl)-2,3,5,6-dibenzobicyclo[2.2.2]octane (diastereomer 1)

The compound was prepared essentially as in example 185 except that the diastereomer 1 from example 198 step a. was used as substrate instead of the compound of example 141. The compound was tested as the N-methyl-D-glucamine salt.

### Example 203 Preparation of cis-7-(2R-(1R-carboxyethylaminocarbonylmethyl)pyrrolidinocarbonyl)-8-(1-adamantylmethylaminocarbonyl)-2,3,5,6-dibenzobicyclo[2.2.2]octane (diastereomer 2)

The compound was prepared essentially as in example 185 except that the diastereomer 2 from example 198 step a. was used as substrate instead of the compound of example 141. The compound was tested as the N-methyl-D-glucamine salt.

### Example 204 Preparation of cis-7-(2R-(carboxyethyl)pyrrolidinocarbonyl)-8-(1-adamantylmethylaminocarbonyl)-2,3,5,6-dibenzobicyclo[2.2.2]octane (mixture of diastereomers)

The compound was prepared essentially as in example 64 but using the benzyl ester of trans-3-(2R-pyrrolidino)-but-2-enoic acid (prepared from benzyl(triphenylphosphoranylidene)acetate and N-(t-butoxycarbonyl)-D-prolinal by Wittig reaction ) in step a. instead of the dibenzyl ester of aspartic acid. The compound was further characterised and tested as the N-methyl-D-glucamine salt. Found: C, 64.81; H, 7.94; N, 5.14. C₄₃H₅₉N₃O₉. 2.0H₂O requires C, 64.77; H, 7.96; N, 5.27%

### Example 205 Preparation of cis-7-(2S-(methoxycarbonylethenyl)pyrrolidinocarbonyl)-8-(1-adamantylmethylaminocarbonyl)-2,3,5,6-dibenzobicyclo[2.2.2]octane (mixture of diastereomers)

The compound was prepared essentially as in example 25 but using the methyl ester of trans-3-(2S-pyrrolidino)-but-2-enoic acid (prepared from methyl(triphenylphosphoranylidene)acetate and N-(t-butoxycarbonyl)-L-prolinal by Wittig reaction) instead of L-alanine methyl ester. Found: C, 76.66; H, 7.39; N, 4.73. C₃₇H₄₂N₂O₄ requires C, 76.79; H, 7.32; N, 4.84%

### Example 206 Preparation of cis-7-(2S-(methoxycarbonylethyl)pyrrolidinocarbonyl)-8-(1-adamantylmethylaminocarbonyl)-2,3,5,6-dibenzobicyclo[2.2.2]octane (mixture of diastereomers)

This was prepared by treating the compound of example 205 with 10% palladium on charcoal in an atmosphere of hydrogen gas. Found: C, 76.73; H, 7.79; N, 4.91. C₃₇H₄₄N₂O₄ requires C, 76.52; H, 7.64; N, 4.82%

### Example 207 Preparation of cis-7-(1S-(aminocarbonylmethylaminocarbonyl)-2-phenylethylaminocarbonyl)-8-(1-adamantylmethylaminocarbonyl)-2,3,5,6-dibenzobicyclo[2.2.2]octane (diastereomer 1)

The reaction was performed essentially as in example 25 but using L-phenylalanylglycinamide as substrate instead of L-alanine methyl ester. The mixture of diastereomers produced by this reaction was separated by column chromatography (silica and ethyl acetate). The less polar material was designated the title compound of this example. Found: C, 72.24; H, 7.02; N, 8.21. C₄₀H₄₄N₄O₄.1.2 H₂O requires C, 74.50; H, 6.87; N, 8.68%

### Example 208 Preparation of cis-7-(1S-(aminocarbonylmethylaminocarbonyl)-2-phenylethylaminocarbonyl)-8-(1-adamantylmethylaminocarbonyl)-2,3,5,6-dibenzobicyclo[2.2.2]octane (diastereomer 2)

The more polar material isolated from the separation in example 207 was designated the compound of this example. Found: C, 72.12; H, 6.95; N, 8.39. C₄₀H₄₄N₄O₄.1.2 H₂O requires C, 74.50; H, 6.87; N, 8.68%

### Example 209 Preparation of (±)-7-(1-adamantylmethylaminocarbonylmethyl)-2,3,5,6-dibenzobicyclo[2.2.2]octane-7-carboxylic acid

The compound was prepared essentially as in example 131 except that itaconic anhydride was used as substrate instead of the methyl ester of aconitic anhydride in step a. The compound was further characterised and tested as the N-methyl-D-glucamine salt, m.p. 115-120° . Found: C, 68.06; H, 7.71; N, 4.37. C₃₇H₅₀N₂O₈ requires C, 68.29; H, 7.74; N, 4.30%

### Example 210 Preparation of 7-(1S-methoxycarbonylethylaminocarbonyl)-7-(1-adamantylmethylaminocarbonylmethyl)-2,3,5,6-dibenzobicyclo[2.2.2]octane (mixture of diastereoisomers)

The compound was prepared essentially as in example 25 except that the compound of example 209 was used as substrate instead of the compound of example 14. Found : C, 75.36; H, 7.56; N, 4.99. C₃₄H₄₀N₂O₄ requires C, 75.53; H, 7.46; N, 5.18%

### Example 211 Preparation of 7-(2R-carboxypyrrolidinocarbonyl)-7-(1-adamantylmethylaminocarbonylmethyl)-2,3,5,6-dibenzobicyclo[2.2.2]octane (mixture of diastereoisomers)

The compound was prepared essentially as in example 160 except that the compound of example 209 was used in step c as substrate instead of the compound of example 160 step b. The compound was further characterised and tested as the N-methyl-D-glucamine salt. Found: C, 67.24; H, 7.74; N, 5.81. C₄₂H₅₇N₃O₉ requires C, 67.45; H, 7.68; N, 5.62%

### Example 212 Preparation of (±)-cis-7-(2-carboxycyclopentylaminocarbonyl)-8-(1-adamantylmethylaminocarbonyl)-2,3,5,6-dibenzobicyclo[2.2.2]octane (mixture of diastereomers 1)

The compound was prepared essentially as in example 64 but using the benzyl ester of cis 2-amino-cyclopentanoic acid in step a. instead of the dibenzyl ester of aspartic acid. The mixture of compounds after step a. was separated by column chromatography (silica 85% dichloromenthane 15% ethyl acetate) to give two pairs of diastereomers. The less polar material was hydrogenated to give the compound of this example. The compound was further characterised and tested as the N-methyl-D-glucamine salt. Found: C, 65.12; H, 7.75; N, 5.42. C₄₂H₅₇N₃O₉.1.5 H₂O requires C, 65.1; H, 7.80; N, 5.42%

### Example 213 Preparation of (±)-cis-7-(2-carboxycyclopentylaminocarbonyl)-8-(1-adamantylmethylaminocarbonyl)-2,3,5,6-dibenzobicyclo[2.2.2]octane (mixture of diastereomers 2)

The compound was prepared essentially as in example 212 except that the more polar material after separation was hydrogenated to give the compound of this example. The compound was further characterised and tested as the N-methyl-D-glucamine salt. Found: C, 64.88; H, 7.91; N, 5.28. C₄₂H₅₇N₃O₉.1.7 H₂O requires C, 64.8; H, 7.82; N, 5.40%

### Example 214 Preparation of cis-7-(2R-(1R-methoxycarbonylethylaminocarbonyl)pyrrolidinocarbonyl)-8-(1-adamantylmethylaminocarbonyl)-2,3,5,6-dibenzobicyclo[2.2.2]octane (single diastereomer)

The compound was prepared essentially as in example 63 except that the compound of example 147 was used as substrate instead of cis-7-(1-S-carboxyl-2-methylpropylaminocarbonyl)-8-(1-adalantylmethylaminocarbonyl)-2,3,5,6-dibenzobicyclo[2.2.2]octane (mixture of diastereomers). Found: C, 72.88; H, 7.51; N, 6.58. C₃₈H₄₅N₃O₅ requires C, 73.17; H, 7.27; N, 6.74%

### Example 215 Preparation of cis-7-(2S-(carboxyethyl)-pyrrolidinocarbonyl)-8-(1-adamantylmethylaminocarbonyl)-2,3,5,6-dibenzobicyclo[2.2.2]octane (mixture of diastereomers)

The compound was prepared essentially as in example 64 but using the benzyl ester of trans-3-(2S-pyrrolidino)-but-2-enoic acid prepared from benzyl(triphenylphosphoranylidene)acetate and N-(t-butoxycarbonyl)-L-prolinal by Wittig reaction) in step a. instead of the dibenzyl ester of aspartic acid. The compound was further characterised and tested as the N-methyl-D-glucamine salt. Found: C, 66.64; H, 8.02; N, 5.60. C₄₃H₅₉N₃O₉.0.6 H₂O requires C, 66.80; H, 7.85; N, 5.44%

### Example 216 Preparation of cis-7-(1S-carboxy-(2-naphthyl)-ethylaminocarbonyl)-8-(1-adamantylmethylaminocarbonyl)-2,3,5,6-dibenzobicyclo[2.2.2]octane (mixture of diastereomers)

The compound was prepared essentially as in example 64 but using the benzyl ester L-3-(2-naphthyl)alanine in step a. instead of the dibenzyl ester of aspartic acid. The compound was further characterised and tested as the N-methyl-D-glucamine salt. Found: C, 70.15; H, 7.33; N, 5.05. C₄₉H₅₉N₃O₉.0.3 H₂O requires C, 70.05; H, 7.16; N, 5.00%

### Example 217 Preparation of cis-7-(1S-carboxy-(1-naphthyl)-ethylaminocarbonyl)-8-(1-adamantylmethylaminocarbonyl)-2,3,5,6-dibenzobicyclo[2.2.2]octane (mixture of diastereomers)

The compound was prepared essentially as in example 64 but using the benzyl ester L-3-(1-naphthyl)alanine in step a. instead of the dibenzyl ester of aspartic acid. The compound was further characterised and tested as the N-methyl-D-glucamine salt. Found: C, 69.94; H, 7.14; N, 5.23. C₄₉H₅₉N₃O₉.0.3 H₂O requires C, 70.05; H, 7.16; N, 5.00%

### Example 218 Preparation of cis-7-(1R-(1R-carboxyethylaminocarbonyl)-2-phenylethylaminocarbonyl)-8-(1-adamantylmethylaminocarbonyl)-2,3,5,6-dibenzobicyclo[2.2.2]octane (mixture of diastereomers)

The compound was prepared essentially as in example 64 but using the benzyl ester D-phenylalanyl-D-alanine in step a. instead of the dibenzyl ester of aspartic acid. The compound was further characterised and tested as the N-methyl-D-glucamine salt. Found: C, 64.21; H, 7.63; N, 6.36. C₄₈H₆₂N₄O₁₀.2.3 H₂O requires C, 64.28; H, 7.63; N, 6.36%

### Example 219 Preparation of cis-7-(3-S-carboxy-1,2,3,4-tetrahydroisoquinolinocarbonyl)-8-(1-adamantylmethylaminocarbonyl)-2,3,5,6-dibenzobicyclo[2.2.2]octane (mixture of diastereomers)

The compound was prepared essentially as in example 64 but using the benzyl ester 1,2,3,4-tetrahydroisoquinoline-3-S-carboxylic acid in step a. instead of the dibenzyl ester of aspartic acid. The compound was further characterised and tested as the N-methyl-D-glucamine salt. Found: C, 66.75; H, 7.37; N, 5.08. C₄₆H₅₇N₃O₉₀.1.7 H₂O requires C, 66.80; H, 7.37; N, 5.08%

### Example 220 Preparation of 7-(2R-carboxypyrrolidinocarbonyl-8-(1-adamantylmethyl-N-(methyl)aminocarbonyl)-2,3,5,6-dibenzobicyclo[2.2.2]octane (mixture of diastereoisomers)

### a. (±)-8-(1-adamantylmethyl-N-(methyl)aminocarbonylmethyl)-2,3,5,6-dibenzobicyclo[2.2.2]octane-7-carboxylic acid

This was prepared essentially as in example 14 except that N-methyl-1-adamantanemethylamine was used as substrate instead of 1-adamantanemethylamine.

### b. 7-(2R-benzyloxycarbonylpyrrolidinocarbonyl)-8-(1-adamantylmethyl-N-(methyl)aminocarbonyl)-2,3,5,6-dibenzobicyclo[2.2.2]octane (mixture of diastereoisomers)

This was prepared essentially as in example 46 except that the product of step a. was used as substrate instead of the product of example 14.

### c. 7-(2R-carboxypyrrolidinocarbonyl)-8-(1-adamantylmethyl-N-(methyl)aminocarbonyl)-2,3,5,6-dibenzobicyclo[2.2.2]octane (mixture of diastereoisomers)

This was prepared essentially as in example 48 except that the product of step b above was used as substrate instead of the product of example 46. The compound was further characterised and tested as the N-methyl-D-glucamine salt. Found: C, 63.54; H, 7.87; N, 5.32. C₄₂H₅₇N₃O₉.2.3 H₂O requires C, 63.91; H, 7.87; N, 5.32%

### Example 221 Preparation of 7-(2R-(carboxymethylaminocarbonyl)-pyrrolidinocarbonyl)-8-(1-adamantylmethyl-N-(methyl)aminocarbonyl)-2,3,5,6-dibenzobicyclo[2.2.2]octane (mixture of diastereoisomers)

The compound was prepared essentially as in example 194 except that the compound of example 220 was used as substrate instead of the compound of example 141 in step a. The compound was tested as the N-methyl-D-glucamine salt.

### Example 222 Preparation of 7-(2R-(1S-carboxyethylaminocarbonyl)pyrrolidinocarbonyl)-8-(1-adamantylmethyl-N-(methyl)aminocarbonyl)-2,3,5,6-dibenzobicyclo[2.2.2]octane (mixture of diastereoisomers)

The compound was prepared essentially as in example 184 except that the compound of example 220 was used as substrate instead of the compound of example 141 in step a. The compound was tested as the N-methyl-D-glucamine salt.

### Example 223 Preparation of 7-(2R-(1R-carboxyethylaminocarbonyl)-pyrrolidinocarbonyl)-8-(1-adamantylmethyl-N-(methyl)aminocarbonyl)-2,3,5,6-dibenzobicyclo[2.2.2]octane (mixture of diastereoisomers)

The compound was prepared essentially as in example 185 except that the compound of example 220 was used as substrate instead of the compound of example 141 in step a. The compound was tested as the N-methyl-D-glucamine salt.

### Example 224 Preparation of cis-7-(2S-(1R-carboxyethylaminocarbonylmethyl)pyrrolidinocarbonyl)-8-(1-adamantylmethyl-N-(methyl)aminocarbonyl)-2,3,5,6-dibenzobicyclo[2.2.2]octane (mixture of diastereomers)

### a. cis-7-(2S-(carboxymethyl)pyrrolidinocarbonyl)-8-(1-adamantylmethyl-N-(methyl)aminocarbonyl)-2,3,5,6-dibenzobicyclo[2.2.2]octane (mixture of diastereomers)

The reaction was performed essentially as in example 141 except that the compound of example 220 step a. was used instead of the compound of example 14.

### b. cis-7-(2S-(1R-carboxyethylaminocarbonylmethyl)-pyrrolidinocarbonyl)-8-(1-adamantylmethyl-N-(methyl)aminocarbonyl)-2,3,5,6-dibenzobicyclo[2.2.2]octane (mixture of diastereomers)

The compound was prepared essentially as in example 185 except that the compound from step a. above was used as substrate instead of the compound of example 141 in step a. The compound was tested as the N-methyl-D-glucamine salt.

### Example 225 Preparation of cis-7-(2S-(1S-carboxyethylaminocarbonylmethyl)pyrrolidinocarbonyl)-8-(1-adamantylmethyl-N-(methyl)aminocarbonyl)-2,3,5,6-dibenzobicyclo[2.2.2]octane (mixture of diastereomers)

The compound was prepared essentially as in example 184 except that the compound from example 224 step a. was used as substrate instead of the compound of example 141 in step a. The compound was tested as the N-methyl-D-glucamine salt.

### Example 226 Preparation of cis-7-(2R-(1S-carboxyethylaminocarbonylmethyl)pyrrolidinocarbonyl)-8-(1-adamantylmethyl-N-(methyl)aminocarbonyl-2,3,5,6-dibenzobicyclo[2.2.2]octane (mixture of diastereomers)

### a. cis-7-(2R-(carboxymethyl)pyrrolidinocarbonyl)-8-(1-adamantylmethyl-N-(Methyl)aminocarbonyl)-2,3,5,6-dibenzobicyclo[2.2.2]octane (mixture of diastereomers)

The reaction was performed essentially as in example 142 except that the compound of example 220 step a. was used instead of the compound of example 14.

### b. cis-7-(2R-(1S-carboxyethylaminocarbonylmethyl)pyrrolidinocarbonyl)-8-(1-adamantylmethyl-N-(methyl)aminocarbonyl)-2,3,5,6-dibenzobicyclo[2.2.2]octane (mixture of diastereomers)

The compound was prepared essentially as in example 184 except that the compound from step a. above was used as substrate instead of the compound of example 141 in step a. The compound was tested as the N-methyl-D-glucamine salt.

### Example 227 Preparation of cis-7-(2S-(methoxycarbonylmethylaminocarbonyl)pyrrolidinocarbonyl)-8-(1-adamantylmethylaminocarbonyl)-2,3,5,6-dibenzobicyclo[2.2.2]octane (mixture of diastereomers)

The compound was prepared essentially as in example 25 but using the methyl ester L-prolylglycine as substrate instead of L-alanine methyl ester. Found: C, 62.87; H, 7.47; N, 5.02. C₃₇H₄₃N₃O₅. 1.1 EtOAc and 4.5 H₂O requires C, 63.12; H, 7.78; N, 5.33% %

### Example 228 Preparation of cis-7-(2S-(1R-carboxyethylaminocarbonyl)pyrrolidinocarbonyl)-8-(1-adamantylmethylaminocarbonyl)-2,3,5,6-dibenzobicyclo[2.2.2]octane (mixture of diastereomers)

The compound was prepared essentially as in example 64 but using the benzyl ester L-prolyl-D-alanine in step a. instead of the dibenzyl ester of aspartic acid. The compound was further characterised and tested as the N-methyl-D-glucamine salt. Found: C, 60.11; H, 8.22; N, 6.22. C₄₄H₆₀N₄O₁₀ .4.4 H₂O requires C, 59.81; H, 7.84; N, 6.34%

### Example 229 Preparation of cis-7-(2R-(carboxymethylaminocarbonyl)-5-oxopyrrolidinocarbonyl)-8-(1-adamantylmethylaminocarbonyl)-2,3,5,6-dibenzobicyclo[2.2.2]octane (diastereomer 1)

The compound was prepared essentially as in example 64 but using the benzyl ester D-pyroglutamyl-glycine in step a. instead of the dibenzyl ester of aspartic acid. The product of step a. was separated into its constituent diastereomers by chromatography ( silica 30% ethyl acetate and 70% dichloromethane). The less polar material was hydrogenated to give the compound of this example. The compound was further characterised and tested as the N-methyl-D-glucamine salt. Found: C, 54.22; H, 7.26; N, 5.54. C₄₃H₅₆N₄O₁₁ .8.0 H₂O requires C, 54.42; H, 7.60; N, 5.90%

### Example 230 Preparation of cis-7-(2R-(carboxymethylaminocarbonyl)-5-oxopyrrolidinocarbonyl)-8-(1-adamantylmethylaminocarbonyl)-2,3,5,6-dibenzobicyclo[2.2.2]octane (diastereomer 2)

The compound was prepared essentially as in example 229 except that the more polar material isolated after the chromatography was hydrogenated to give the compound of this example. The compound was further characterised and tested as the N-methyl-D-glucamine salt. Found: C, 54.88; H, 7.20; N, 6.11. C₄₃H₅₆N₄O₁₁ .8.0 H₂O requires C, 54.42; H, 7.60; N, 5.90%

### Example 231 Preparation of cis-7-(2R-(1R-carboxyethylaminocarbonylmethyl)pyrrolidinocarbonyl)-8-(1-adamantylmethyl-N-(methyl) aminocarbonyl)-2,3,5,6-dibenzobicyclo[2.2.2]octane (mixture of diastereomers)

The compound was prepared essentially as in example 185 except that the compound from example 226 step a. was used as substrate instead of the compound of example 141 in step a.

### Example 232 Preparation of cis-7-(2S-(1R-(methoxycarbonyl)-ethylaminocarbonylmethyl)-pyrrolidinocarbonyl)-8-(1-adamantylmethylaminocarbonyl)-2,3,5,6-dibenzobicyclo[2.2.2]octane (single diastereomer)

The compound was prepared essentially as in example 63 except that the compound of example 186 was used as substrate instead of cis-7-(1-S-carboxyl-2-methylpropylaminocarbonyl)-8-(1-adamantylmethylaminocarbonyl)-2,3,5,6-dibenzobicyclo[2.2.2]octane (mixture of diastereomers)

### Example 233 Preparation of cis-7-(2R-(carboxymethylaninocarbonyl)-4-thiopyrrolidinocarbonyl)-8-(1-adamantylmethylaminocarbonyl)-2,3,5,6-dibenzobicyclo[2.2.2]octane

### a. cis-7-(2R-(t-butoxycarboylmethylaminocarbonyl)-4-thiopyrrolidinocarbonyl)-8-(1-adamantylmethylaminocarbonyl)-2,3,5,6-dibenzobicyclo[2.2.2]octane

This compound was prepared essentially as in example 25 except that 2R-(t-butoxycarbonylmethylaminocarbonyl)-4-thiopyrrolidine was used as substrate instead of L-alanine methyl ester.

### b. cis-7-(2R-(carboxymethylaminocarbonyl)-4-thiopyrrolidinocarbonyl)-8-(1-adamantylmethylaminocarbonyl)-2,3,5,6-dibenzobicyclo[2.2.2]octane

The compound of step a. above was treated with trifluoroacetic acid to give the title compound of this example.

### Example 234 Preparation of cis-7-(2R-(carboxymethylaminocarbonyl)-4-oxothio-pyrrolidinocarbonyl)-8-(1-adamantylmethylaminocarbonyl)-2,3,5,6-dibenzobicyclo[2.2.2]octane

This was prepared from the compound of example 233 step a. by treatment with ozone followed by treatment with trifluoroacetic acid.

### Example 235 Preparation of cis-7-(2R-(carboxymethylaminocarbonyl)-4-dioxothio-pyrrolidinocarbonyl)-8-(1-adamantylmethylaminocarbonyl)-2,3,5,6-dibenzobicyclo[2.2.2]octane

This was prepared from the compound of example 233 step a. by treatment with the tetrabutylammonium salt of oxone followed by treatment with trifluoroacetic acid.

### Example 236 Preparation of cis-7-(2R-(3,5-dicarboxyphenylaminocarbonyl)-pyrrolidinocarbonyl)-8-(1-adamantylmethylaninocarbonyl)-2,3,5,6-dibenzobicyclo[2.2.2]octane (diastereomer 1)

The compound was prepared essentially as in example 64 but using the dibenzyl ester of D-prolyl-5-aminoisophthalic acid in step a. instead of the dibenzyl ester of aspartic acid. The diastereomers were separated at the benzyl ester stage by column chromatography (silica, 92% dichloromethane and 8% ethyl acetate). The compound with the higher R_{f} was converted to the title compound by hydrogenation. The compound was further characterised and tested as the mono-N-methyl-D-glucamine salt, HPLC; R_{T}=17.4 mins, C8 column, CH₃CN 50%, H₂O 50%, 0.1% CH₃COOH.

### Example 237 Preparation of cis-7-(2R-(3,5-dicarboxyphenylaminocarbonyl)-pyrrolidinocarbonyl)-8-(1-adamantylmethylaminocarbonyl)-2,3,5,6-dibenzobicyclo[2.2.2]octane (diastereomer 2)

The compound was prepared essentially as an example 237 but using the compound with lower R_{f} after diastereomer separation in the final hydrogenation step. The compound was further characterised and tested as the mono-N-methyl-D-glucamine salt, HPLC; R_{T}=9.4 mins, C8 column, CH₃CN 60%, H₂O 40%, 0.1% CH₃COOH.

### Example 238 Preparation of cis-7-(2S-(1-R-carboxyethylaminocarbonylethyl)pyrrolidinocarbonyl)-8-(1-adamantylmethylaminocarbonyl)-2,3,5,6-dibenzobicyclo[2.2.2]octane (mixture of diastereomers)

The compound was prepared essentially as in example 185 but using the product of example 215 instead of the compound of example 141. The compound was further characterised and tested as the N-methyl-D-glucamine salt, HPLC; R_{T}=21.7 mins, C8 column, CH₃CN 50%, H₂O 50%, 0.1% CH₃COOH.

The following ¹H NMR data were obtained for the compounds described in the Examples:
Ex.1a. (d⁶-DMSO) δ 7.5 (2H, m), 7.3 (2H, m), 7.2 (4H, m), 4.8 (2H, s), 3.6 (2H,s).
Ex.1b. (d⁶-DMSO) δ 11.6 (1H, br s), 7.9 (1H, t), 7.4-6.9 (13H, m), 4.5 (1H, s), 4.4 (1H, s), 3.1 (1H, d), 2.9 (2H, m), 2.8 (1H, d), 2.5 (2H, t), 1.6 (2H, m).
Ex.2 (d⁶-DMSO) δ 11.6 (1H, br s), 10.8 (1H, s), 8.0 (1H, t), 7.6-6.8 (13H, m), 4.5 (1H, s), 4.3 (1H, d), 3.3-3.0 (3H, d), 2.2-2.4 (3H, m).
Ex.3 (d⁶-DMSO) δ 8.4 (1H, br s), 8.4 (1H, t), 7.4-6.8 (13H, m), 4.4 (2H, d), 4.1 (2H, m), 3.2 (1H, d), 2.8 (1H, d).
Ex.4 (d⁶-DMSO) δ 8.4 (1H, t), 8.0-6.9 (15H, m), 4.7 (1H, dd), 4.5 (1H, dd), 4.5 and 4.3 (2H, 2xs), 3.3 (1H, d), 2.8 (1H, d).
Ex.5 (d⁶-DMSO) δ 8.5 (1H, t), 8.0-6.9 (15H, m), 4.5 (2H, d), 4.3 (2H, m), 3.2 (1H, d), 2.8 (1H, d).
Ex.6 (d⁶-DMSO) δ 11.6 (1H, br s), 7.8 (1H, m), 7.4-6.3 (8H, m), 4.5 (1H, s), 4.4 (1H, s), 3.2-2.6 (4H, m), 2.2-0.9 (11H, m).
Ex.7 (d⁶-DMSO) δ 11.6 (1H, br s), 7.8 (1H, m), 7.4-6.9 (8H, m), 4.5 (1H, s), 4.4 (1H, s), 3.2 (1H, dd), 3.0-2.8 (2H, m), 2.8 (1H, dd) 1.2 (8H, m), 0.9 (3H, t).
Ex.8 (d⁶-DMSO) δ 11.6 (1H, br s), 7.8 (1H, t), 7.4-6.9 (8H, m), 4.5 (1H, d), 4.4 (1H, d), 3.1 (1H, dd), 3.0-2.8 (2H, m), 2.7 (1H, dd) 1.3 (12H, m), 0.9 (3H, t).
Ex.9 (d⁶-DMSO) δ 11.6 (1H, br s), 7 8 (1H, t), 7.4-6.9 (8H, m), 4.5 (1H, d), 4.4 (1H, d), 3.1 (1H, dd), 2.8-2.6 (3H, m), 1.8-0.7 (11H, m).
Ex.10 (d⁶-DMSO) δ 12.6 (1H b or s), 7.7 (1H, t), 7.4-6.9 (8H, m), 4.5 (1H, d), 4.4 (1H, d), 3.0 (1H, dd), 2.9 (2H, m), 2.8 (1H, dd), 1.3 (2H, m), 0.9 (9H, s).
Ex.11 (d⁶-DMSO) δ 7.3 (3H, m), 7.2 (1H, m), 7.1 (5H, m), 4.5 (1H, d), 4.4 (1H, d), 3.1 (1H, dd), 2.7 (1H, dd), 1.9 (3H, s), 1.8 (6H, m), 1.6 (6H, m).
Ex.12 (d⁶-DMSO) δ 7.6 (1H, t), 7.4-6.9 (8H, m), 4.5 (1H, d), 4.4 (1H, d), 3.0 (1H, dd), 2.9 (2H, m), 2.8 (1H, dd), 1.9 (3H, s), 1.6 (6H, m), 1.4 (6H, m), 1.1 (2H, t).
Ex.13 (d⁶-DMSO) δ 12.2 (1H br s), 7.4-7.0 (8H, m), 4.6 (1H, dd), 3.4 (1H, d), 3.3 (1H, d), 3.1 (1H, d), 1.9 (3H, s), 1.6 (6H, m), 1.4 (6H, m).
Ex.14 (d⁷-DMF) δ 7.7 (1H, t), 7.4 (3H, m), 7.2 (3H,m), 7.1 (2H, m), 4.7 (1H, d), 4.6 (1H, d), 3.5 (1H, dd), 3.0 (1H, dd), 2.9 (1H, dd), 2.7 (1H, dd), 2.0 (3H, s), 1.7 (6H, m), 1.5 (6H, s).
Ex.15 (d⁶-DMSO) δ 7.9 (1H, t), 7.0-7.4 3H, m), 6.6 (1H,t), 4.5 (2H, d), 3.8-3.5 (2H, 2xdd), 3.6 (3H, s), 3.2 (1H, d), 3.0 (1H, dd), 2.5 (1H, dd), 1.9 (3H, b s), 1.2-1.7 (12H, m).
Ex.16a (d⁶DMSO) δ 8.0 (1H, m), 7.4-7.0 (13, m), 6.6 (1H, m), 5.1 (2H, s), 4.4 (2H, s), 3.8 (1H, dd), 3.6 (1H, dd), 3.1 (1H, m), 3.0 (1H, d), 2.5 (2H, m), 1.9 (3H, s), 1.6 (6H, q), 1.3 (6H, d).
Ex.16b (d⁶-DMSO) δ 7.8 (1H, m), 7.3-7.0 (8H, m), 6.6 (1H, m), 4.5 (2H, s), 3.7 (1H, dd), 3.4 (1H, m), 3.1 (1H, d), 2.9 (1H, d), 2.5 (2H, m), 1.9 (3H, s), 1.6 (6H, q), 1.2 (6H, d).
Ex.17 (CDCl₃) δ 7.6 (1H, d), 7.1-7.4 (7H, m), 4.9 (1H, t), 4.6 (1H, d), 4.5 (1H, d), 3.6 (3H, s), 3.3 (1H, dd), 3.2 (1H, dd), 2.9 (1H, m), 2.6 (1H, m), 2.0 (3H, s), 1.6 (6H, m), 1.3 (6H, s).
Ex.18a (CDCl₃) δ 7.6-7.1 (8H, m), 6.0 (1H, d), 5.9 (1H, d), 4.0 (1H, dd), 3.8 (1H, dd).
Ex.18b (CDCl₃) δ 7.8-6.9 (15H, m), 4.9 (2H, d), 4.6 (1H, d), 4.4 (1H, d), 4.0 (1H, dd),3.8 (1H, dd).
Ex.18c (d⁶-DMSO) δ 9.0 (1H, bs), 7.9-7.0 (15H, m), 5.4 (1H, s), 4.6 (2H, d), 4.4 (1H, m), 4.3 (1H, m), 3.1 (1H, m), 2.9 (1H, m).
Ex.19 (d⁶-DMSO) δ 8.9 (1H, bs), 7.4-6.8 (13H, m), 4.6 (2H, s), 4.3 (1H, dd), 4.1 (1H, dd), 3.5 (1H, m), 3.0 (1H, dd), 2.8 (1H, dd).
Ex.20a (CDCl₃) δ 7.6-7.1 (8H, m), 5.0 (1H, d), 4.9 (1H, d), 4.2 (1H, dd), 4.1 (1H, dd)
Ex.20b (d⁶-DMSO) δ 8.2 (1H, bs), 7.9-6.9 (15H, m), 4.7 and 4.5 (2H, 2xs), 4.2 (1H, d), 4.1 (1H, d), 3.1 (2H, m).
Ex.21 (d⁶-DMSO) δ 10.8 (1H, s), 8.0 (1H, bs), 7.5-6.8 (13H, m), 4.7 and 4.4 (2H, 2xs), 3.2-2.9 (6H, m).
Ex.22 (d⁶-DMSO) δ 7.7 (1H, bs), 7.4-6.9 (8H, m), 4.7 and 4.4 (2H, 2xs), 3.1 (2H, m), 2.7 (1H, dd), 2.3 (1H, d), 1.8 (3H, s), 1.6 (6H, m), 1.2 (6H, m).
Ex.23 (d⁶-DMSO) δ 8.0 (1H, t), 7.9 (1H, m), 7.4-7.0 (8H, m), 4.6 (2H, d), 3.9 (1H, m), 3.2 (1H, m), 3.1 (1H, m), 2.9 (1H, m), 2.5 (1H, m), 1.9 (3H, s), 1.6 (6H, q), 1.4 (6H, s), 1.2 (3H, dd).
Ex.24 (CDCl₃) δ 7.5-7.1 (8H, m), 6.2 (1H, t), 5.3 (1H, t), 4.5 (2H, s), 3.7 (3H, s), 3.3 (2H, m), 3.2 (2H, q), 2.7 (2H, ddd), 2.4 (2H, t), 1.9 (3H, s), 1.4 (6H, q), 1.2 (6H, s).
Ex.25 (CDCl₃) δ 7.5-7.1 (8H, m), 5.9 and 5.7 (1H, 2 xd), 5.3 and 5.1 (1H, 2 x t), 4.6 (2H, m), 4.3 (1H, m), 3.7 (3H, s), 3.3-3.1 (2H, dd), 2.9-2.5 (2H, m), 1.9 (3H, s), 1.7 (6H, q), 1.3 (6H, d), 1.2 and 0.9 (3H, d).
Ex.26 (CDCl₃) δ 7.5-7.1 (8H, m), 5.7 (1H, d ), 5.3 (1H, t), 4.6 (2H, m), 4.3 (1H, m), 3.7 (3H, s), 3.2 (2H, s), 2.8-2.6 (2H, dd), 1.9 (3H, s), 1.7 (6H, q), 1.3 (6H, d), 1.1 (3H, d).
Ex.27 (CDCl₃) δ 7.5-7.1 (8H, m), 6.1 (1H, t ), 5.2 (1H, d), 4.6 (2H, m), 4.2 (1H, m), 3.7 (3H, s), 3.2 (2H, dd), 2.8-2.5 (2H, dd), 1.9 (3H, s), 1.6 (6H, q), 1.3 (6H, d), 0.9 (3H, d).
Ex.28 (CDCl₃) δ 7.5-7.1 (8H, m), 6.1 and 5.9 (1H, 2 x d), 5.4 and 5.2 (1H, 2 x t), 4.6 (2H, d), 4.3 (1H, m), 3.7 (3H, s), 3.2 (2H, ddd), 2.9-2.5 (2H, m), 2.0 (3H, s), 1.7 (6H, q), 1.3 (6H, d), 1.2 (3H, d).
Ex.29 (CDCl₃) δ 7.5-7.1 (13H, m), 5.8 (1H, t), 5.2 (2H, s), 5.1(1H, t) 4.6 (2H, d), 3.3 (2H, q), 3.3-3.0 (2H, q), 2.8-2.6 (2H, ddd), 2.4 (2H, q), 1.8 (3H, s), 1.7 (6H, q), 1.3 (6H, d).
Ex.30 (CDCl₃) δ 7.6 (2H, d), 7.3-7.1 (7H, m), 4.8(1H, t) 4.6 (1H, d), 4.5 (1H, d), 3.5 (1H, m), 3.3 (1H, d), 3.1 (2H, m), 2.9 (1H, q), 2.4 (3H, m), 1.9 (3H, s), 1.6 (6H, q), 1.2 (6H, d).
Ex.31 (d⁶-DMSO) δ 7.5-6.8 (12H, m), 4.5 (2H, m), 4.0 (1H, m), 3.3-2.5 (4H, m), 1.9 (3H, s), 1.6 (6H, m), 1.4 and 1.3 (6H, 2 x s), 1.1 and 1.0 (3H, 2 x d).
Ex.32 (d⁶-DMSO) δ 7.3 (4H, m), 7.1 (4H, m), 6.9-6.6 (2H, m), 4.5 and 4.3 (1H, 2 x t), 4.5 (2H, m), 3.6 (1H, m), 3.2 (1H, m), 3.0-2.8 (3H, m), 2.5 (2H, m), 1.9 (3H, s), 1.6 (6H, m), 1.3 (6H, m), 0.9 and 0.8 (3H, 2 x d).
Ex.33 (CDCl₃) δ 7.4-7.1 (13H, m), 5.7 (1H, d), 5.3 (1H, t), 5.1 (2H, s), 4.6 (1H, s), 4.5 (1H, s), 4.3 (1H, m), 3.2 (2H, s), 2.8 (1H, dd), 2.6 (1H, dd), 1.9 (3H, s), 1.6 (6H, q), 1.3 (6H, d), 1.1 (3H, d).
Ex.34 (CDCl₃) δ 7.5-7.1 (13H, m), 6.0 (1H, d), 5.1 (3H, m), 4.6 (2H, m), 4.4 (1H, m), 3.3 (1H, dd), 3.2 (1H, dd) 2.9 (1H, dd), 2.5 (1H, dd), 2.0 (3H, s), 1.7 (6H, q), 1.3 (9H, m).
Ex.35 (d⁶-DMSO) δ 7.5 (1H, d), 7.4-7.2 (4H, m), 7.0 (4H, m), 6.8 (1H, t), 4.5 (2H, s), 4.0 (1H, m), 3.0 (2H, m), 2.5 (2H, m), 1.8 (3H, s), 1.6 (6H, q), 1.2 (6H, m), 1.1 (3H, d).
Ex.36 (d⁶-DMSO) δ 7.7 (1H, d), 7.3 (2H, m), 7.2 (2H, m), 7.1 (2H, m), 7.0 (2H, m), 6.6 (1H, t), 4.5 (2H, s), 4.0 (1H, m), 3.0 (1H, d), 2.9 (1H, d), 2.6 (2H, m), 1.9 (3H, s), 1.6 (6H, q), 1.3 (6H, m), 1.1 (3H, d).
Ex.37b (d⁶-DMSO) δ 7.6 (1H, t), 7.3 (2H, m), 7.0 (4H, m), 4.8 (1H, d), 4.6 (1H, d), 3.7 (6H, 2 x s), 3.1 (1H, dd), 2.7-2.4 (3H, m), 1.9 (3H, s), 1.6 (6H, q), 1.4 (6H, m).
Ex.38 (d⁶-DMSO) δ 7.4 (1H, t), 7.3 (2H, m), 7.0 (2H, m), 6.6 (2H, m), 4.8 (1H, d), 4.7 (1H, d), 3.7 (6H, 2 x s), 3.1 (1H, dd), 2.7-2.4 (3H, m), 1.9 (3H, s), 1.6 (6H, q), 1.4 (6H, m).
Ex.39 (d⁶-DMSO) δ 11.6 (1H, bs), 7.3 (1H, t), 7.3 (3H, m), 7.0 (5H, m), 4.5 (1H, d), 4.4 (1H, d), 3.1 (2H, m), 2.9 (1H, m), 2.7 (1H, dd), 2.0-1.4 (15H, m).
Ex.40 (d⁶-DMSO) δ 7.7 and 7.5 (1H, 2 x d), 7.4-7.0 (8H, m), 6.8 and 6.7 (1H, 2 x t), 4.5 (1H, m), 4.4 (2H, s), 3.0-2.7 (8H, m), 2.5 (2H, m), 1.9 (3H, s), 1.6 (6H, m), 1.3 (6H, 2 x s), 1.1 and 1.0 (3H, 2 x d).
Ex.41 (CDCl₃) δ 7.5-7.1 (8H, m), 6.0 and 5.7 (1H, 2 x d), 5.4 and 5.0 (1H, 2 x t), 4.6 (2H, m), 4.3 (1H, m), 3.7 (3H, s), 3.3-3.1 (2H, m), 3.0-2.6 (2H, m), 1.6-1.4 (5H, m), 1.2-1.1 (7H, m),0.7 (2H, m).
Ex.42 (CDCl₃) δ 7.6 (1H, m), 7.3-7.0 (7H, m), 4.6 (3H, m), 4.3 (1H, m), 3.7 (3H, s), 3.5-3.3 (3H, m), 3.0 and 2.8 (3H, 2 x s), 2.8 (1H, m), 2.3 (1H, m), 1.9 (3H, s), 1.6 (6H, q), 1.1 (6H, d).
Ex.43 (CDCl₃) δ 7.6 (1H, m), 7.3-7.0 (7H, m), 4.6 (3H, m), 4.3 (1H, m), 4.2 (2H, m), 3.5-3.3 (3H, m), 3.0 and 2.8 (3H, 2 x s), 2.8 (1H, m), 2.4 (1H, m), 1.9 (3H, s), 1.6 (6H, q), 1.3 (3H, m), 1.1 (6H, d).
Ex.44a (d⁶-DMSO) δ 12.7 (1H, s), 7.4-7.1 (8H, m) 4.8 (2H, m), 4.0 (2H, m) 3.2 (1H, dd), 3.15 (1H, dd), 1.43 (3H, t).
Ex.44b (d⁶-DMSO) δ 8.1 (1H, t), 7.4-7.0 (8H, m), 4.7 (1H, d), 4.65 (1H, d), 4.0 (2H, m), 3.4 (1H, dd), 3.1 (1H, dd), 2.9 (1H, dd), 2.6 (1H, dd), 1.9-1.4 (15H, m), 1.1 (3H, t).
Ex.44c (d⁶-DMSO) δ 15-13 (1H, br s), 7.5-7.0 (9H, m), 4.8 (2H, s), 3.2 (2H, s), 2.7 (2H, s), 1.7-0.9 (15H, m).
Ex.45 (CDCl₃) δ 7.3 (4H, m), 7.1 (4H, m), 4.6 (2H, m), 3.5 (3H, s), 3.4-3.6 (2H, m), 3.2 (1H, 2 x s), 2.0 (3H, br s), 1.7 (6H, q), 1.4 (6H, d).
Ex.46 (CDCl₃) δ 7.6 (1H, m), 7.3 (13H, m), 5.4-5.0 (2H, m), 4.9-4.4 (3H, m), 3.7-3.1 (4H, m), 3.0-2.2 (2H, m), 2.2-1.8 (7H, m), 1.6 (6H, q), 1.2(6H, m).
Ex.47 (CCCl₃) δ 7.6 (1H, m), 7.3 (13H, m), 5.4-5.0 (2H, m), 4.9-4.4 (3H, m), 3.7-3.1 (4H, m), 3.0-2.2 (2H, m), 2.2-1.8 (7H, m), 1.6 (6H, q), 1.2 (6H, m).
Ex.48 (CDCl₃) δ 7.6 (1H, m), 7.3 (7H, m), 5.3 (1H, br s), 4.7-4.2 (3H, m), 3.8-3.2 (5H, m), 3.1 and 2.8 (1H, m) 2.3 (2H, m), 2.0 (5H, m), 1.6 (6H, q), 1.2 (6H, m).
Ex.49 (CDCl₃) δ 7.6 (1H, m), 7.3 (7H, m), 5.3 (1H, br s), 4.7-4.2 (3H, m), 3.8-3.2 (5H, m), 3.1 and 2.8 (1H, m) 2.3 (2H, m), 2.0 (5H, m), 1.6 (6H, q), 1.2 (6H, m).
Ex.50 (CDCl₃) δ 7.6 (1H, m), 7.3 (7H, m), 4.7-4.3 (4H, m), 3.8-3.2 (7H, in), 3.0 and 2.8 (1H, m), 2.4-1.8 (8H, m), 1.6 (6H, q), 1.2 (6H, m).
Ex.51 (CDCl₃) δ 7.6 (1H, m), 7.3 (7H, m), 4.7-4.3 (4H, m), 3.8-3.2 (7H, m), 3.0 and 2.8 (1H, m), 2.4-1.8 (8H, m), 1.6 (6H, q), 1.2 (6H, m).
Ex.52 (d⁶-DMSO) δ 10.8 (1H, s), 7.5 (1H, d), 7.3 (6H, m), 7.0 (7H, m), 6.5 (1H, m), 4.5 (1H, m), 4.4 (1H, m), 3.1 (2H, s), 3.0 (1H, dd), 2.9 (1H, dd), 2.7 (2H, m), 2.5 (2H, m), 1.8 (3H, s), 1.6 (6H, q), 1.3 (6H, d).
Ex.53 (d⁶-DMSO) δ 10.9 (1H, 2 x s), 7.9 and 7.6 (1H, 2 x d), 7.5-7.2 (6H, m), 7.1 (7H, m), 6.6 (1H, m), 4.5 (2H, m), 4.3 (1H, m), 3.5 (3H, s), 3.0 (4H, dd), 2.5 (2H, m), 1.8 (3H, s), 1.6 (6H, q), 1.2 (6H, d).
Ex.54 (d⁶-DMSO) δ 10.9 (1H, 2 x s), 7.9 and 7.6 (1H, 2 x d), 7.5-7.2 (6H, m), 7.1 (7H, m), 6.6 (1H, m), 4.5 (2H, m), 4.3 (1H, m), 3.5 (3H, s), 3.0 (4H, dd), 2.5 (2H, m), 1.8 (3H, s), 1.6 (6H, q), 1.2 (6H, d).
Ex.55a (diastereomer 1, higher R_{f}) (d⁶-DMSO) δ 10.9 (1H, s), 7.5-6.9 (19H, m), 6.7 (1H, m), 4.9 (2H, m), 4.5 (2H, m), 4.3 (1H, m), 3.0 (4H, m), 2.5 (2H, m), 1.8 (3H, s), 1.6 (6H, q), 1.2 (6H, d).
Ex.55a (diastereomer 2, lower R_{f}) (d⁶-DMSO) δ 10.8 (1H, s), 7.9 (1H, d), 7.4-6.9 (18H, m), 6.6 (1H, m), 5.0 (2H, m), 4.5 (1H, s), 4.42 (1H, s), 4.38 (1H, m), 3.1-2.9 (4H, m), 2.5 (2H, m), 1.8 (3H, s), 1.6 (6H, q), 1.2 (6H, d).
Ex.55b (d⁶-DMSO) δ 10.9 (1H, s), 7.5 (2H, m), 7.4-6.9 (12H, m), 6.6 (1H, t), 4.4 (2H, m), 4.2 (1H, s), 3.0 (4H, m), 2.5 (2H, m), 1.9 (3H, s), 1.6 (6H, q), 1.2 (6H, m).
Ex.56 (d⁶-DMSO) δ 10.8 (1H, s), 7.8 (1H, d), 7.4-6.9 (13H, m), 6.5 (1H, t), 4.5 (1H, s), 4.4 (1H, s), 4.3 (1H, m), 3.1 (1H, m), 3.0 (1H, dd), 2.9 (2H, m), 2.4 (2H, m), 1.8 (3H, s), 1.6 (6H, q), 1.2 (6H, m).
Ex.57 (d⁶-DMSO) δ 10.9 (1H, s), 7.6 (2H, m), 7.4-6.9 (12H, m), 6.6 (1H, t), 4.4 (2H, m), 4.2 (1H, s), 3.0 (4H, m), 2.5 (2H, m), 1.8 (3H, s), 1.6 (6H, q), 1.2 (6H, m).
Ex.58 (CDCl₃) δ 8.1 (1H, s), 7.6 (1H, d), 7.4-6.9 (11H, m), 6.6 (1H, m), 6.5 (1H, m), 5.5 (1H, m), 4.8 (1H, m), 4.4 (1H, s), 4.2 (1H, m), 3.2 (4H, m), 2.7 (1H, m), 2.3 (1H, m), 2.0 (3H, s), 1.6 (6H, q), 1.2 (6H, m).
Ex.59 (d⁶-DMSO) δ 8.6 (1H,t) 8.1 (1H,t), 7.3-7.0 (9H,m), 6.4 (1H,d), 6.2 (1H,d), 4.6 (2H,dd), 4.2 (2H,m), 3.2 (2H,d), 2.9(1H,m), 2.5 (1H,m), 1.9 (3H,s), 1.6 (6H,q), 1.4 (6H,d).
Ex.60 (CDCl₃) δ 7.7 (1H,d), 7.4 (1H,d), 7.3-7.0 (9H,m), 4.9 (1H,s), 4.7 (1H,d), 4.6 (1H,t), 3.9 (3H,m), 3.3 (2H,s), 2.6 (1H,q), 2.2 (1H,q), 1.7 (3H,s), 1.5(6H,q), 0.9 (6H,s).
Ex.61 (CDCl₃) δ 7.6-7.0 (13H, m), 6.1 and 5.8 (1H 2xd), 5.5 and 5.3 (1H, 2xt), 5.1 (2H, m), 4.6-4.2 (3H, m), 3.3-3.1 (2H, m), 2.9 (1H, m), 2.6 (1H, m), 1.9 (4H, s), 1.6 (6H, m), 1.3 (6H, m), 0.8-0.6 (6H, m).
Ex.62 (CDCl₃) δ 7.5-7.1 (8H, m), 6.9 and 6.5 (1H 2xm), 5.5 and 5.4 (1H, 2xm), 4.5 (2H, m), 4.3 and 4.2 (1H, m), 3.3 (2H, m), 3.0-2.4 (2H, m), 2.0 (4H, s), 1.6 (6H, m), 1.3 (6H, m), 0.8 (6H, m).
Ex.63 (CDCl₃) δ 7.5-7.2 (8H, m), 5.9 and 5.7 (1H 2xd), 5.3 and 5.2 (1H, m), 4.6(2H, bt), 4.2 (1H, m), 3.7 (3H, d), 3.3 (2H, m), 2.9 and 2.6 (2H, m), 1.9 (4H, s), 1.6 (6H, m), 1.3 (6H, m), 0.98-0.76 (6H, m).
Ex.64b (d⁶-DMSO) δ 12.4 (1H, br s), 7.8-6.8 (11H, m), 4.4 (2H, m), 4.2 (1H, m), 3.0 (2H, m), 2.6-2.3 (4H, m), 1.9 (3H, s), 1.6 (6H, m), 1.3 (6H, m).
Ex.65 (d⁶-DMSO) δ 12.6 (1H, br s), 7.9-6.9 (14H, m), 6.6 and 6.5 (1H, 2xt), 4.5 (2H, m), 4.4 (1H, m), 3.0 (2H, m), 2.8-2.3 (4H, m), 1.9 (3H, s), 1.6 (6H, m), 1.3 (6H, m).
Ex.66 (d⁶-DMSO) δ 7.3-7.0 (10H, m), 4.5 (1H, s), 4.4 (1H, s), 3.6 (3H, s), 3.0 (2H, m), 2.7 (1H, m), 2.5 (1H, m), 1.9 (3H, s), 1.6 (6H, m), 1.3 (6H, m), 1.1 (6H, 2 x s).
Ex.67 (d⁶-DMSO) δ 7.4-7.0 (10H, m), 4.5 (1H, s), 4.4 (1H, s), 3.1 (1H, m), 2.9 (1H, m), 2.7 (1H, m), 2.5 (1H, m), 1.9 (3H, s), 1.6 (6H, m), 1.3 (6H, m), 1.1 (6H, 2 x s).
Ex.68 (CDCl₃) δ 7.6 (1H, m), 7.4-7.0 (7H, m), 5.8 (1H, brs), 4.9-4.3 (5H, m), 3.7-3.1 (4H, m), 2.9-2.1 (4H, m), 2.0 (3H, s), 1.6 (6H, m), 1.3 (6H, m).
Ex.69 (CDCl₃) δ 7.6 (1H, d), 7.4-7.1 (8H, m), 4.7 (1H, t), 4.6 (1H, d), 4.5 (1H, d), 4.4 (2H, m), 3.8 (1H, m), 3.6 (1H, d), 3.4 (1H, dd), 3.2 (1H, d), 2.8 (1H, dd), 2.4 (2H, m), 2.2 (1H, m), 2.0 (3H, s), 1.6 (6H, m), 1.3 (6H, m).
Ex.70 (CDCl₃) δ 7.6 (1H, m), 7.4-7.0 (7H, m), 5.8 (1H, brs), 4.9-4.3 (5H, m), 3.7-3.1 (4H, m), 2.9-2.1 (4H, m), 2.0 (3H, s), 1.6 (6H, m), 1.3 (6H, m).
Ex.71 (CDCl₃) δ 7.6 (1H, d), 7.4-7.1 (8H, m), 4.7 (1H, d), 4.6 (1H, d), 4.4 (3H, m), 3.8 (3H, s), 3.7 (1H, m), 3.6-3.2 (4H,m), 2.8 (1H, dd), 2.4 (1H, dd), 2.1 (1H, m), 1.9 (3H, s), 1.6 (6H, m), 1.2 (6H, m).
Ex.72 (CDCl₃) δ 7.6 (1H, m), 7.4-7.0 (8H, m), 4.8-4.4 (5H, m), 3.9-3.1 (8H, m), 2.9-2.1 (3H, m), 2.0 (3H, s), 1.6 (6H, m), 1.3 (6H, m).
Ex.73 (CDCl₃) δ 7.6 (1H, m), 7.4-7.0 (8H, m), 4.8-4.4 (4H, m), 3.9-3.7 (3H, m), 3.3-2.6 (3H, m), 2.5-2.1 (3H, m), 2.0 (3H, s), 1.6 (8H, m), 1.1 (6H, m).
Ex.74 (CDCl₃) δ 7.6 (1H, m), 7.4-7.0 (8H, m), 4.8-4.2 (4H, m), 3.9-2.1 (12H, m), 2.0 (3H, s), 1.6 (8H, m), 1.1 (6H, m).
Ex.75 (CDCl₃) δ 7.5-7.1 (8H, m), 6.2 and 6.1 (1H, 2 xd), 5.4 and 5.3 (1H, 2 x t), 4.5 (3H, s), 3.3-3.1 (2H, m), 2.7 (2H, m), 1.9 (3H, s), 1.7 (6H, m), 1.3 (9H, m).
Ex.76 (CDCl₃) δ 7.5-7.1 (8H, m), 6.2 and 5.9 (1H, 2 xd), 5.4 and 5.2 (1H, 2 x t), 4.6 (2H, m), 4.3 (1H, m), 3.2 (2H, m), 2.9-2.4 (2H, m), 2.1 (3H, s), 1.9 (3H, s), 1.7 (6H, m), 1.3 (9H, m).
Ex.77 (CDCl₃) δ 7.5-7.0 (8H, m), 6.2 and 5.8 (1H, 2 x d), 5.4 and 5.1 (1H, 2 x t), 4.6 (2H, m), 4.3 (1H, m), 4.1 (2H, m), 3.5-3.2 (2H, m), 2.8 (1H, m), 2.6 (1H, m), 1.9 (3H, s), 1.7 (8H, m), 1.2 (9H, m), 0.9 (3H, m)
Ex.78 (CDCl₃) δ 7.6 (1H, d), 7.4-7.1 (8H, m), 4.6-4.2 (3H, m), 3.6-3.2 (5H, m), 2.8 (1H, dd), 2.4 (2H, m), 2.0 (5H, m), 1.6 (6H, m), 1.3 (6H, m).
Ex.79 (CDCl₃) δ 7.6-7.1 (8H, m), 5.4 (1H, br s), 4.86-4.4 (3H, m), 3.7-2.8 (6H, m), 2.4 (2H, m), 2.0 (5H, m), 1.6 (6H, m), 1.3 (6H, m).
Ex.80 (CDCl₃) δ 7.6 (1H, d), 7.4-7.1 (7H, m), 4.6-4.3 (4H, m), 3.8 (3H, s), 3.5 (3H, m), 3.2 (1H, dd), 2.8 (1H, dd), 2.4 (1H, dd), 2.0 (7H, m), 1.6 (6H, m), 1.2 (6H, s).
Ex.81 (CDCl₃) δ 7.6-7.1 (8H, m), 4.8-4.4 (4H, m), 3.7 and 3.6 (3H, 2 x s), 3.5-2.9 (5H, m), 2.4-1.8 (8H, m), 1.6 (6H, m), 1.2 (6H, m).
Ex.82 (d⁶-DMSO) δ 7.6-6.9 (8H, m), 4.5 (2H, m), 4.1 (1H, m), 3.7-3.3 (2H, m), 3.1 (2H, m), 2.5 (2H, m), 1.9 (3H, s), 1.6 (6H, m), 1.2 (6H, m).
Ex.83 (d⁶-DMSO) δ 7.7-6.9 (8H, m), 4.5 (2H, m), 4.1 (1H, m), 3.6-3.3 (2H, m), 3.1 (2H, m), 2.6 (2H, m), 1.9 (3H, s), 1.6 (6H, m), 1.2 (6H, m).
Ex.84 (CDCl₃) δ 7.6 (1H, d), 7.3-7.1 (7H, m), 6.6 (1H, d), 5.9 (1H, t), 4.6 (1H, d), 4.4 (2H, m), 3.8 (3H, s), 3.7 (2H, m), 3.6 (1H, m) 3.2 (1H, dd), 3.0 (1H, dd), 2.9 (1H, dd), 2.7 (1H, dd), 2.0 (3H, s), 1.7 (6H, m), 1.4 (6H, m).
Ex.85 (CDCl₃) δ 7.5-7.1 (8H, m), 6.5 (1H, d), 5.6 (1H, t), 4.5 (2H, 2 x s), 4.4 (1H, m), 3.7 (6H, m), 3.3 (1H, dd), 3.2 (1H, dd), 2.9 (1H, dd), 2.5 (1H, dd), 2.0 (3H, s), 1.7 (6H, m), 1.3 (6H, m).
Ex.86 (CDCl₃) δ 7.7-7.1 (8H, m), 6.4 (1H, s), 5.8 (1H, s), 5.3 (1H, s), 5.0 (1H, t), 4. (1H, d), 4.5 (1H, d), 3.8 (3H, s), 3.4 (1H, dd), 3.2 (1H, dd), 2.7 (1H, dd), 2.5 (1H, dd), 1.9 (3H, s), 1.6 (6H, m), 1.2 (6H, s).
Ex.87 (d⁶-DMSO) δ 8.0-6.9 (9H, m), 4.9 and 4.8 (1H, 2 x m), 4.6-4.3 (3H, m), 3.5-2.7 (7H, m), 2.5-2.2 (2H, m), 1.8 (3H, s), 1.5 (6H, m), 1.1 (6H, m).
Ex.88 (CDCl₃) δ 7.6 (1H, m), 7.4-7.0 (8H, m), 5.5-5.2 (1H, m), 4.6-4.4 (3H, m), 3.7- 3. (3H, m), 3.1-2.6 (2H, m), 2.3 (2H, m), 1.9 (3H, s), 1.6 (6H, m) 1.4 (5H, m), 1.2 (6H, m).
Ex.89 (CDCl₃) δ 7.6 (1H, m), 7.4-7.0 (7H, m), 5.3-4.3 (4H, m), 3.8-2.6 (8H, m), 2.3 (2H, m), 1.9 (1H, s), 1.6 (6H, m), 1.4 (5H, m), 1.2 (6H, m).
Ex.90 (d⁶-DMSO) δ 8.1-6.9 (9H, m), 4.9-4.1 (5H, m), 3.7-3.1 (7H, m), 2.9-2.1 (4H, m), 2.0 (3H, s), 1.6 (6H, m), 1.3 (6H, m).
Ex.91 (d⁶-DMSO) δ 8.1-6.9 (9H, m), 4.9-4.1 (5H, m), 3.8-3.1 (7H, m), 2.9-2.1 (4H, m), 2.0 (3H, s), 1.6 (6H, m), 1.1 (6H, m).
Ex.92 (d⁶-DMSO) δ 7.5-7.0 (13H, m), 6.4 (1H, d), 5.2 (1H, t), 4.5 (3H, m), 3.7 (3H, s), 3.6 (2H, s), 3.2 (2H, dd), 2.6 (4H, m), 2.0 (3H, s), 1.6 (6H, m), 1.2 (6H, s).
Ex.93 (CDCl₃) δ 7.4-6.9 (13H, m), 6.3 (1H, d), 5.2 (1H, t), 4.5 (3H, m), 3.6 (3H, s), 3.5 (2H, s), 3.1 (2H, dd), 2.6 (4H, m), 1.9 (3H, s), 1.6 (6H, m), 1.2 (6H, s).
Ex.94 (d⁶-DMSO) δ 7.3 (3H, m), 7.1 (6H, m), 4.9-4.5 (3H, m), 3.3 (1H, m), 3.0 (1H, m), 2.8-2.5 (5H, m), 1.9 (3H, s), 1.6 (6H, m), 1.4 (6H, m), 1.1 (3H, m).
Ex.95 (d⁶-DMSO) δ 7.3 (3H, m), 7.1-6.9 (6H, m), 4.9-4.5 (3H, m), 3.3 (1H, m), 3.0 (1H, m), 2.8-2.5 (5H, m), 1.9 (3H, s), 1.6 (6H, m), 1.4 (6H, m), 1.1 (3H, m).
Ex.96 (d⁶-DMSO) δ 7.4 (3H, m), 7.1 (5H, m), 5.0-4.4 (3H, m), 3.6 and 3.5 (3H, 2 x s), 3.2 (1H, m), 3.0 (1H, m), 2.8 and 2.7 (3H, 2 x s), 2.5 (2H, m), 1.9 (3H, s), 1.6 (6H, m), 1.3 (6H, m), 1.2-0.9 (3H, m).
Ex.97 (d⁶-DMSO) δ 7.4 (3H, m), 7.1-6.9 (5H, m), 5.0-4.3 (3H, m), 3.6 and 3.5 (3H, 2 x s), 3.3 (1H, m), 3.0 (1H, m), 2.8-2.5 (5H, m), 1.9 (3H, s), 1.6 (6H, m), 1.3-1.0 (9H, m).
Ex.98 (CDCl₃) δ 7.7 (1H, d), 7.4-7.1 (7H, m), 4.6 (2H, m), 4.5 (1H, t), 3.5 (1H, m), 3.4 (2H, m), 3.3 (2H, m), 3.1 (1H, m), 2.7 (1H, q), 2.4 (1H, q), 1.9 (3H, s), 1.9-1.8 (4H, m), 1.6 (6H, m), 1.2 (6H, m).
Ex.99 (d⁶-DMSO) δ 7.4-7.0 (9H, m), 6.4 (1H, t), 4.4 (2H, m), 3.3 (1H, d), 3.1 (1H, dd), 2.9 (1H, dd), 2.5 (1H, q), 2.4 (3H, d), 1.9 (3H, s), 1.6 (6H, m), 1.2 (6H, m).
Ex.100 (CDDl₃) δ 7.7 (1H, d), 7.4-7.1 (7H, m,), 4.6 (2H, m), 4.4 (1H, t), 3.5 (1H, d), 3.1 (1H, dd), 2.9 (6H, 2 x s), 2.7 (1H, dd), 2.5 (1H, dd), 1.9 (3H, s), 1.6 (6H, m), 1.2 (6H, m).
Ex.101 (CDCl₃) δ 7.4 (2H, m), 7.3-7.1 (6H, m), 5.9 (1H, m), 5.7 (1H, m), 4.5 (2H, m), 3.2 (2H, d), 3.0 (2H, m), 2.6 (2H, m), 1.9 (3H, s), 1.6 (6H, m), 1.2 (6H, m), 0.9 (3H, t).
Ex.102 (CDCl₃) δ 7.5-7.1 (8H, m), 5.9 (1H, t), 5.3 (1H, d), 4.5 (2H, d), 3.7 (2H, m), 3.1 (2H, s), 2.8 (1H, m), 2.6 (1H, m), 1.9 (3H, s), 1.6 (6H, m), 1.2 (6H, m), 0.8 (6H, d).
Ex.103 (d⁶-DMSO) δ 7.4-7.0 (8H, m), 6.7 (1H, m), 6.6 (2H, m), 4.5 (2H, d), 3.0 (1H, d), 2.9 (1H, d), 2.6 (1H, m), 2.5 (1H, m), 1.9 (3H, s), 1.6 (6H, m), 1.3 (6H, m).
Ex.104 (CDCl₃) δ 7.7 (1H, m), 7.6 (1H, m), 7.4 (1H, m), 7.4-7.2 (10H, m), 6.0 (1H, t), 5.3 (1H, t), 5.1 (2H, s), 4.5 (1H, d), 3.4 (1H, m), 3.3 (2H, dt), 3.1 (1H, dd), 2.8 (1H, dd), 2.7 (1H, dd), 2.4 (2H, t), 1.9 (3H, s), 1.6 (6H, m), 1.3 (6H, s).
Ex.105 (CDCl₃) δ 7.8 (1H, m), 7.7 (1H, m), 7.4 (1H, m), 7.4-7.2 (10H, m), 6.3 (1H, t), 5.2 (1H, t), 5.1 (2H, s), 4.6 (1H, d), 3.4 (1H, m), 3.3 (2H, dd), 3.2 (1H, d), 2.8 (1H, dd), 2.7 (1H, dd), 2.4 (2H, t), 1.9 (3H, s), 1.6 (6H, m), 1.3 (6H, m).
Ex.106 (CDCl₃) δ 7.5-7.1 (8H, m), 6.3-6.0 (1H, 2 x d), 5.6 and 5.3 (1H, 2 x t), 4.6 (2H, m), 4.3 (1H, m), 3.7 (3H, m), 3.3 (2H, m), 2.9 (1H, m), 2.7 (1H, m), 1.2 (3H, dd), 0.8 (9H, d).
Ex.107 (d⁶-DMSO) δ 8.0-6.9 (9H, m), 4.5-3.9 (3H, m), 3.5-2.6 (6H, m), 2.2-1.6 (4H, m), 0.8 (9H, d).
Ex.108 (CDCl₃) δ 7.4 (4H, m), 7.0 (4H, m), 5.7 (2H, d), 5.2 (1H, d), 3.0 (2H, d), 2.0 (3H, s), 1.7 (6H, q), 1.5 (6H, s).
Ex.109 (CDCl₃) δ 7.4-7.1 (8H, m), 5.0 (1H, t), 4.6 (1H, d), 4.4 (1H, d), 2.8 (3H, m), 2.2 (1H, m), 1.9 (4H, m), 1.6 (6H, m), 1.3 (6H, s).
Ex.110 (CDCl₃) δ 7.4-7.0 (8H, m), 4.7 (1H, d), 4.4 (1H, t), 3.6 (2H, dd), 3.0 (1H, m), 2.2 (1H, m), 2.1 (1H, m), 2.0 (3H, s), 1.7 (6H, m), 1.5 (6H, q).
Ex.111 (CDCl₃) δ 7.5-7.1 (8H, m), 5.6 (1H, t), 4.6 (1H, s), 4.5 (1H, dd), 4.3 (1H, m), 3.7 (3H, 2 x s), 3.6 (1H, t), 3.4-3.1 (3H, m), 1.9 (3H, m), 1.7 (6H, q), 1.4 (6H, s), 1.2 (3H, dd).
Ex.112 (CDCl₃) δ 7.5-7.1 (8H, m), 5.6 (1H, t), 4.6 (1H, s), 4.5 (1H, dd), 4.3 (1H, m), 3.8 (3H, 2 x s), 3.6 (1H, t), 3.4-3.1 (3H, m), 1.9 (3H, m), 1.7 (6H, q), 1.4 (6H, s), 1.2 (3H, dd)
Ex.113 (d⁶-DMSO) δ 7.4 (5H, m), 7.3 (4H, m), 7.1 (4H, m), 5.1 (2H, q), 3.1 (2H, m), 2.8 (2H, q), 2.4 (2H, m), 1.8 (3H, s), 1.6 (6H, m), 1.2 (6H, s).
Ex.114 (d⁶-DMSO) δ 7.7-6.8 (10H, m), 4.6 (1H, s), 4.1 (1H, m), 3.6 (4H, m), 3.4 (1H, m), 2.7 (1H, q), 2.3 (1H, m), 1.8 (3H, s), 1.6 (6H, m), 1.3 (6H, s), 1.2 (3H, d).
Ex.115 (d⁶-DMSO) δ 8.1-6.8 (10H, m), 4.6 (1H, s), 4.0 (1H, m), 3.6 (4H, m), 3.4 (1H, m), 2.7 (1H, q), 2.3 (1H, m), 1.8 (3H, s), 1.6 (6H, m), 1.3 (6H, s), 1.2 (3H, m).
Ex.116 (d⁶-DMSO) δ 8.0-6.8 (10H, m), 4.5 (1H, s), 4.0 (1H, m), 3.6 (4H, m), 3.4 (1H, m), 2.6 (1H, q), 2.3 (1H, m), 1.9 (3H, s), 1.6 (6H, m), 1.4 (6H, s), 1.0 (3H, d).
Ex.117 (CDCl₃) δ 8.6 and 8.2 (1H, 2 x s), 7.5-7.1 (13H, m), 6.3 and 6.0 (1H, 2 x d), 5.4 and 5.2 (1H, 2 x t), 4.8-4.4 (3H, m), 3.6 (3H, s), 3.3-2.3 (6H, m), 0.8 (9H, 2 x s).
Ex.118 (CDCl₃) δ 8.2-6.4 (15H, m), 5.6 and 5.4 (1H, 2 x t), 4.8-4.2 (3H, m), 3.3-2.5 (6H, m), 0.8 (9H, 2 x s).
Ex.119 (CDCl₃) δ 7.6-7.0 (10H, m), 6.4 (1H, m) 5.0 (1H, m), 4.6-4.412H, m), 4.3-4.0 (2H, m), 3.9-3.2 (3H, m), 2.9-2.6 (2H, m), 2.3 (1H, m), 2.1-1.9 (7H, m), 1.6 (6H, m), 1.4 and 1.2 (6H, 2 x s).
Ex.120 (CDCl₃) δ 7.6-7.0 (10H, m), 6.1 (1H, m), 5.0-4.4 (3H, m), 4.2 (2H, m), 3.9-3.2 (3H, m), 2.9-2.6 (2H, m), 2.1-1.9 (8H, m), 1.6 (6H, m), 1.2 (6H, s).
Ex.121 (CDCl₃) δ 7.6-7.0 (10H, m), 6.1 (1H, s), 4.8-4.4 (3H, m), 4.2-3.3 (5H, m), 2.9 (2H, m), 2.3 (1H, m), 2.1-1.9 (7H, m), 1.6 (6H, m), 1.2 (6H, s).
Ex.122 (CCCl₃) δ 7.6-7.1 (10H, m), 6.0 (1H, m), 4.8-4.3 (3H, m), 3.8-3.2 (7H, m), 2.9 (1H, m), 2.6 (2H, m), 2.1-1.9 (7H, m), 1.6 (6H, m), 1.2 (6H, s).
Ex.123 (CDCl₃) δ 7.6-7.1 (9H, m), 6.0 (1H, m), 4.6-4.0 (5H, m), 3.9-3.2 (6H, m), 2.9-2.3 (3H, m), 2.1-1.9 (7H, m), 1.6 (6H, m), 1.4 and 1.2 (6H, 2 x s).
Ex.124 (CDCl₃) δ 7.6-7.1 (9H, m), 5.9 (1H, m), 4.6-4.2 (3H, m), 3.8-3.1 (8H, m), 3.0-2.2 (5H, m), 2.1-1.9 (7H, m), 1.6 (6H, m), 1.4 and 1.2 (6H, 2 x s).
Ex.125 (d⁶-DMSO) δ 7.7-7.5 (2H, m), 7.4-6.9 (9H, m), 4.5 (2H, m), 4.0 (1H, m), 3.8-3.2 (4H, m), 2.8-2.6 (2H, m), 2.3 (2H, m), 1.9 (3H, m), 1.6 (6H, m), 1.3 (6H, 2 x s), 1.1 (3H, 2 x d).
Ex.126 (CDCl₃) δ 7.4-6.9 (9H, m), 5.9 (1H, m), 5.6 (1H, m), 4.5 (2H, m), 4.2 (1H, m), 3.7 (3H, s), 3.4 (4H, m), 2.8 (2H, m), 2.5 (2H, m), 2.0 (3H, s), 1.7 (6H, m), 1.3 (6H, 2 x s), 1.1 (3H, 2 x d).
Ex.127 (CDCl₃) δ 7.5-7.1 (8H, m), 6.8 (1H, m),6.1 and 5.9 (2H, m), 4.5 (2H, m), 4.3 (1H, m), 3.2 (2H, s), 2.9-2.5 (5H, m), 2.0 (3H, s), 1.6 (6H, m), 1.3 (9H, m).
Ex.128b (CDCl₃) δ 7.5-7.1 (8H, m), 6.0 and 5.4 and 5.3 and 5.2 (2H, 4 x d), 4.6 (2H, m), 4.3 (1H, m), 3.7 (3H, 2 x s), 3.5 (1H, m), 3.2 (2H, m), 2.0 (3H, s), 1.6-0.9 (15H, m), 0.8 (3H, 2 x d).
Ex.129 (CDCl₃) δ 7.5-7.1 (8H, m), 6.2 and 6.0 and 5.3 and 5.2 (2H, 4 x d), 4.6 (2H, m), 4.4 (1H, m), 3.7 (3H, 2 x s), 3.5 (1H, m), 3.2 (2H, m), 2.0 (3H, m), 1.8-0.9 (15H, m), 0.8 (3H, 2 x d).
Ex.130 (CDCl₃) δ 7.5-7.1 (8H, m), 6.2 and 6.0 (1H, 2 x d), 5.4 and 5.2 (1H, 2 x t), 4.6 (2H, m), 4.3 (1H, m), 3.3-3.1 (2H, m), 2.9-2.3 (4H, m), 2.0 (3H, s), 1.6 (6H, m),1.3 (6H, 2 x s), 1.1 (6H, m).
Ex.131b (d⁶-DMSO) δ 7.6-6.9 (9H, m), 4.6 (1H, s), 4.3 (1H, s), 3.6 (3H, s), 3.5 (1H, m), 2.9-2.5 (4H, m), 2.0 (3H, s), 1.6 (6H, m),1.3 (6H, s).
Ex.132 (d⁶-DMSO) δ 7.9 and 7.5 (1H, 2 x d), 7.4-6.9 (9H, m), 4.4 (2H, m), 4.2-4.0 (1H, m), 3.6 (3H, 2 x s), 3.0 (4H, m), 2.0 (3H, s), 1.6 (6H, m),1.3 (6H, 2 x s), 1.2 (3H, m).
Ex.133 (CDCl₃) δ 7.6-7.1 (9H, m), 5.3 (2H, m), 4.6-4.2 (3H, m), 3.6-3.1 (4H, m), 2.7 (1H, m), 2.1-1.2 (18H, m).
Ex.134d (d⁶-DMSO) δ 13.5-12.5 (1H, br s), 8.3 (1H, t), 7.4-7.0 (8H, m), 5.7 (1H, s), 5.4 (1H, s), 2.8 (2H, m), 1.9 (3H, s), 1.6 (6H, m), 1.4 (6H, m).
Ex.135 (d⁶-DMSO) δ 8.8 (1H, d), 8.1 (1H, t), 7.5-7.0 (8H, m), 5.59 (1H, s), 5.56 (1H, s), 4.4 (1H, m), 3.6 (3H, s), 2.9 (2H, d), 1.9 (3H, s), 1.6 (6H, m), 1.4 (6H, m), 1.3 (3H, d).
Ex.136 (d⁶-DMSO) δ 12.6 (1H, br s),8.4 (1H, t), 8.1 (1H, t), 7.5-6.9 (8H, m), 5.8 (1H, s), 5.4 (1H, s), 4.4 (1H, m), 3.9-3.5 (2H, m), 3.3-2.5 (4H, m), 2.1-1.4 (13H, m), 1.3 (6H, m).
Ex.137d (CDCl₃) δ 7.8 (4H, m), 7.3 (4H, m), 6.1 and 5.5 (1H, br s), 4.6 and 4.33 (1H, dd), 3.9-2.3 (7H, m), 2.0 (6H, m), 1.7 (6H, m), 1.4 (6H, m).
Ex.138c (CDCl₃) δ 7.6-7.0 (8H, m), 5.9 and 5.0 (1H, 2xd), 4.6-4.3 (5H, m), 3.8-3.1 (4H, m), 2.4-1.9 (2H, m), 1.8-1.0 (15H, m).
Ex.139 (CDCl₃) δ 7.7-7.0 (8H, m), 4.6-4.3 (3H, m), 3.8-2.8 (7H, m), 2.3 (2H, m), 1.9-1.0 (19H, m).
Ex.140 (CDCl₃) δ 7.7-7.0 (8H, m), 4.6-4.0 (4H, m), 3.8-2.2 (8H, m), 1.9 (4H, m), 1.6 (9H, m), 1.0 (6H, m).
Ex.141 (CDCl₃) δ 7.6-7.0 (8H, m), 5.0-4.5 (3H, m), 4.3 and 4.2 (1H, 2xm), 3.6-3.0 (5H, m), 2.7 (1H, m), 2.4 (1H, m), 2.2 (1H, m), 1.8 (7H, m), 1.6 (6H, m), 1.2 (6H, m).
Ex.142 (CDCl₃) δ 7.6 (1H, m), 7.2 (7H, m), 5.0-4.6 (3H, m), 4.3 and 4.1 (1H, 2xm), 3.6-3.0 (5H, m), 2.9 (1H, m), 2.7 (1H, m), 2.4 (1H, m), 2.2 (1H, m), 1.9 (6H, m), 1.6 (6H, m), 1.2 (6H, m).
Ex.143 (CDCl₃) δ 7.6 (1H, m), 7.2 (7H, m), 4.6 (3H, m), 4.4 and 4.2 (1H, 2m), 3.65 and 3.6 (3H, 2s), 3.5-3.0 (5H, m), 2.7 (1H, m), 2.3 (1H, m), 2.1-1.4 (14H, m), 1.2 (6H, m).
Ex.144 (CDCl₃) δ 7.6-7.1 (8H, m), 4.8-4.5 (3H, m), 4.4 and 4.2 (1H, 2m), 3.6 (3H, m), 3.5-2.0 (7H, m), 2.0-1.4 (14H, m), 1.2 (6H, m).
Ex.145 (CDCl₃) δ 7.6-6.9 (8H, m), 5.5 (1H, m), 4.7 (1H, t), 4.5 (2H, m), 4.2 (1H, m), 3.7-3.2 (5H, m), 2.9 (1H, m), 2.7 (1H, m), 2.2-1.7 (7H, m), 1.5 (9H, m), 1.2 (6H, m).
Ex.146 (CDCl₃) δ 7.6-7.0 (8H, m), 6.3 (1H, m), 4.5 (2H, d), 4.3 (2H, m), 3.8-3.2 (5H, m), 2.9 (1H, m), 2.6 (1H, m), 2.0 (7H, m), 1.7 (6H, m), 1.4 (9H, m).
Ex.147 (CDCl₃) δ 7.6-7.0 (8H, m), 6.1 (1H, m), 4.8-4.2 (4H, m), 3.6-3.2 (5H, m), 2.9 (1H, m), 2.3 (1H, m), 2.2-1.8 (7H, m), 1.6 (9H, m), 1.2 (6H, m).
Ex.148 (CDCl₃) δ 7.8-7.0 (8H, m), 4.8-4.2 (5H, m), 3.7-3.3 (3H, m), 3.2 (1H, m), 2.7 (1H, d), 2.6 (1H, m), 2.4 (1H, m), 2.2-1.2 (13H, m), 1.1 (6H, m), 0.8 (3H, d).
Ex.149 (d⁶-DMSO) δ 12.5 (1H, br s), 7.4-6.8 (9H, m), 5.1-4.1 (5H, m), 3.9 (1H, m), 3.7 (1H, m), 3.3-2.9 (4H, m), 2.5 (2H, m), 1.9 (3H, s), 1.6 (6H, m), 1.3 (6H, m).
Ex.150 (d⁶-DMSO) δ 7.3 (3H,m), 7.1 (5H, m), 6.8-6.3 (1H, m), 4.5 (2H, s), 3.7-2.7 (7H, m), 2.5 (2H, m), 1.9 (5H, m), 1.6 (6H, m), 1.2 (6H, s).
Ex.151 (CDCl₃) δ 7.7-7.1 (8H, m), 4.6 (2H, br,s), 4.5 (1H, m), 3.7 (3H, m), 3.65-2.0 (9H, m), 1.9 (3H, br,s), 1.6 (7H, m), 1.3 (1H, m), 1.1 (6H, s).
Ex.152 (CDCl₃) δ 7.6 (1H, d), 7.2 (7H, m), 4.8-4.2 (3H, m), 4.1 (2H, m), 3.9-2.0 (9H, m), 1.9 (3H, s), 1.6 (10H, m), 1.2 (3H, m), 1.1 (6H, s).
Ex.153 (CDCl₃) δ 7.6 (1H, d), 7.2 (7H, m), 4.8-4.2 (3H, m), 4.1 (2H, m), 3.9-2.0 (9H, m), 1.9 (3H, s), 1.6 (10H, m), 1.2 (3H, m), 1.1 (6H, s).
Ex.154b (CDCl₃) δ 7.7 (1H, m), 7.2 (7H, m), 4.8-4.2 (3H, m), 3.8 (1H, m), 3.62 and 3.64 (3H, 2xs), 3.6-2.0 (8H, m), 1.9 (3H, s), 1.6 (8H, m), 1.2 (2H, m), 1.1 (6H, s).
Ex.155 (CDCl₃) δ 7.7 (1H, m), 7.2 (7H, m), 4.6-4.2 (3H, m), 3.8 (1H, m), 3.63 and 3.65 (3H, 2xs), 3.6-2.0 (8H, m), 1.9 (3H, s), 1.6 (10H, m), 1.1 (6H, s).
Ex.156 (CDCl₃) δ 7.7 (1H, m), 7.2 (7H, m), 4.8-4.2 (3H, m), 3.8 (1H, m), 3.63 and 3.65 (3H, 2xs), 3.6-2.0 (8H, m), 1.9 (3H, s), 1.6 (8H, m), 1.3 (2H, m), 1.1 (6H, s).
Ex.157 (CDCl₃) δ 7.6 (1H, d), 7.2 (7H, m), 4.8-4.3 (3H, m), 3.8 (1H, m), 3.63 and 3.65 (3H, 2xs), 3.6-2.0 (8H, m), 1.9 (3H, s), 1.6 (10H, m), 1.1 (6H, s).
Ex.158 (d⁶-DMSO) δ 8.2 (1H, s), 7.3 (3H, m), 7.0 (5H, m), 6.7 (1H, t), 4.47 and 4.48 (2H, 2xs), 3.9 (1H, m), 3.5-2.9 (4H, m), 2.6 (1H, m), 2.4 (1H, m), 1.8 (4H, m), 1.6 (9H, m), 1.3 (8H, m), 1.0 (2H,s).
Ex.159 (d⁶-DMSO) δ 8.3 (1H, t), 7.5 (1H, s), 7.3 (3H, m), 7.0 (5H, m), 4.50 and 4.52 (2H, 2xs), 4.1 (1H, t), 3.4 (2H, m), 3.1 (1H, m), 2.9 (1H, d), 2.7 (2H, m), 1.9 (4H, m), 1.6 (9H, m), 1.3 (6H, m), 1.2(1H, m), 0.9 (1H, m), 0.7 (2H,m).
Ex.160d (CDCl₃) δ 7.7 (1H, d), 7.4-7.1 (6H, m), 6.9 (1H, m), 5.0 (1H, d), 4.6 (1H, s), 4.3 (1H, m), 3.5 (3H, m), 3.3 (1H, m), 2.9 (1H, m), 2.5-1.4 (14H, m), 1.2 (6H, m).
Ex.161 (CDCl₃) δ 7.6 (1H, d), 7.4-7.0 (6H, m), 6.9 (1H, m), 5.0 (1H, s), 4.6 (2H, m), 4.3 (1H, m), 3.5 (3H, m), 3.2 (2H, m), 2.8-1.4 (13H, m), 1.2 (6H, m).
Ex.162 (CDCl₃) δ 7.7 (1H, d), 7.4-7.1 (6H, m), 6.9 (1H, m), 5.0 (1H, m), 4.6 (1H, m), 4.3 (1H, m), 3.5 (3H, m), 3.3 (1H, m), 2.9 (1H, m), 2.5-1.4 (14H, m), 1.2 (6H, m).
Ex.163 (d⁶-DMSO) δ 8.2 (1H, t), 7.5 (1H, s), 7.3 (3H, m), 7.0 (5H, m), 4.5 (2H, d), 4.0 (1H, m), 3.1-3.4 (2H, m), 3.0 (1H, m), 2.9 (1H, d), 2.8-2.6 (2H, m), 1.9-1.3 (19H, m), 1.08 and 1.13 (6H, 2xs).
Ex.164 (d⁶-DMSO) δ 7.9(1H, s), 7.3-6.8 (8H, m), 6.8 (1H, t), 4.5 (2H, s), 4.0 (1H, m), 3.1-3.4 (3H, m), 3.0 (1H, m), 2.85 (2H, m), 2.1 (1H, m), 2.0-1.0 (24H, m).
Ex.165 (CDCl₃) δ 7.7-6.9 (8H, m), 5.7 (1H, m), 5.4 (1H, m), 4.9-4.4 (2H, m), 3.8-2.4 (5H, m), 2.2-1.2 (19H, m).
Ex.166 (CDCl₃) δ 7.5 (1H, m), 7.4-7.1 (7H, m), 5.4 and 5.1 (1H, 2xbr,s), 4.5 (2H, m), 4.4 (2H, m), 4.2 (2H, m), 3.75 and 3.72 (3H, 2xs), 3.5 (2H, m), 2.8 (1H, m), 2.5 (1H, m), 1.9 (3H, br, s), 1.6 (6H, m), 1.2 (6H, m).
Ex.167 (d⁶-DMSO) δ 12.8 (1H, br s), 7.5 (1H, d), 7.4-6.9 (9H, m), 4.4 (2H, s), 4.1 (1H, m), 3.8 (2H, m), 3.4 and 3.0 (2H, 2xdd), 3.1 and 2.8 (2H, 2xdd), 2.5 (2H, m), 2.45 and 2.0 (2H, 2xdd), 1.9 (3H, s), 1.6 (6H, m), 1.3 (6H, s).
Ex.168 (d⁶-DMSO) δ 12.8 (1H, br s), 7.7 (1H, d), 7.4-6.9 (8H, m), 6.8 (1H, t), 4.4 (2H, s), 4.1 (1H, m), 3.9 (2H, m), 3.5 and 3.1 (2H, 2xdd), 2.9 (2H, 2xdd), 2.6 (2H, m), 2.4 and 1.9 (2H, 2xdd), 1.85 (3H, s), 1.6 (6H, m), 1.3 (6H, s).
Ex.169 (d⁶-DMSO) δ 7.5 (1H, d), 7.4-6.9 (9H, m), 4.4 (2H, s), 4.2-3.8 (3H, m), 3.6 (3H, s), 3.5-2.9 (4H, m), 2.8 (1H, d), 2.5 (2H, m), 2.0 (1H, m), 1.9 (3H, s), 1.5 (6H, m), 1.3 (6H, s).
Ex.170 (d⁶-DMSO) δ 7.6 (1H, d), 7.4-6.9 (8H, m), 6.8 (1H, t), 4.4 (2H, s), 4.0 (3H, m), 3.6 (3H, s), 3.6-1.95 (8H, m), 1.9 (3H, s), 1.6 (6H, m), 1.3 (6H, s).
Ex.171 (d⁶-DMSO) δ 8.1 (1H, m), 7.9 and 7.5 (1H, 2xm), 7.4-6.9 (14H, m), 4.6-4.2 (3H, m), 3.8-2.4 (8H, m), 1.9 (3H, br,s), 1.6 (6H, m), 1.3 (6H, m).
Ex.172 (d⁶-DMSO) δ 8.0-6.8 (16H, m), 4.5-4.0 (4H, m), 3.4-2.4 (6H, m), 1.8 (3H, s), 1.6 (6H, m), 1.2 (6H, m), 1.1 (3H, d).
Ex.173 (d⁶-DMSO) δ 8.0-6.9 (16H, m), 4.5-4.0 (3H, m), 3.3-2.5 (8H, m), 1.8 (3H, m), 1.6 (6H, m), 1.2 (6H, m).
Ex.174 (d⁶-DMSO) δ 10.85 and 10.8 (1H, 2xs), 8.1 (1H, m), 7.7-6.7 (15H, m), 4.5-4.1 (3H, m), 3.8-2.3 (8H, m), 1.9 (3H, m), 1.6 (6H, m), 1.2 (6H, m).
Ex.175 (CDCl₃) δ 7.6-7.0 (8H, m), 4.8 (2H, m), 4.6 (2H, m), 4.3 (1H, m), 3.6-2.2 (9H, m), 2.1-1.4 (13H, m), 1.2 (6H, s).
Ex.176 (CDCl₃) δ 7.6-6.7 (8H, m), 4.8-4.0 (5H, m), 3.3-2.0 (9H, m), 1.8-0.8 (19H, m).
Ex.177 (CDCl₃) δ 7.1 (1H, m), 7.0-6.6 (8H, m), 5.6 (1H, br,s), 4.3 (1H, s), 4.1 (1H, s), 3.3 (1H, m), 2.9 (1H, d), 2.7 (1H, d), 0.9 (16H, m), 0.6 (6H, t).
Ex.178 (d⁶-DMSO) δ 12.8 (1H, br s), 7.7 (1H, m), 7.4-6.9 (8H, m), 6.7 and 6.6 (1H, 2xm), 4.4 (2H, m), 4.3-3.8 (3H, m), 3.4-2.8 (4H, m), 2.5 (2H, m), 2.1 (1H, m), 1.9 (3H, s), 1.6 (7H, m), 1.3 (6H, m).
Ex.179 (d⁶-DMSO) δ 12.8 (1H, br s), 7.7 (1H, m), 7.5-7.0 (8H, m), 6.7 and 6.6 (1H, 2xm), 4.4 (2H, m), 4.3-3.5 (3H, m), 3.4-2.9 (4H, m), 2.5 (2H, m), 2.1 (1H, m), 1.9 (3H, s), 1.6 (7H, m), 1.3 (6H, m).
Ex.180 (d⁶-DMSO) δ 8.3-6.9 (10H, m), 4.5 (2H, m), 4.0-2.5 (8H, m), 1.9 (4H, m), 1.7 (9H, m), 1.4-1.2 (9H, m).
Ex.181 (d⁶-DMSO) δ 8.3 and 8.25 (1H, 2t), 7.7-6.9 (10H, m), 6.6 and 6.5 (1H, 2s), 4.5 (2H, m), 4.1 and 3.8 (1H, 2m), 3.6-2.4 (8H, m), 2.0-1.5 (13H, m), 1.4 and 1.3 (6H, m).
Ex.182 (d⁶-DMSO) δ 8.1-6.8 (18H, m), 4.5-4.1 (3H, m), 3.6-2.4 (8H, m), 1.9 (3H, sm), 1.6 (6H, m), 1.3 (6H, m).
Ex.183 (d⁶-DMSO) δ 12.0 (1H, br s), 7.9 (1H, m), 7.4 (4H, m), 7.1-6.8 (5H, m), 4.6-4.3 (3H, m), 3.9 (1H, m), 3.7 (2H, m), 3.5-2.9 (5H, m), 2.6 (2H, m), 1.9-1.5 (11H, m), 1.3 (6H, m).
Ex.184b (d⁶-DMSO) δ 12.6 (1H, br s), 8.1 and 6.3 (1H, 2xt), 7.4-6.9 (9H, m), 4.5 (2H, m), 4.2-3.9 (2H, m), 3.5-2.8 (6H, m), 2.6 (2H, m), 2.1-1.5 (13H, m), 1.4-1.1 (9H, m).
Ex.185 (d⁶-DMSO) δ 12.6 (1H, br s), 8.0 and 6.3 (1H, 2xm), 7.4-6.9 (9H, m), 4.5 (2H, m), 4.2-3.9 (2H, m), 3.5-2.8 (6H, m), 2.6 (2H, m), 2.1-1.5 (13H, m), 1.4-1.1 (9H, m).
Ex.186b (d⁶-DMSO) δ 12.5 (1H, br s), 8.2 and 8.0 (1H, 2xd), 7.4-6.9 (9H, m), 4.5 (2H, m), 4.1 (2H, m), 3.5-2.3 (8H, m), 2.2-1.5 (13H, m), 1.4-1.1 (9H, m).
Ex.187 (d⁶-DMSO) δ 12.4 (1H, br s), 8.1 (1H, d), 7.3 (3H, m), 7.1 (5H, m), 4.5 (2H, d), 4.1 (1H, m), 3.9 (1H, m), 3.3-2.9 (6H, m), 2.5 (2H, m), 2.1-1.4 (13H, m), 1.2 (9H, m).
Ex.188 (CDCl₃) δ 9.1 and 6.0 (1H, 2xt), 7.6-7.0 (8H, m), 4.6-4.3 (3H, m), 4.1 (1H, m), 3.8 and 3.3 (3H, 2xs), 3.6-3.1 (6H, m), 2.9 (1H, m), 2.7-2.2 (2H, m), 2.0-1.6 (12H, m), 1.4 and 1.2 (6H, 2xs).
Ex.189 (CDCl₃) δ 7.6-7.0 (8H, m), 5.9 (1H, m), 4.5 (3H, m), 4.1 (1H, m), 3.7 (3H, s), 3.6-3.4 (4H, m), 3.3 (2H, s), 2.8 (1H, m), 2.6 (1H, m), 2.3 (1H, m), 2.0-1.6 (12H, m), 1.4 (6H, s).
Ex.190 (d⁶-DMSO) δ 7.4-6.9 (8H, m), 6.8 (1H, t), 4.8-4.1 (3H, m), 3.6-2.6 (9H, m), 2.5 (2H, m), 1.9-1.4 (13H, m), 1.2 (6H, m).
Ex.191 (d⁶-DMSO) δ 7.8 (1H, t), 7.5-6.9 (8H, m), 6.8 and 6.7 (1H, 2xt), 4.8-3.8 (5H, m), 3.6 (3H, m), 3.4-2.9 (4H, m), 2.5 (2H, m), 2.1 (1H, m), 1.9 (3H, br s), 1.7-1.2 (13H, m).
Ex.192 (CDCl₃) δ 7.6-7.1 (8H, m), 5.1 and 4.7 (1H 2xm), 4.5 and 4.3 (1H, 2xd), 3.5 (2H, m), 3.4-2.8 (4H, m), 2.4-1.1 (25H, m).
Ex.193 (CDCl₃) δ 7.5 (1H, d), 7.4-7.1 (7H, m), 4.6 (1H, m), 4.2 (1H, m), 3.5 (2H, m), 3.2 (2H, m), 2.9 (1H, m), 2.3 (1H, m), 2.2-1.8 (13H, m), 1.6 (6H, m), 1.2 (6H, m).
Ex.194 (d⁶-DMSO) δ 12.5 (1H, br s), 8.1 (1H, t), 7.3 (3H, m), 7.0 (5H, m), 6.3 (1H, t), 4.5 (2H, m), 3.9 (1H, m), 3.7 (2H, m), 3.4-2.9 (4H, m), 2.7-2.3 (4H, m), 2.1 (1H, m), 1.8 (3H, s), 1.6 (3H, m), 1.3 (6H, s).
Ex.195 (CDCl₃) δ 7.7-7.1 (8H, m), 6.9 and 6.0 (1H 2xt), 4.6(3H, m), 4.4-4.0 (3H, m), 3.8 (1H, m), 3.5 (4H, m), 3.2 (2H, m), 2.8 (1H, m), 2.3 (2H, m), 1.9 (6H, m), 1.8-1.2 (22H, m).
Ex.196 (CDCl₃) δ 7.6 (1H, d), 7.5-7.1 (7H, m), 6.9 (1H, d), 5.8 and 5.2 (2H, dd), 4.5(3H, m), 4.3 (1H, m), 3.5 (4H, m), 1.2 (1H, d), 2.9 (1H, m), 2.5-2.1 (2H, m), 2.0-1.1 (30H, m).
Ex.197 (d⁶-DMSO) δ 8.2 and 6.7 (1H,2xm), 7.4-6.8 (10H, m), 4.5 (2H, m), 4.1 and 3.8 (1H, 2xm), 3.6-2.5 (6H, m), 1.9 (3H, m), 1.8-1.5 (10H, m), 1.4 and 1.2 (6H, 2xs).
Ex.198b (d⁶-DMSO) δ 12.6 (1H, br s), 8.0 (1H, t), 7.4 (3H, m), 7.0 (6H, m), 4.5 (2H, d), 4.1 (1H, m), 3.6 (2H, m), 3.4-2.8 (4H, m), 2.6-2.2 (4H, m), 1.9 (3H, s), 1.6 (10H, m), 1.35 (6H, s).
Ex.199 (d⁶-DMSO) δ 12.6 (1H, br s), 8.1 (1H, t), 7.3 (3H, m), 7.0 (5H, m), 6.4 (1H, t), 4.5 (2H, m), 3.9 (1H, m), 3.6 (2H, m), 3.4-2.9 (4H, m), 2.6-2.4 (4H, m), 2.1 (1H, m), 1.8 (2H, s), 1.6 (9H, m), 1.2 (6H, s).
Ex.200 (d⁶-DMSO) δ 12.6 (1H, br s), 8.0 (1H, d), 7.3 (3H, m), 7.0 (6H, m), 4.5 (2H, m), 4.0 (2H, m), 3.4-2.8 (4H, m), 2.6-2.1 (5H, m), 1.9 (3H, s), 1.6 (9H, m), 1.2 (9H, m).
Ex.201 (d⁶-DMSO) δ 12.6 (1H, br s), 8.0 (1H, d), 7.3 (3H, m), 7.0 (5H, m), 6.3 (1H, t), 4.5 (2H, d), 4.1 (1H, m), 3.9 (1H, m), 3.4-2.9 (4H, m), 2.6-2.4 (4H, m), 2.0 (1H, m), 1.9 (3H, s), 1.6 (9H, m), 1.2 (9H, m).
Ex.202 (d⁶-DMSO) δ 12.6 (1H, br s), 8.0 (1H, d), 7.3 (3H, m), 7.0 (6H, m), 4.5 (2H, d), 4.1 (2H, m), 3.5-2.8 (4H, m), 2.7-2.2 (5H, m), 1.9 (3H, s), 1.6 (9H, m), 1.4-1.1 (9H, m).
Ex.203 (d⁶-DMSO) δ 12.6 (1H, br s), 8.1 (1H, d), 7.3 (3H, m), 7.0 (5H, m), 6.4 (1H, t), 4.5 (2H, d), 4.1 (1H, m), 3.9 (1H, m), 3.4-2.9 (4H, m), 2.7-2.3 (4H, m), 2.0 (1H, m), 1.8 (3H, s), 1.6 (9H, m), 1.2 (9H, m).
Ex.204 (CDCl₃) δ 7.6-7.1 (8H, m), 5.1-4.5 (3H, m), 4.0 (1H, m), 3.6-2.2 (10H, m), 2.0-1.4 (13H, m), 1.2 (6H, m).
Ex.205 (CDCl₃) δ 7.6 (1H, m), 7.4-7.0 (7H, m), 6.8 (1H, m), 5.8 (1H, dd), 4.7-4.4 (4H, m), 3.8 and 3.75 (3H, 2xs), 3.6-2.2 (6H, m), 2.0-1.4 (13H, m), 1.2 (6H, m).
Ex.206 (CDCl₃) δ 7.7-7.1 (8H, m), 4.5 (3H, m), 3.9 (1H, m), 3.7 and 3.6 (3H, 2xs), 3.5-2.1 (10H, m), 2.0-1.5 (13H, m), 1.2 (6H, m).
Ex.207 (d⁶-DMSO) δ 8.0 (1H, t), 7.5 (1H, d), 7.4-6.8 (16H, m), 4.4 (2H, s), 4.1 (1H, m), 3.6-3.1 (3H, m), 2.9 (3H, m), 2.5 (2H, m), 1.9 (3H, s), 1.6 (6H, m), 1.3 (6H, s).
Ex.208 (d⁶-DMSO) δ 8.0 (1H, t), 7.8 (1H, d), 7.5 (1H, t), 7.4-6.9 (15H, m), 4.5 (1H, s), 4.3 (1H, m), 4.2 (1H, s), 3.6-3.0 (4H, m), 2.7 (2H, m), 2.5 (2H, m), 1.9 (3H, s), 1.6 (6H, m), 1.3 (6H, m).
Ex.209 (CDCl₃) δ 7.4-7.0 (8H, m), 5.6 (1H, t), 4.6 (1H, s), 4.4 (1H, s), 2.9 (3H, m), 2.3 (2H, dd), 2.0 (3H, s), 1.6 (6H, m), 1.3 (1H, d), 1.2 (6H, s).
Ex.210 (CDCl₃) δ 7.2 (8H, m), 7.1 and 6.2 (1H, 2xd), 5.7 and 5.6 (1H, 2xt), 4.7-4.2 (3H, m), 3.7 (3H, s), 3.0-2.2 (5H, m), 2.0 (3H, s), 1.7 (7H, m), 1.43 and 1.40 (6H, 2xs), 1.3 and 1.1 (3H, 2xd).
Ex.211 (CDCl₃) δ 7.5-7.0 (8H, m), 5.8 and 5.6 (1H, 2xt), 4.8-4.3 (3H, m), 3.9-3.2 (3H, m), 3.0 (3H, m), 2.6-1.0 (21H, m).
Ex.212 (CDCl₃) δ 7.5 (1H, d), 7.4-7.1 (7H, m), 6.2 (1H, d), 5.8 (1H, t), 4.5 (2H, d), 3.8 (1H, m), 3.2 (2H, dd), 2.8 (1H, m), 2.7 (1H, m), 2.4 (1H, m), 2.0 (3H, s), 1.8-1.1 (18H, m).
Ex.213 (CDCl₃) δ 7.5-7.1 (8H, m), 6.3 (1H, d), 5.7 (1H, t), 4.5 (2H, d), 3.8 (1H, m), 3.2 (2H, dd), 2.8 (1H, m), 2.7 (1H, m), 2.5 (1H, m), 2.0 (3H, s), 1.8-1.1 (18H, m).
Ex.214 (CDCl₃) δ 7.6-6.8 (9H, m,), 6.0 (1H, m), 4.8-4.2 (4H, m), 3.8 (3H, s), 3.6-2.1 (7H, m), 2.0-1.1 (21H, m).
Ex.215 (CDCl₃) δ 7.6-7.1 (8H, m), 5.1-4.5 (3H, m), 4.0 (1H, m), 3.6-2.2 (10H, m), 2.0-1.4 (13H, m), 1.2 (6H, m).
Ex.216 (d⁶-DMSO) δ 8.0-7.0 (15H, m), 6.8-6.5 (2H, m), 4.5-4.0 (3H, m), 3.4-3.0 (4H, m), 2.5 (2H, m), 1.82 and 1.78 (3H, 2xs), 1.6 (6H, m), 1.1 and 1.2 (6H, 2xs).
Ex.217 (d⁶-DMSO) δ 8.2-6.8 (16H, m), 6.5 (1H, t), 4.5-4.1 (3H, m), 3.4-2.2 (6H, m), 1.8 and 1.7 (3H, 2xs), 1.6 (6H, m), 1.1 and 1.2 (6H, 2xs).
Ex.218 (d⁶-DMSO) δ 8.0 (1H, t), 7.7 (1H, d), 7.4-6.7 (14H, m), 4.5-4.0 (4H, m), 3.6-2.3 (6H, m), 1.8 (3H, s), 1.6 (6H, m), 1.3-1.0 (9H, m).
Ex.219 (d⁶-DMSO) δ 12.6 (1H, br s), 7.4-6.9 (13H, m), 5.0-4.0 (4H, m), 3.6-3.0 (3H, m), 2.8-2.1 (4H, m), 1.7 (3H, brs), 1.6 (6H, m), 1.1 and 1.08 (6H, 2xs).
Ex.220c (CDCl₃) δ 7.6-7.0 (8H, m), 4.8-4.3 (3H, m), 3.8-2.3 (9H, m), 2.2-1.4 (19H, m).
Ex.221 (CDCl₃) δ 7.6-7.0 (8H, m), 6.4 (1H, m,), 4.6-4.4 (2H, m), 4.3-4.0 (1H, m), 3.9-3.1 (9H, m), 2.9-2.6 (2h, m), 2.3 (1H, m), 2.1-1.9 (6H, m), 1.6 (6H, m), 1.4 and 1.2 (6H, 2 x s).
Ex.222 (CDCl₃) δ 7.6-6.9 (8H, m), 5.5 (1H, m), 4.5 (2H, m), 4.2 (1H, m), 3.7-3.1 (8H, m), 2.9 (1H, m), 2.7 (1H, m), 2.2-1.7 (7H, m), 1.5 (9H, m), 1.2 (6H, m).
Ex.223 (CDCl₃) δ 7.6-7.0 (8H, m), 6.1 (1H, m), 4.7-4.2 (3H, m), 3.6-3.1 (8H, m), 2.9 (1H, m), 2.3 (1H, m), 2.2-1.8 (7H, m), 1.6 (9H, m), 1.2 (6H, m).
Ex.224b (d⁶-DMSO) δ 12.6 (1H, br s), 7.4-6.9 (9H, m), 4.5 (2H, m), 4.2-3.9 (2H, m), 3.5-2.8 (9H, m), 2.6 (2H, m), 2.1-1.5 (13H, m), 1.4-1.1 (9H, m).
Ex.225 (d⁶-DMSO) δ 12.6 (1H, br s), 7.4-6.9 (9H, m), 4.5 (2H, m), 4.2-3.9 (2H, m), 3.5-2.8 (9H, m), 2.6 (2H, m), 2.1-1.5 (13H, m), 1.4-1.1 (9H, m).
Ex.226b (d⁶-DMSO) δ 12.6 (1H, br s), 8.0 (1H, d), 7.3 (3H, m), 7.0 (5H, m), 4.5 (2H, d), 4.1 (1H, m), 3.9 (1H, m), 3.4-2.9 (7H, m), 2.6-2.4 (4H, m), 2.0 (1H, m), 1.9 (3H, s), 1.6 (9H, m), 1.2 (9H, m).
Ex.227 (CDCl₃) δ 7.6-7.0 (9H, m), 6.0 (1H, m), 4.6-4.0 (5H, m), 3.8-3.2 (6H, m), 2.9-2.2 (3H, m), 2.0-1.2 (19H, m).
Ex.228 (CDCl₃) δ 7.8-7.1 (9H, m), 6.6 (1H, m), 4.6-4.2 (4H, m), 3.6-3.0 (4H, m), 2.9-2.0 (3H, m), 2.0-1.2 (21H, m).
Ex.229 (CDCl₃) δ 8.1 (1H, t), 7.6 (1H, d), 7.4-7.1 (7H, m), 6.0 (1H, t), 4.8 (1H, m), 4.6 (1H, s), 4.4 (1H, s), 4.1 (1H, dd), 3.8 (1H, dd), 3.5 (2H, m), 2.9-2.2 (6H, m), 2.0 (3H, s), 1.6 (6H, m), 1.4 (6H, s).
Ex.230 (CDCl₃) δ 7.6-6.9 (9H, m), 5.8 (1H, m), 4.5 (2H, m), 4.2-3.6 (3H, m), 3.2 (2H, m), 2.5 (2H, m), 2.2-1.2 (19H, m).
Ex.231 (d⁶-DMSO) δ 12.6 (1H, br s), 8.1 (1H, d), 7.3 (3H, m), 7.0 (5H, m), 4.5 (2H, d), 4.1 (1H, m), 3.9 (1H, m), 3.4-2.9 (7H, m), 2.7-2.3 (4H, m), 2.0 (1H, m), 1.8 (3H, s), 1.6 (9H, m), 1.2 (9H, m).
Ex.232 (d⁶-DMSO) δ 12.5 (1H, br s), 8.2 and 8.0 (1H, 2xd), 7.4-6.9 (9H, m), 4.5 (2H, m), 4.1 (2H, m), 3.7 (3H, s), 3.5-2.3 (8H, m), 2.2-1.5 (13H, m), 1.4-1.1 (9H, m).
Ex.233 (d⁶-DMSO) δ 12.6 (1H, br s), 8.6 and 8.4 (1H, 2xt), 8.2 and 7.8 (1H, 2xt), 7.6 (1H,m), 7.3 (3H, m), 7.0 (4H, m), 5.0 and 4.7 (1H, 2xd), 4.55 (2H, d), 4.5-4.1 (2H, m), 3.9-3.5 (4H, m), 3.3-2.4 (4H, m), 1.9 (3H, s), 1.6 (6H, m), 1.3 (6H, m).
Ex.234 (d⁶-DMSO) δ 12.6 (1H, br s), 8.9 and 8.3 (1H, 2xm), 7.6 (1H, t), 7.3 (3H, m), 7.0 (5H, m), 5.1 and 4.6 (1H, 2xm), 4.5 (1H, s), 4.4 (1H, s), 4.3-3.6 (6H, m), 3.4-2.2 (4H, m), 1.9 (3H, s), 1.6-1.3 (12H, m).
Ex.235 (d⁶-DMSO) δ 12.7 (1H, br s), 9.0 (1H, m), 8.6-8.2 (3H, m), 7.7-6.9 (6H, m), 4.9-3.0 (9H, m), 2.8-2.2 (4H, m), 1.9 (3H, m), 1.6-1.3 (12H, m).
Ex.236 (d⁶-DMSO) δ 9.4 (1H, s), 8.4 (2H, s), 8.3 (1H, t), 8.1 (1H, s), 7.3 (3H, m), 7.1 (3H, m), 6.9 (2H, m), 4.6 (2H, s), 4.3 (1H, m), 3.5 (2H, m), 3.1 and 3.0 (2H, 2xd), 2.8 and 2.6 (2H, m), 2.1-1.2 (19H, m).
Ex.237 (d⁶-DMSO) δ 11.7 (1H, s), 10.2 (1H, s), 8.44 (2H, s), 8.4 (1H, t), 8.1 (1H, s), 7.3 (3H, m), 7.1 (3H, m), 6.9 (2H, m), 4.5 (2H, s), 4.0 (1H, m), 3.5 (2H, m), 3.1 and 3.0 (2H, 2xd), 2.8-2.5 (2H, m), 2.0-1.1 (19H, m).
Ex.238 (d⁶-DMSO) δ 8.1 and 8.0 (1H, 2xd), 7.3 (3H, m), 7.0 (5H, m), 7.0 and 6.3 (1H, 2xt), 4.5 (2H, m), 4.2-2.1 (12H, m), 2.0-1.1 (22H, m).

The compounds of the examples were tested for binding at the CCK₈ receptor in mouse cortical membranes by means of a radioligand binding assay. The procedure was as follows:

The whole brains from male mice (CD1 22-25g; Charles River) were removed and placed in ice-cold buffer (pH7.2 @ 21 ± 3°C) of the following composition (mM); 10 HEPES, 130 NaCl, 4.7 KCl, 5 MgCl₂, 1 EDTA and containing 0.25g.l⁻¹ bacitracin. The cortex was dissected, weighed and homogenised in 40ml ice-cold buffer using a Teflon-in-glass homogeniser. The homogenate was centrifuged at 39,800g for 20 min at 4°C, the supernatant discarded and the pellet resuspended by homogenisation in fresh buffer. The homogenate was recentrifuged (39,800g; 20 min @ 4°C) and the final pellet was resuspended in HEPES buffer to give a tissue concentration of 2mg.ml⁻¹ (original wet weight).

The membranes (400ml) were incubated for 150 min at 21 ± 3°C in a final volume of 0.5ml with HEPES buffer containing [¹²⁵I]-CCK8S (0.05ml; 200pM NEN 2200Ci.mmol⁻¹) and competing compound. Total and non-specific binding of [¹²⁵I]-CCK8S were defined using 0.05ml of buffer and 0.05ml of 10mM L-365,260, respectively. The assay was terminated by rapid filtration through pre-soaked Whatman GF/B filters using a Brandell Cell harvester. The filters were washed (3 x 3ml) with ice-cold 50mM Tris-HCl (pH7.4 @ 4°C) and bound radioactivity determined by counting (1 min.) in a gamma-counter.

The results obtained from the CCK_{B} assays are set out in Table 1.

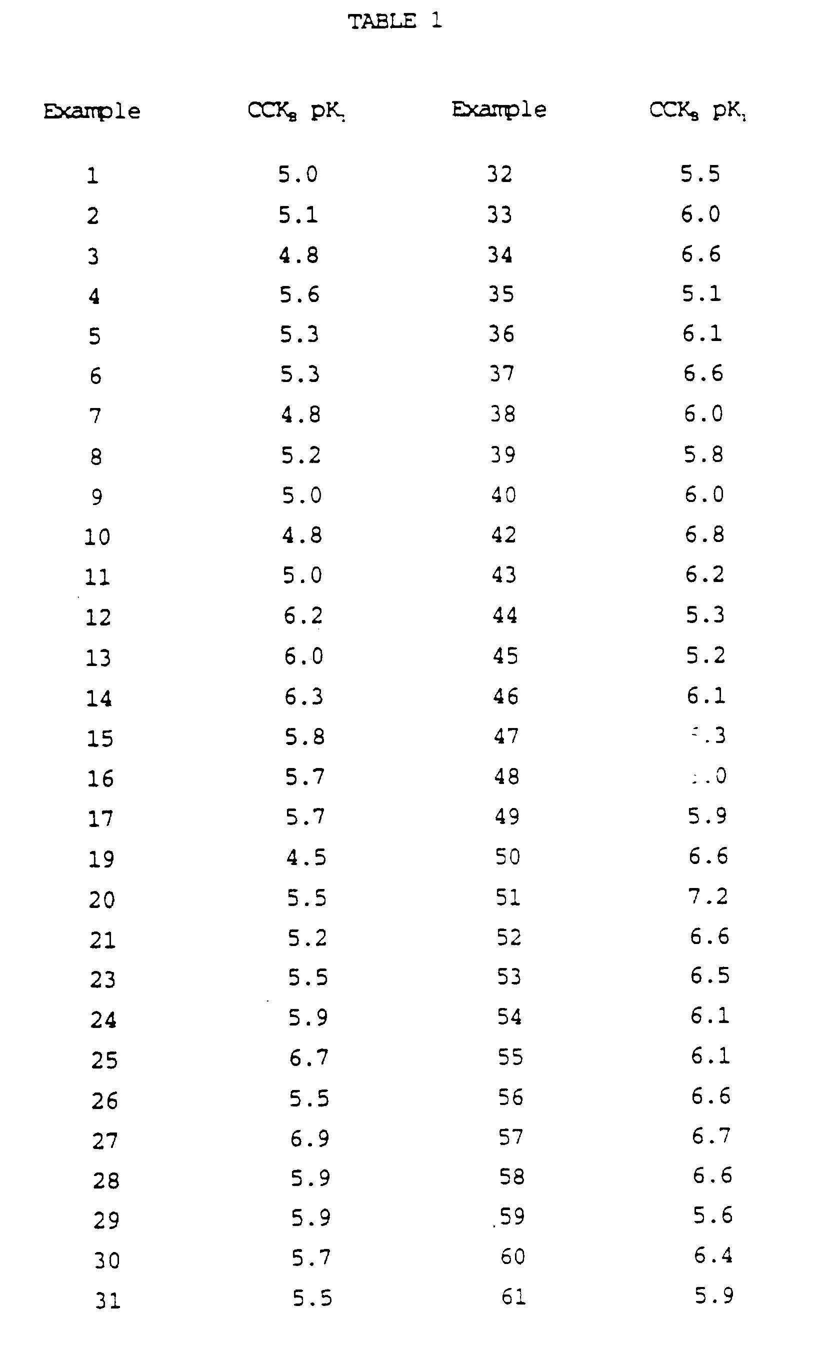

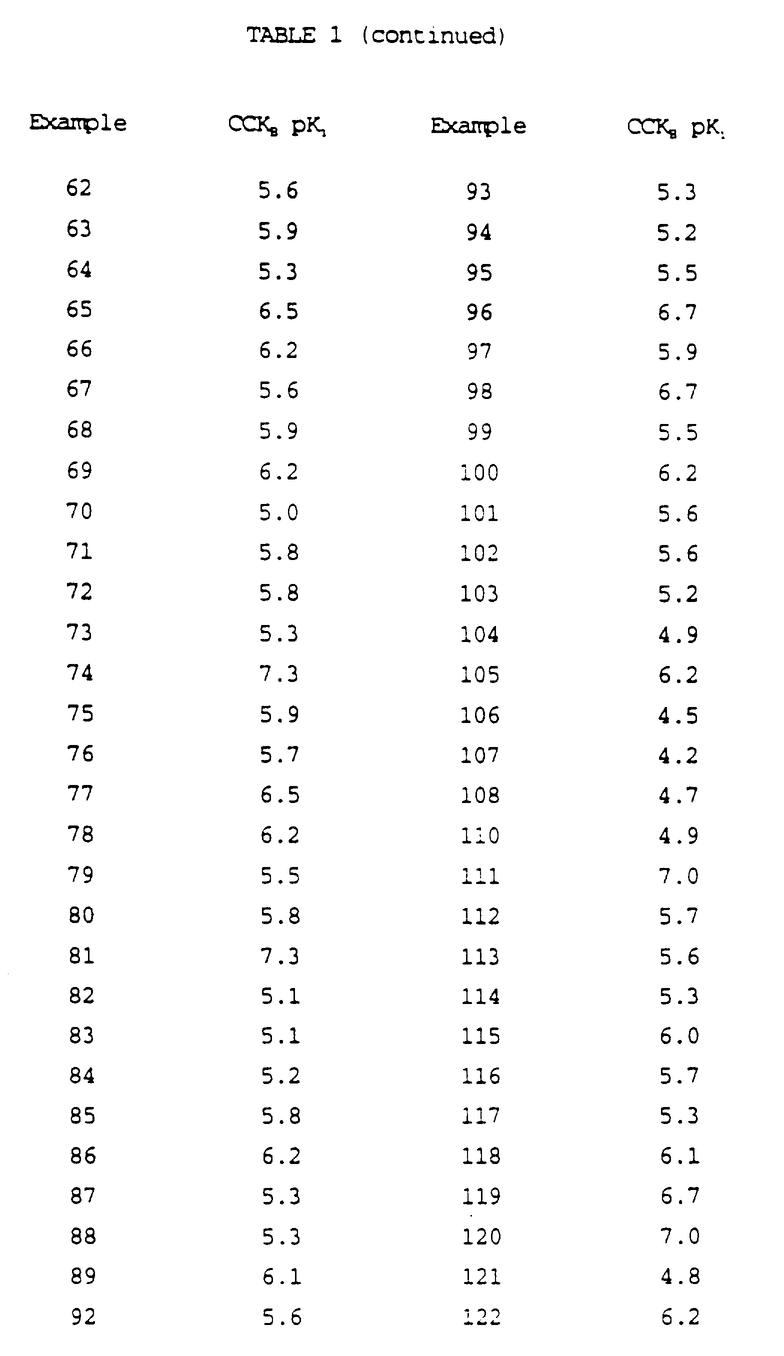

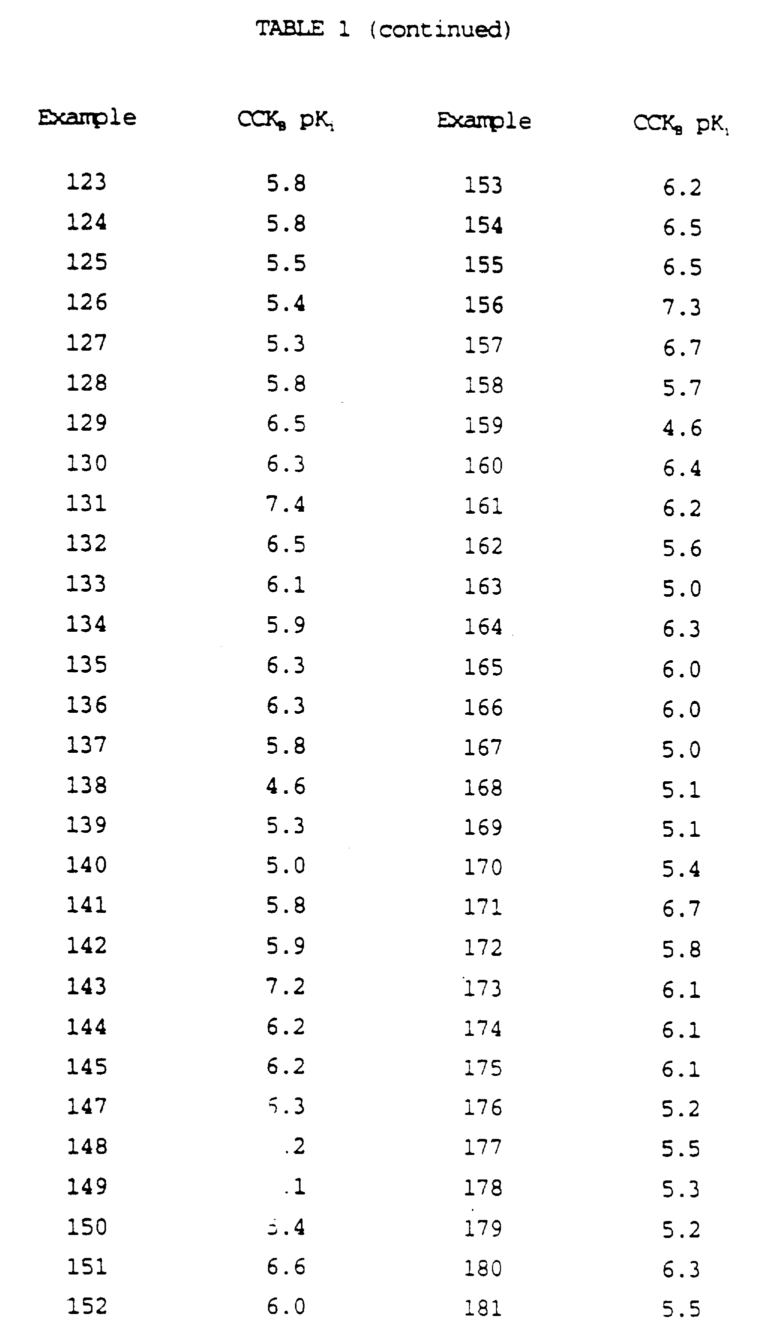

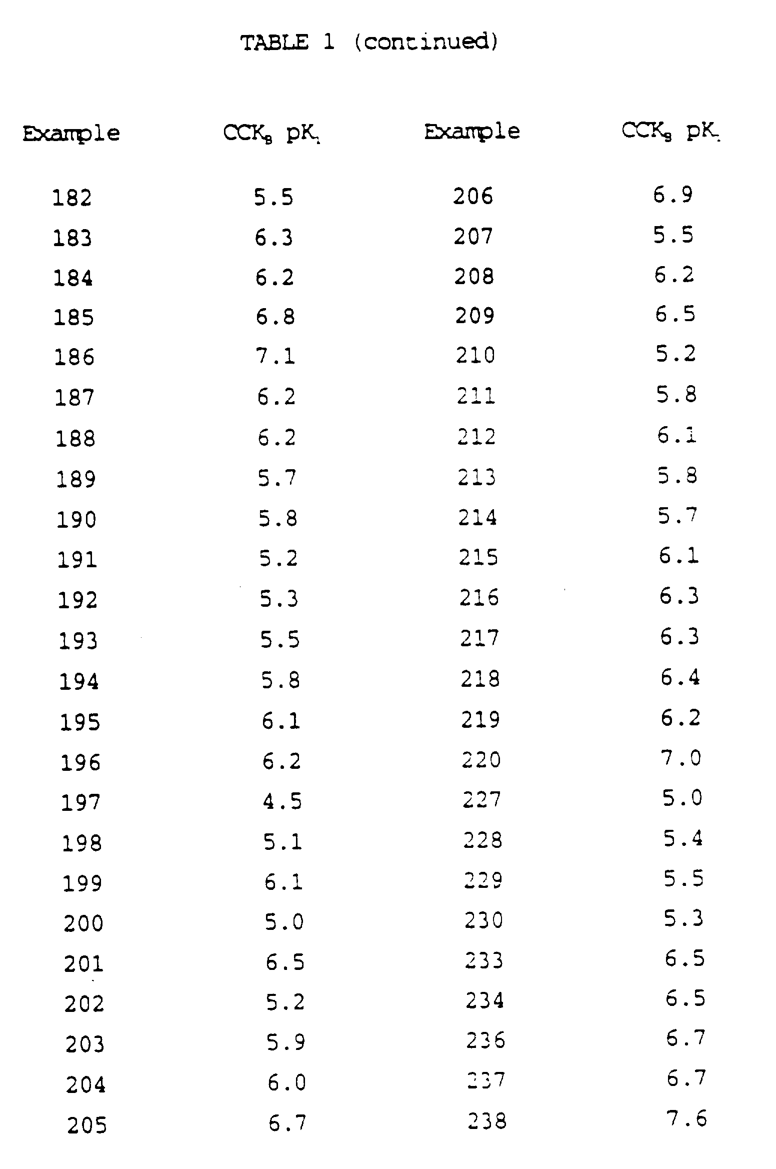

The compounds of the examples were also tested for gastrin antagonist activity in an immature rat stomach assay. The procedure was as follows:

The oesophagus of immature rats (33-50 g, ca. 21 days old) was ligated at the level of the cardiac sphincter and the duodenal sphincter was cannulated. The stomach was excised and flushed with ca. 1 ml of unbuffered physiological saline solution. The fundus was punctured and cannulated. A further 4-5 ml of unbuffered solution was flushed through the stomach to ensure the preparation was not leaking. The stomach was lowered into a jacketed organ bath containing 40 ml of buffered solution containing 3x10⁻⁸M 5-methylfurmethide, maintained at 37° and gassed vigorously with 95% O₂/ 5% CO₂. The stomach was continuously perfused at a rate of 1 ml min⁻¹ with unbuffered solution gassed with 100% O₂ with the perfusate passing over an internally referenced pH-electrode fixed 12 cm above the stomach.

After 120 min of stabilisation the drugs were added directly to the serosal solution in the organ bath and after a further 60 min cumulative pentagastrin dose-response curves were started. Changes in acid secretion were monitored and the curves analysed according to Black et.al., Br. J. Pharmacol., 1985, 86, 581.

The results obtained from the gastrin assays are set out in Table 2.

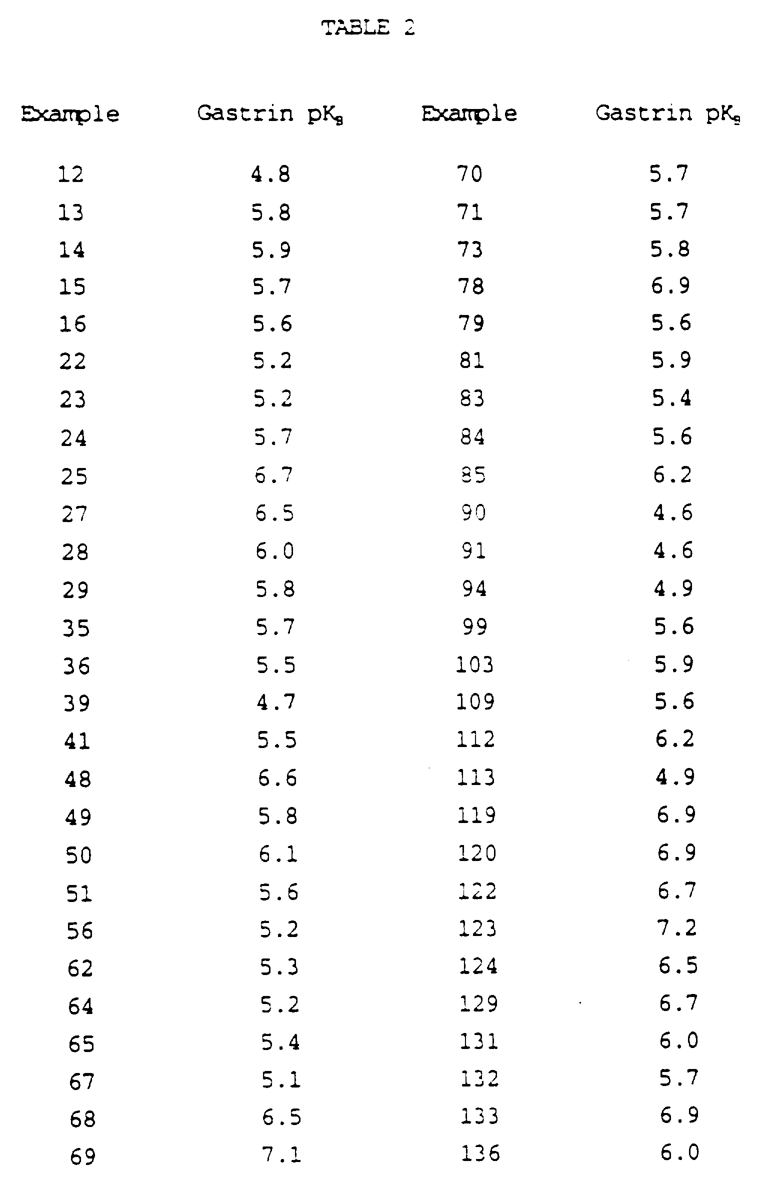

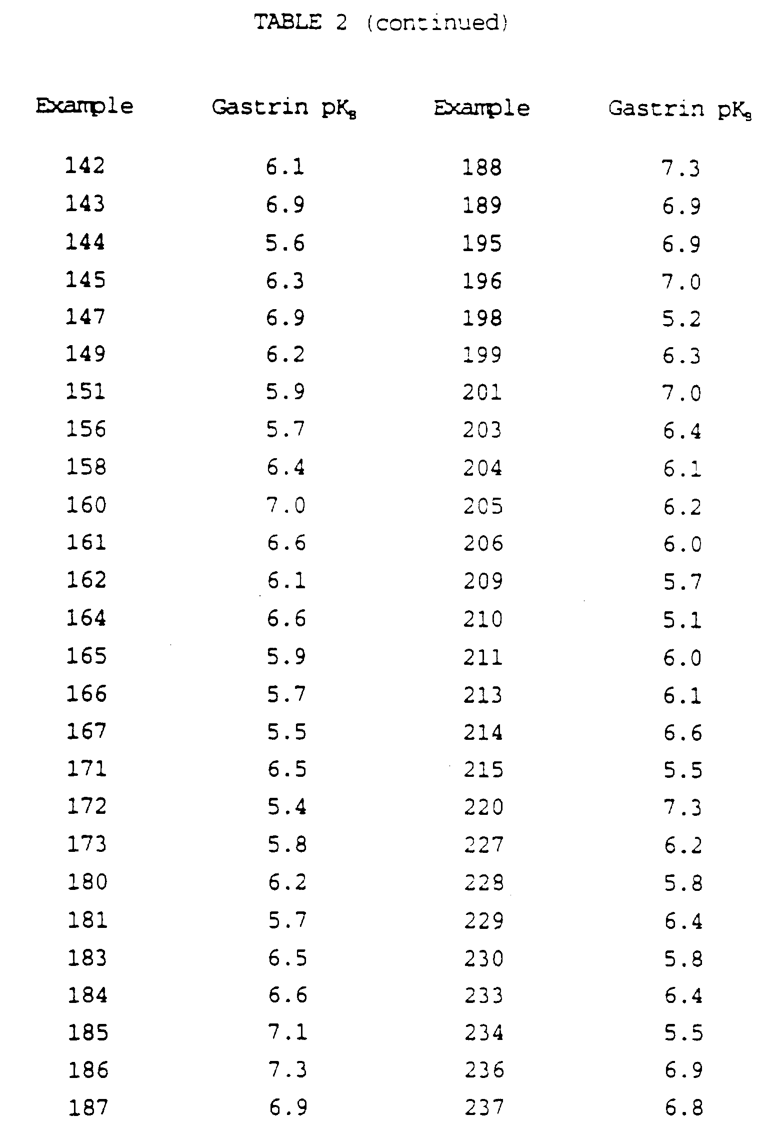

The compounds of the examples were also tested in a CCK_{A} binding assay as follows:

The pancreatata were removed from male guinea-pigs (200-300g; Dunkin Hartley) and placed in ice-cold HEPES buffer (pH 7.2 @ 21 ± 3° ). The pancreatata were homoogenised in 40 ml ice-cold HEPES buffer using a polytron (Brinkmann, PT10, setting 10) 4 x 1 second.

The homogenate was centrifuged at 39,800g for 15 min at 4°. The supernatant was discarded and the pellet re-suspended using a Teflon-in-glass homogeniser in 20 volumes of fresh buffer and recentrifuged as above. The final pellet was re-suspended using a Teflon-in-glass homogeniser to a tissue concentration of 1 mg.ml⁻¹ (original wet weight), and filtered through 500 µm pore-size Nytex mesh.

The membranes (400µl; containing 0.375 µM PD134,308) are incubated for 150 minutes at 21 ± 3° in a final volume of 0.5ml with HEPES buffer containing [¹²⁵I]-CCK₈(S) (50µl; 200pM) and competing compound. Total and non-specific binding of [¹²⁵I]-CCK₈(S) are defined using 50µl of buffer and 50µl of 100nM L-364,718 respectively. The assay is terminated by rapid filtration through pre-soaked Whatman GF/B filters using a Brandell Cell Harvester. The filters were washed (3 x 3ml) with ice-cold 50mM Tris HCl (pH 7.4 at 4° ) and bound radioactivity is determined by counting (1 min) in a gamma counter.

The results are set out in Table 3.

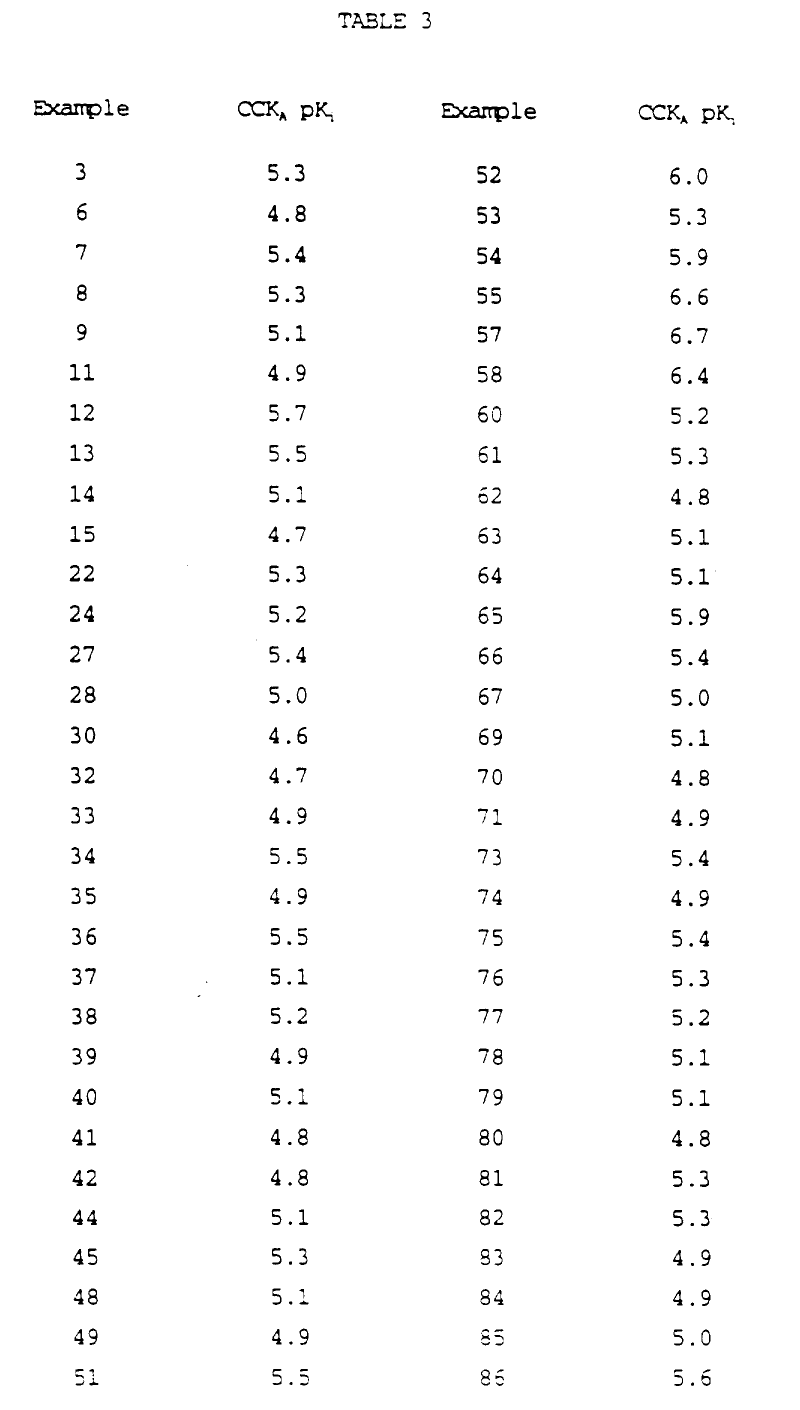

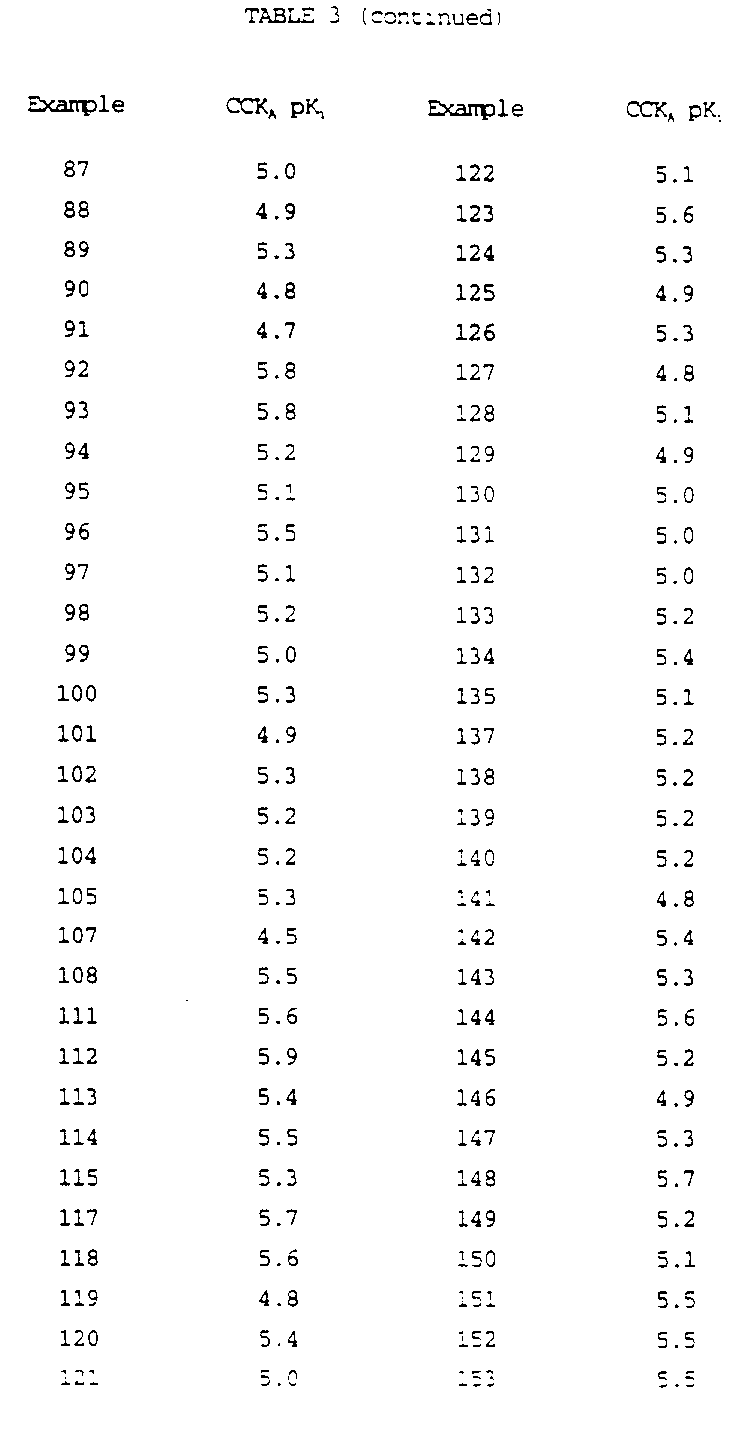

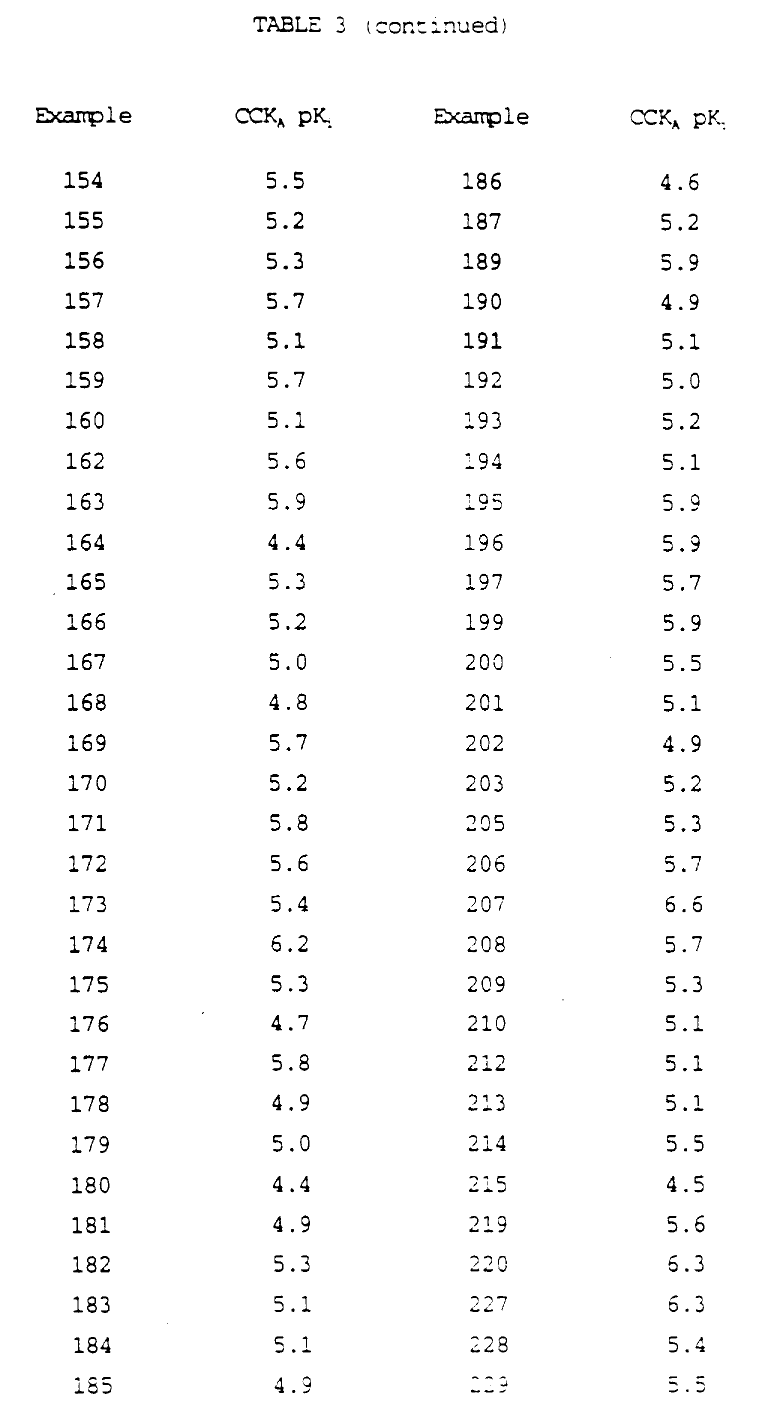

## Claims

1. A compound of the formula 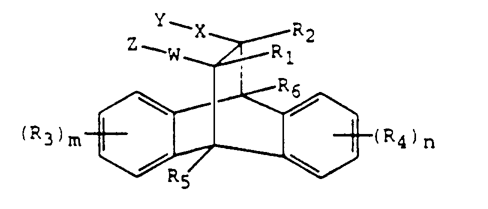 wherein
W is a carbonyl, sulphonyl or sulphinyl group, and X is a carbonyl, sulphonyl or sulphinyl group or -C(O)-CH₂- (in which the carbonyl group is bonded to Y), provided that at least one of W and X contains carbonyl,
Y is R₇-O- or R₇-N(R₈) (wherein R₇ is H or C₁ to C₁₅ hydrocarbyl, up to two carbon atoms of the hydrocarbyl moiety optionally being replaced by a nitrogen, oxygen or sulphur atom provided that Y does not contain a -O-O-group, and R₈ is H, C₁ to C₃ alkyl, carboxymethyl or esterified carboxymethyl),
Z is selected from
i) -O-R₉
wherein R₉ is H, C₁ to C₅ alkyl, phenyl, substituted phenyl, benzyl or substituted benzyl; 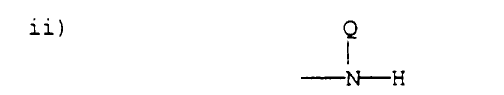
wherein Q is H, C₁ to C₅ hydrocarbyl, or -R₁₀-U, wherein R₁₀ is a bond or C₁ to C₃ alkylene and U is aryl, substituted aryl, heterocyclic, or substituted heterocyclic, 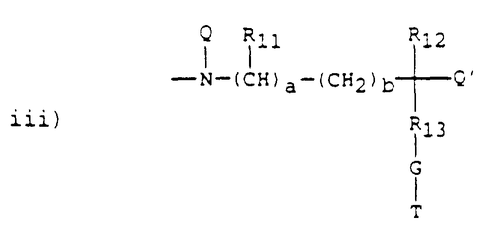
wherein a is 0 or 1 and b is from 0 to 3,
R₁₁ is H or methyl,
R₁₂ is H or methyl; or R₁₁ is CH₂ = and Q' is absent; or R₁₁ and R₁₂ are linked to form a 3- to 7-membered ring,
R₁₃ is a bond or C₁ to C₅ hydrocarbylene,
G is a bond, -CHOH- or -C(O)-
Q' is as recited above for Q or -R₁₀-(C(O))_{d}-L-(C(O))ₑ-R (wherein R₉ and R₁₀ are as defined above, L is O, S or -N(R₁₄)-, in which R₁₄ is as defined above for R₈, and d and e are 0 or 1, provided that d+e<2) ; or Q' and R₁₂, together with the carbon atom to which they are attached, form a 3- to 7-membered ring,
Q is as defined above; or Q and R₁₂ together form a group of the formula -(CH₂)_{f}-V-(CH₂)_{g}- wherein V is -S-, -S(O)-, -S(O)₂-, -CH₂-, -CHOH- or -C(O)-, f is from 0 to 2 and g is from 0 to 3; or, when Q' is -R₁₀-U and U is an aromatic group, Q may additionally represent a methylene link to U, which link is *ortho* to the R₁₀ link to U,
T is H, cyano, C₁ to C₄ alkyl, -CH₂OH, carboxy, esterified carboxy or amidated carboxy; or 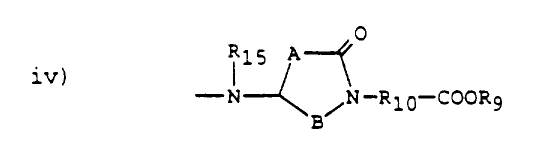
wherein A and B are independently a bond or C₁ to C₃ alkylene, provided that A and B together provide from 2 to 4 carbon atoms in the ring, R₉ and R₁₀ are as defined above, and R₁₅ is as defined above for R₈
or Z is absent and W is H,
R₁ is H, methyl, halo, carboxy, esterified carboxy, amidated carboxy, carboxymethyl, esterified carboxymethyl or amidated carboxymethyl,
R₂ is selected from the groups recited above for R₁ ; or, when Z is absent and W is H, R₂ may additionally represent -C(O)-Z' wherein Z' is selected from the groups recited above for Z; or R₁ and R₂ together form a second bond between the carbon atoms to which they are attached,
R₃ and R₄ (or each R₃ and R₄ group, when m or n is 2 or more) are independently selected from halo, amino, nitro, cyano, sulphamoyl, C₁ to C₃ alkyl, C₁ to C₃ alkoxy, carboxy, esterified carboxy and amidated carboxy,
R₅ and R₆ are independently selected from H and the groups recited above for R₃
m is from 0 to 4, provided that m is not more than 2 unless R₃ is exclusively halo,
n is from 0 to 4, provided that n is not more than 2 unless R₄ is exclusively halo,
or a pharmaceutically acceptable salt thereof, for use in therapy.

2. A compound of the formula 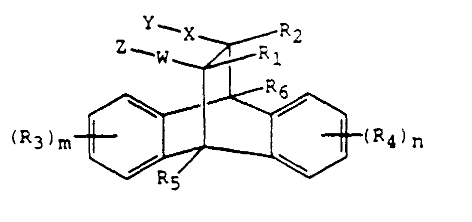 wherein
W is a carbonyl, sulphonyl or sulphinyl group, and X is a carbonyl, sulphonyl or sulphinyl group or -C(O)-CH₂- (in which the carbonyl group is bonded to Y), provided that at least one of W and X contains carbonyl,
Y is R₇-O- or R₇-N(R₆)- (wherein R₇ is H or C₁ to C₁₅ hydrocarbyl, up to two carbon atoms of the hydrocarbyl moiety optionally being replaced by a nitrogen, oxygen or sulphur atom provided that Y does not contain a -O-O-group, and R₈ is H, C₁ to C₉ alkyl, carboxymethyl or esterified carboxymethyl),
Z is selected from
i) -O-R₉
wherein R₉ is H, C₁ to C₅ alkyl, phenyl, substituted phenyl, benzyl or substituted benzyl; 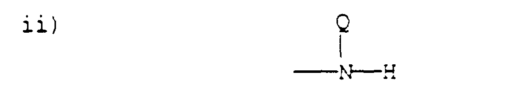
wherein Q is H, C₁ to C₅ hydrocarbyl, or -R₁₀-U, wherein R₁₀ is a bond or C₁ to C₃ alkylene and U is aryl, substituted aryl, heterocyclic, or substituted heterocyclic, 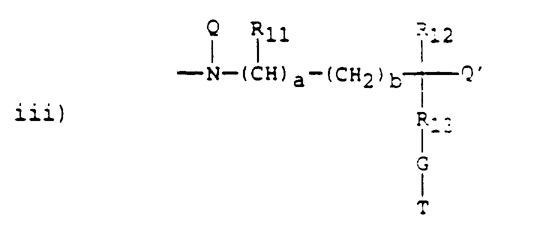
wherein a is 0 or 1 and b is from 0 to 3,
R₁₁ is H or methyl,
R₁₂ is H or methyl; or R₁₂ is CH = and Q' is absent; or R₁₁ and R₁₂ are linked to form a 3- to 7-membered ring,
R₁₃ is a bond or C₁ to C₅ hydrocarbylene,
G is a bond, -CHOH- or -C(O)-
Q' is as recited above for Q or -R₁₀-(C(O))_{d}-L-(C(O))ₑ-R₉ (wherein R₉ and R₁₀ are as defined above, L is O, S or -N(R₁₄)-, in which R₁₄ is as defined above for R₁₂, and d and e are 0 or 1, provided that d+e<2) ; or Q' and R₁₂, together with the carbon atom to which they are attached, form a 3- to 7-membered ring,
Q is as defined above; or Q and R₁₂ together form a group of the formula -(CH₂)_{f}-V-(CH₂)_{g}- wherein V is -S-, -S(O)-, -S(O)₂-, -CH₂-, -CHOH- or -C(O)-, f is from 0 to 2 and g is from 0 to 3; or, when Q' is -R₁₀-U and U is an aromatic group, Q may additionally represent a methylene link to U, which link is ortho to the R₁₀ link to U,
T is H, cyano, C₁ to C₄ alkyl, -CH₂OH, carboxy, esterified carboxy or amidated carboxy; or 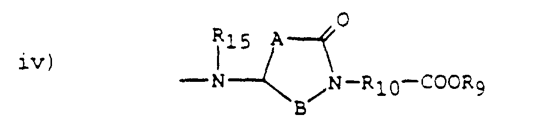
wherein A and B are independently a bond or C₁ to C₃ alkylene, provided that A and B together provide from 2 to 4 carbon atoms in the ring, R₉ and R₁₀ are as defined above, and R₁₅ is as defined above for R₈
or Z is absent and W is H,
R₁ is H, methyl, halo, carboxy, esterified carboxy, amidated carboxy, carboxymethyl, esterified carboxymethyl or amidated carboxymethyl,
R₂ is selected from the groups recited above for R₁; or, when Z is absent and W is H, R₂ may additionally represent -C(O)-Z' wherein Z' is selected from the groups recited above for Z; or R₁ and R₂ together form a second bond between the carbon atoms to which they are attached,
R₃ and R₄ (or each R₃ and R₄ group, when m or n is 2 or more) are independently selected from halo, amino, nitro, cyano, sulphamoyl, C₁ to C₃ alkyl, C₁ to C₃ alkoxy, carboxy, esterified carboxy and amidated carboxy,
R₅ and R₆ are independently selected from H and the groups recited above for R₃
m is from 0 to 4, provided that m is not more than 2 unless R₃ is exclusively halo,
is from 0 to 4, provided than n is not more than 2 unless R₄ is exclusively halo,
or a pharmaceutically acceptable salt thereof, provided that
if one (but only one) of R₁ and R₂ is methyl, m and n are not both 0,
Z is not hydroxy or methoxy when Y is hydroxy or methoxy,
Z and Y are not trans to each other when Z is R₉-O- and Y is R₇-O- ,
-X-Y is not equal to -W-Z when R₁=R₂=H and m=n=0, and
if Z is absent and R₁ and R₂ are both H or form part of a double bond, Y is not R₇-O-,
and further provided that said compound is not 7-(N,N-dimethylaminocarbonyl)-8-methyl-2,3,5,6-dibenzobicyclo[2.2.2]octane or 7-(N-methyl-N-phenylaminocarbonyl)-8-methyl-2,3,5,6-dibenzobicyclo[2.2.2]octane.

3. A compound according to claim 1 or claim 2 wherein R₇ is C₆ to C₈ straight or branched chain alkyl, or R₂₅-(CH₂)ₚ-, wherein R₂₅ is selected from phenyl, 1-naphthyl, 2-naphthyl, indolyl, norbornyl, adamantyl or cyclohexyl, and p is from 0 to 3.

4. A compound according to any preceding claim wherein W is carbonyl and X is sulphonyl.

5. A compound according to any of claims 1 to 3 wherein W is carbonyl and X is carbonyl.

6. A compound according to any of claims 1 to 3 wherein W is sulphonyl and X is carbonyl.

7. A compound according to any preceding claim wherein R₁ and R₂ are both H.

8. A compound according to any preceding claim wherein m and n are both 0.

9. A pharmaceutical composition comprising a compound according to any preceding claim, together with a pharmaceutically acceptable diluent or carrier.

10. A method of making a compound according to claim 2 wherein W is carbonyl, said method including the step of reacting a compound of the formula 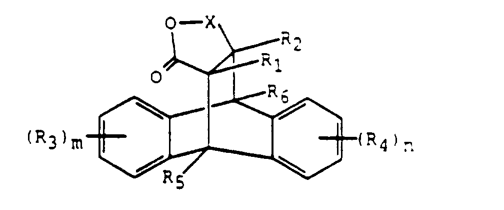 with a compound of formula YH, wherein Y is as defined in claim 2.

11. A method of making a compound according to claim 2 wherein W is sulphonyl, said method comprising the step of reacting a compound of the formula 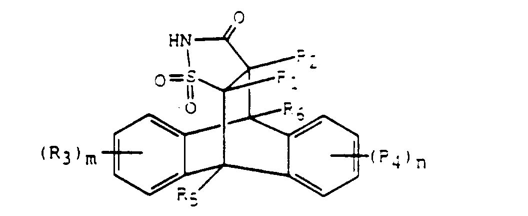 with a compound of the formula R₇-Hal, wherein Hal represents a halogen atom and R₇ is as defined in claim 2, and then reacting the product with an alkoxide.

12. A method of making a compound according to claim 2 wherein W or X is sulphinyl, said method comprising the step of reacting a compound of the formula: 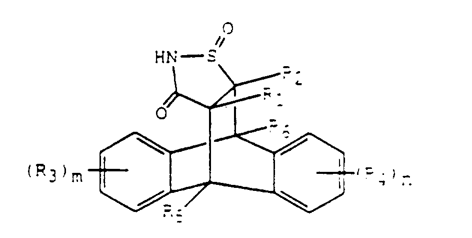 with a compound of the formula R₇-Hal, wherein Hal represents a halogen atom and R₇ is as defined in claim 2, and then reacting the product with an alkoxide.

13. A method of making a pharmaceutical composition according to claim 9, comprising mixing a compound according to any of claims 1 to 8 with a pharmaceutically acceptable diluent or carrier.

## Patentansprüche

1. Verbindung der Formel 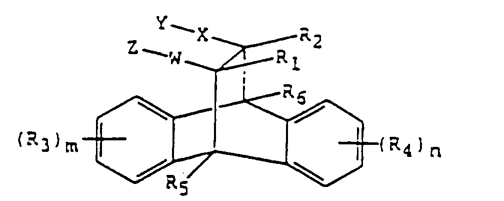 worin
W eine Carbonyl-, Sulfonyl- oder Sulfinylgruppe und X eine Carbonyl-, Sulfonyl- oder Sulfinylgruppe oder -C(O)-CH₂- ist (worin die Carbonylgruppe an Y gebunden ist), vorausgesetzt, daß wenigstens eines von W und X Carbonyl enthält,
Y R₇-O- oder R₇-N(R₉)- ist (worin R₇ H oder C₁ bis C₁₅-Hydrocarbyl ist, wobei bis zu 2 Kohlenstoffatome des Hydrocarbylanteils gegebenenfalls durch ein Stickstoff-, Sauerstoff- oder Schwefelatom ersetzt sind, vorausgesetzt, daß Y keine -O-O-Gruppe enthält und R₈ H, C₁ bis C₃-Alkyl, Carboxymethyl oder verestertes Carboxymethyl ist),
Z ausgewählt ist aus
i) -O-R₉
worin R₉ H, C₁ bis C₅-Alkyl, Phenyl, substituiertes Phenyl, Benzyl oder substituiertes Benzyl ist, 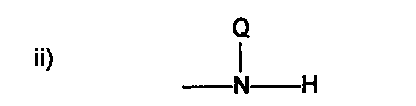
worin Q H, C₁ bis C₅-Hydrocarbyl oder -R₁₀-U ist, worin R₁₀ eine Bindung oder C₁ bis C₃- Alkylen und U Aryl, substituiertes Aryl, heterocyclisch oder substituiert heterocyclisch ist, 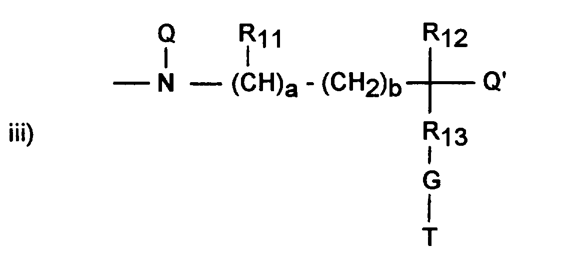
worin a 0 oder 1 und b von 0 bis 3 bedeutet,
R₁₁ H oder Methyl ist,
R₁₂ H oder Methyl ist, oder R₁₂ CH₂= und Q' nicht vorhanden ist, oder R₁₁ und R₁₂ zu einem 3- bis 7-gliedrigen Ring verbunden sind,
R₁₃ eine Bindung oder C₁ bis C₅-Hydrocarbylen ist,
G eine Bindung, -CHOH- oder -C(O)- ist,
Q' die oben für Q angeführte Bedeutung hat oder -R₁₀-(C(O))_{d}-L-(C(O))ₑ-R₉ ist (worin R₉ und R₁₀ die oben angegebene Bedeutung haben, L O, S oder -N(R₁₄)- ist, worin R₁₄ die oben für R₈ angegebene Bedeutung hat und d und e 0 oder 1 sind, vorausgesetzt, daß d+e<2 sind), oder Q' und R₁₂ zusammen mit dem Kohlenstoffatom, an das sie gebunden sind, einen 3- bis 7-gliedrigen Ring bilden,
Q die oben angegebene Bedeutung hat oder Q und R₁₂ gemeinsam eine Gruppe der Formel -(CH₂)_{f}V-(CH₂)_{g}- bilden, worin V -S-, -S(O)-, -S(O)₂-, -CH₂-, -CHOH- oder -C(O)- ist, f von 0 bis 2 und g von 0 bis 3 bedeutet, oder, wenn Q' -R₁₀-U und U eine aromatische Gruppe ist, Q zusätzlich ein Methylenglied zu U darstellen kann, wobei sich das Glied in ortho-Stellung zum R₁₀-Glied zu U befindet,
T H, Cyano, C₁ bis C₄-Alkyl, -CH₂OH, Carboxy, verestertes Carboxy oder amidiertes Carboxy ist oder 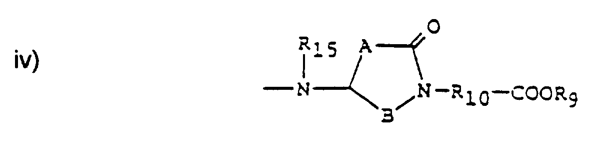
worin A und B unabhängig eine Bindung oder C₁ bis C₃-Alkylen sind, vorausgesetzt, daß A und B zusammen 2 bis 4 Kohlenstoffatome in dem Ring bereitstellen, R₉ und R₁₀ die oben angegebene Bedeutung haben und R₁₅ die oben angegebene Bedeutung für R₈ hat,
oder Z nicht vorhanden und W H ist,
R₁ H, Methyl, Halogen, Carboxy, verestertes Carboxy, amidiertes Carboxy, Carboxymethyl, verestertes Carboxymethyl oder amidiertes Carboxymethyl ist,
R₂ ausgewählt ist aus den oben für R₁ angegebenen Gruppen oder, wenn Z nicht vorhanden und W H ist, R₂ zusätzlich -C(O)-Z' darstellen kann, worin Z' ausgewählt ist aus den oben für Z angegebenen Gruppen, oder R₁ und R₂ gemeinsam eine zweite Bindung zwischen den Kohlenstoffatomen, an die sie gebunden sind, bilden,
R₃ und R₄ (oder jede R₃ und R₄-Gruppe, wenn m oder n 2 oder mehr darstellen) unabhängig ausgewählt sind aus Halogen, Amino, Nitro, Cyano,
Sulfamoyl, C₁ bis C₃-Alkyl, C₁ bis C₃-Alkoxy, Carboxy, verestertem Carboxy und amidiertem Carboxy,
R₅ und R₆ unabhängig ausgewählt sind aus H und den oben für R₃ genannten Gruppen,
m von 0 bis 4 bedeutet, vorausgesetzt, daß m nicht größer als 2 ist, wenn R₃ nicht ausschließlich Halogen ist,
n von 0 bis 4 bedeutet, vorausgesetzt, daß n nicht größer als 2 ist, wenn R₄ nicht ausschließlich Halogen ist,
oder ein pharmazeutisch unbedenkliches Salz daraus für den Einsatz bei der Therapie.

2. Verbindung der Formel 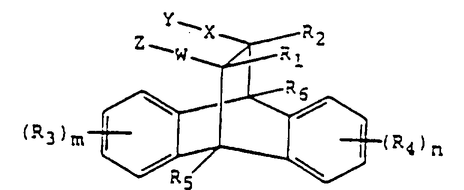 worin
W eine Carbonyl-, Sulfonyl- oder Sulfinylgruppe und X eine Carbonyl-, Sulfonyl- oder Sulfinylgruppe oder -C(O)-CH₂- ist (worin die Carbonylgruppe an Y gebunden ist), vorausgesetzt, daß wenigstens eines von W und X Carbonyl enthält,
Y R₇-O- oder R₇-N(R₆)- ist (worin R₇ H oder C₁ bis C₁₅-Hydrocarbyl ist, wobei bis zu 2 Kohlenstoffatome des Hydrocarbylanteils gegebenenfalls durch ein Stickstoff-, Sauerstoff- oder Schwefelatom ersetzt ist, vorausgesetzt, daß Y keine -O-O-Gruppe enthält und R₉ H, C₁ bis C₃-Alkyl, Carboxymethyl oder verestertes Carboxymethyl ist),
Z ausgewählt ist aus
i) -O-R₉
worin R₉ H, C₁ bis C₅-Alkyl, Phenyl, substituiertes Phenyl, Benzyl oder substituiertes Benzyl ist, 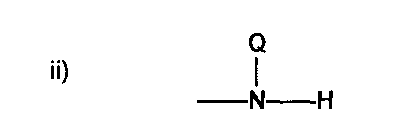
worin Q H, C₁ bis C₅-Hydrocarbyl oder -R₁₀-U ist, worin R₁₀ eine Bindung oder C₁ bis C₃- Alkylen und U Aryl, substituiertes Aryl, heterocyclisch oder substituiert heterocyclisch ist, 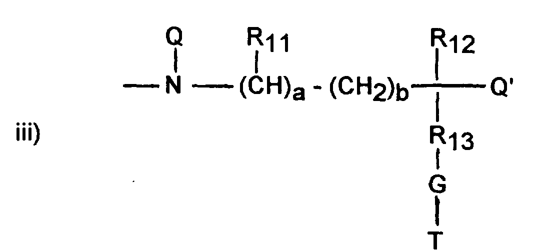
worin a 0 oder 1 und b von 0 bis 3 bedeutet,
R₁₁ H oder Methyl ist,
R₁₂ H oder Methyl ist, oder R₁₂ CH₂= und Q' nicht vorhanden ist, oder R₁₁ und R₁₂ zu einem 3- bis 7-gliedrigen Ring verbunden sind,
R₁₃ eine Bindung oder C₁ bis C₅-Hydrocarbylen ist,
G eine Bindung, -CHOH- oder -C(O)- ist,
Q' die oben für Q angeführte Bedeutung hat oder -R₁₀-(C(O))_{d}-L-(C(O))ₑ-R₉ ist (worin R₉ und R₁₀ die oben angegebene Bedeutung haben, L O, S oder -N(R₁₄)- ist, worin R₁₄ die oben für R₈ angegebene Bedeutung hat und d und e 0 oder 1 sind, vorausgesetzt, daß d+e<2 sind), oder Q' und R₁₂ zusammen mit dem Kohlenstoffatom, an das sie gebunden sind, einen 3- bis 7-gliedrigen Ring bilden,
Q die oben angegebene Bedeutung hat oder Q und R₁₂ gemeinsam eine Gruppe der Formel -(CH₂)_{f}V-(CH₂)_{g}- bilden, worin V -S-, -S(O)-, -S(O)₂-, -CH₂-, -CHOH- oder -C(O)- ist, f von 0 bis 2 und g von 0 bis 3 bedeutet, oder, wenn Q' -R₁₀-U und U eine aromatische Gruppe ist, Q zusätzlich ein Methylenglied zu U darstellen kann, wobei sich das Glied in ortho-Stellung zum R₁₀-Glied zu U befindet,
T H, Cyano, C₁ bis C₄-Alkyl, -CH₂OH, Carboxy, verestertes Carboxy oder amidiertes Carboxy ist oder 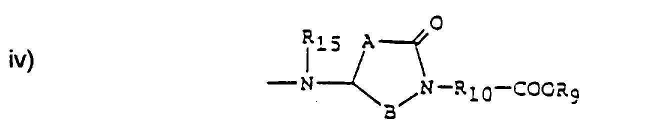
worin A und B unabhängig eine Bindung oder C₁ bis C₃-Alkylen sind, vorausgesetzt, daß A und B zusammen 2 bis 4 Kohlenstoffatome in dem Ring bereitstellen, R₉ und R₁₀ die oben angegebene Bedeutung haben und R₁₅ die oben angegebene Bedeutung für R₈ hat,
oder Z nicht vorhanden und W H ist,
R₁ H, Methyl, Halogen, Carboxy, verestertes Carboxy, amidiertes Carboxy, Carboxymethyl, verestertes Carboxymethyl oder amidiertes Carboxymethyl ist,
R₂ ausgewählt ist aus den oben für R₁ angegebenen Gruppen oder, wenn Z nicht vorhanden und W H ist, R₂ zusätzlich -C(O)-Z' darstellen kann, worin Z' ausgewählt ist aus den oben für Z angegebenen Gruppen, oder R₁ und R₂ gemeinsam eine zweite Bindung zwischen den Kohlenstoffatomen, an die sie gebunden sind, bilden,
R₃ und R₄ (oder jede R₃ und R₄-Gruppe, wenn m oder n 2 oder mehr darstellen) unabhängig ausgewählt sind aus Halogen, Amino, Nitro, Cyano, Sulfamoyl, C₁ bis C₃-Alkyl, C₁ bis C₃-Alkoxy, Carboxy, verestertem Carboxy und amidiertem Carboxy,
R₅ und R₆ unabhängig ausgewählt sind aus H und den oben für R₃ genannten Gruppen,
m von 0 bis 4 bedeutet, vorausgesetzt, daß m nicht größer als 2 ist, wenn R₃ nicht ausschließlich Halogen ist,
n von 0 bis 4 bedeutet, vorausgesetzt, daß n nicht größer als 2 ist, wenn R₄ nicht ausschließlich Halogen ist,
oder ein pharmazeutisch unbedenkliches Salz daraus, vorausgesetzt, daß
wenn eines (aber nur eines) von R₁ und R₂ Methyl ist, m und n nicht beide 0 sind,
Z nicht Hydroxy oder Methoxy ist, wenn Y Hydroxy oder Methoxy ist,
Z und Y nicht in trans-Stellung zueinander stehen, wenn Z R₉-O- und Y R₇-O- ist,
-X-Y nicht gleich -W-Z ist, wenn R₁=R₂=H und m=n=0 und
wenn Z nicht vorhanden ist und R₁ und R₂ beide H sind oder Teil einer Doppelbindung bilden, Y nicht R₇-O- ist,
und weiter vorausgesetzt, daß die Verbindung nicht 7-(N,N-Dimethylaminocarbonyl)-8-methyl-2,3,5,6-dibenzobicyclo[2.2.2]octan oder 7-(N-Methyl-N-phenylaminocarbonyl)-8-methyl-2,3,5,6-dibenzobicyclo[2.2.2]octan ist.

3. Verbindung nach Anspruch 1 oder Anspruch 2, bei der R₇ gerad- oder verzweigtkettiges C₆ bis C₈-Alkyl oder R₂₅-(CH₂)ₚ- ist, worin R₂₅ ausgewählt ist aus Phenyl, 1-Naphthyl, 2-Naphthyl, Indolyl, Norbornyl, Adamantyl oder Cyclohexyl und p von 0 bis 3 bedeutet.

4. Verbindung nach einem der vorhergehenden Ansprüche, bei der W Carbonyl und X Sulfonyl ist.

5. Verbindung nach einem der Ansprüche 1 bis 3, bei der W Carbonyl und X Carbonyl ist.

6. Verbindung nach einem der Ansprüche 1 bis 3, bei der W Sulfonyl und X Carbonyl ist.

7. Verbindung nach einem der vorhergehenden Ansprüche, bei der R₁ und R₂ beide H sind.

8. Verbindung nach einem der vorhergehenden Ansprüche, bei der m und n beide 0 sind.

9. Pharmazeutische Zusammensetzung, enthaltend eine Verbindung nach einem der vorhergehenden Ansprüche zusammen mit einem pharmazeutisch unbedenklichen Verdünnungsmittel oder Träger.

10. Verfahren zur Herstellung einer Verbindung nach Anspruch 2, wobei W Carbonyl ist, umfassend den Schritt, eine Verbindung der Formel 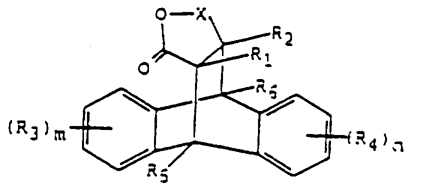 mit einer Verbindung der Formel YH, in der Y die in Anspruch 2 angegebene Bedeutung hat, umzusetzen.

11. Verfahren zur Herstellung einer Verbindung nach Anspruch 2, wobei W Sulfonyl ist, enthaltend den Schritt, eine Verbindung der Formel 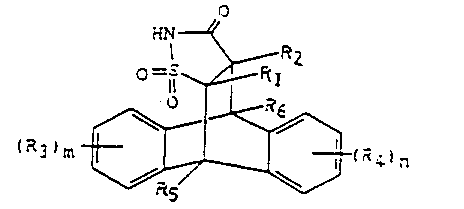 mit einer Verbindung der Formel R₇-Hal umzusetzen, in der Hal für ein Halogenatom steht und R₇ die in Anspruch 2 angegebene Bedeutung hat, und danach das Produkt mit einem Alkoholat umzusetzen.

12. Verfahren zur Herstellung einer Verbindung nach Anspruch 2, wobei W oder X Sulfinyl ist, enthaltend den Schritt, eine Verbindung der Formel: 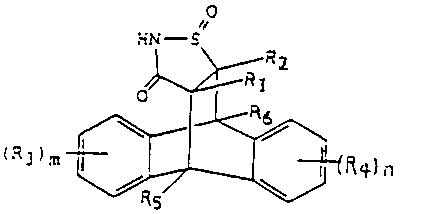 mit einer Verbindung der Formel R₇-Hal umzusetzen, in der Hal für ein Halogenatom steht und R₇ die in Anspruch 2 angegebene Bedeutung hat, und danach das Produkt mit einem Alkoholat umzusetzen.

13. Verfahren zur Herstellung einer pharmazeutischen Zusammensetzung nach Anspruch 9, enthaltend Mischen einer Verbindung nach einem der Ansprüche 1 bis 8 mit einem pharmazeutisch unbedenklichen Verdünnungsmittel oder Träger.

## Revendications

1. Composé répondant à la formule 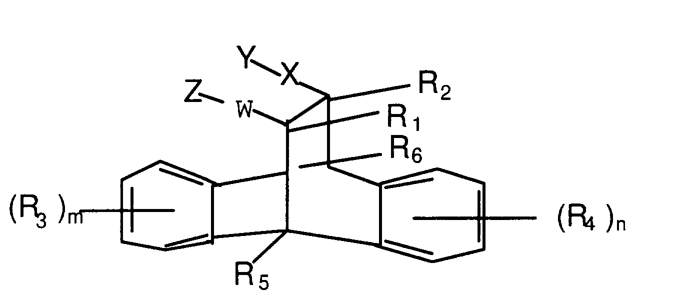 dans laquelle
W représente un groupe carbonyle, un groupe sulfonyle ou un groupe sulfinyle et X représente un groupe carbonyle, un groupe sulfonyle ou un groupe sulfinyle ou un groupe -C(O)-CH₂- (dans lequel le groupe carbonyle est lié à Y), pour autant qu'au moins un des groupes W et X contienne un groupe carbonyle.
Y représente un groupe R₇-0- ou un groupe R₇-N(R₈)- (dans lequel R₇ représente un atome d'H ou un groupe hydrocarbyle en C₁ à C₁₅, avec un remplacement facultatif de jusqu'à 2 atomes de carbone dans la partie hydrocarbyle par un atome d'azote, un atome d'oxygène ou un atome de soufre pour autant qu'Y ne contienne pas de groupe -O-O-, et R₈ représente un atome d'H, un groupe alkyle en C₁ à C₃, un groupe carboxyméthyle ou un groupe carboxyméthyle esterifié),
Z est choisi parmi les groupes :
i) -O-R₉
dans lequel le groupe R₉ est un atome d'H, un groupe alkyle en C₁ à C₅, un groupe phényle, un groupe phényle substitué, un groupe benzyle ou un groupe benzyle substitué; 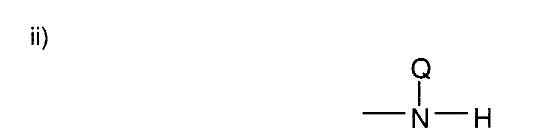
dans lequel Q est un atome d'H, un groupe hydrocarbyle en C₁ à C₅, ou un groupe -R₁₀-U, dans lequel R₁₀ représente une liaison ou un groupe alkylène en C₁ à C₃ et U représente un groupe aryle, un groupe aryle substitué, un groupe hétérocyclique ou un groupe hétérocyclique substitué, 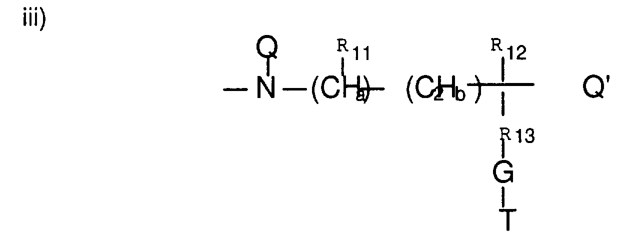
dans lequel a se situe entre 0 ou 1 et b se situe entre 0 et 3
R₁₁ représente un atome d'H ou un groupe méthyle
R₁₂ représente un atome d'H ou un groupe méthyle; ou R₁₂ représente un groupe CH₂= et Q' est absent; ou R₁₁ et R₁₂ sont liés pour former un cycle de 3 à 7 membres,
R₁₃ représente une liaison ou un groupe hydrocarbylène en C₁ à C₃
G représente une liaison, un groupe -CHOH- ou un groupe -C(O)-
Q' est comme énuméré ci-dessus pour Q ou un groupe -R₁₀-(C(O))_{d}-L-(C(O))ₑ-R₉ (dans lequel R₉ et R₁₀ sont définis comme ci-dessus, L est un atome d'O, un atome de S ou un groupe -N(R₁₄)-, dans lequel R₁₄ est défini comme l'est R₈ ci-dessus, et d et e représentent 0 ou 1, pour autant que la somme d+e soit inférieure à 2); ou alors Q' et R₁₂, ainsi que l'atome de carbone auquel ils sont liés, forment un cycle renfermant de 3 à 7 membres.
Q est défini comme ci-dessus; ou alors Q et R₁₂ forment ensemble un groupe de formule -(CH₂)_{f}-V-(CH₂)_{g}- dans lequel V représente un groupe -S-, un groupe -S(O)-, un groupe -S(O)₂-, un groupe -CH₂-, un groupe -CHOH- ou un groupe -C(O)-, f se situe entre 0 et 2 et g se situe entre 0 et 3; ou alors, si Q' représente un groupe -R₁₀-U avec U étant un groupe aromatique,
Q peut additionnellement représenter une liaison méthylène avec U, dont la liaison est *ortho* par rapport à la liaison entre R₁₀ et U,
T représente un atome d'H, un groupe cyano, un groupe alkyle en C₁ à C₄, un groupe -CH₂OH-, un groupe carboxy, un groupe carboxy esterifié ou un groupe carboxy amidé; ou alors 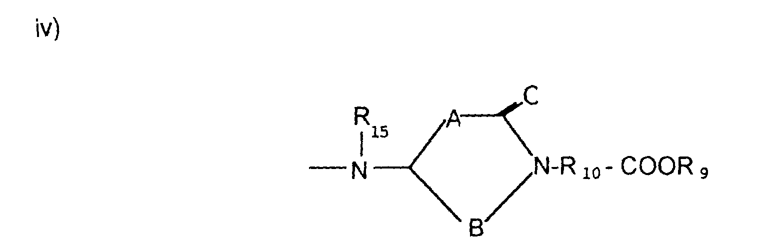
dans lequel A et B représentent de manière indépendante une liaison, ou un groupe alkylène en C₁ à C₃, pour autant que A et B fournissent ensemble entre 2 et 4 atomes de carbone dans le cycle; R₉ et R₁₀ sont définis comme ci-dessus, et R₁₅ est comme l'a été défini R₈ ci-dessus,
ou alors, Z est absent et W est un atome d'H,
R₁ représente un atome d'H, un groupe méthyle, un groupe halo, un groupe carboxy, un groupe carboxy esterifié, un groupe carboxy amidé, un groupe carboxyméthyle, un groupe carboxyméthyle esterifié, ou un groupe carboxyméthyle amidé,
R₂ est choisi parmi les groupes énumérés ci-dessus pour R₁; ou alors lorsque Z est absent et W représente un atome d'H, R₂ peut additionnellement représenter un groupe -C(O)-Z', dans lequel Z' est choisi parmi les groupes énumérés ci-dessus pour Z; ou alors R₁ et R₂ forment ensemble une seconde liaison entre les atomes de carbone auxquels ils sont liés,
R₃ et R₄ (ou chacun des groupes R₃ et R₄, lorsque m ou n est égal à 2 ou plus) sont choisis de manière indépendante parmi le groupe halo, le groupe amino, le groupe nitro, le groupe cyano, le groupe sulfamoyle, le groupe alkyle en C₁ à C₃, le groupe alkoxy en C₁ à C₃, le groupe carboxy, le groupe carboxy esterifié et le groupe carboxy amidé,
R₅ et R₆ sont choisis de manière indépendante entre l'atome d'H et les groupes énumérés ci-dessus pour R₃
m se situe entre 0 et 4, pour autant que m ne dépasse pas 2 sauf si R₃ est exclusivement un groupe halo,
n se situe entre 0 et 4, pour autant que n ne dépasse pas 2 sauf si R₄ est exclusivement un groupe halo,
ou alors un sel pharmaceutiquement acceptable correspondant, afin de l'utiliser à des fins thérapeutiques.

2. Composé répondant à la formule 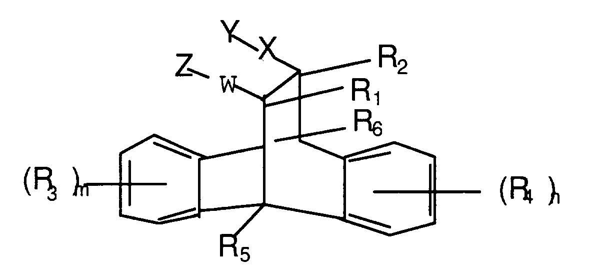 dans laquelle
W représente un groupe carbonyle, un groupe sulfonyle ou un groupe sulfinyle et X représente un groupe carbonyle, un groupe sulfonyle ou un groupe sulfinyle ou un groupe -C(O)-CH₂- (dans lequel le groupe carbonyle est lié à Y), pour autant qu'au moins un des groupes W et X contienne un groupe carbonyle.
Y représente un groupe R₇-0- ou un groupe R₇-N(R₆)- (dans lequel R₇ représente un atome d'H ou un groupe hydrocarbyle en C₁ à C₁₅, avec un remplacement facultatif de jusqu'à 2 atomes de carbone dans la partie hydrocarbyle par un atome d'azote, un atome d'oxygène ou un atome de soufre pour autant qu'Y ne contienne pas de groupe -O-O-, et R₈ représente un atome d'H, un groupe alkyle en C₁ à C₃, un groupe carboxyméthyle ou un groupe carboxyméthyle esterifié),
Z est choisi parmi les groupes :
i) -O-R₉
dans lequel le groupe R₉ est un atome d'H, un groupe alkyle en C₁ à C₅, un groupe phényle, un groupe phényle substitué, un groupe benzyle ou un groupe benzyle substitué; 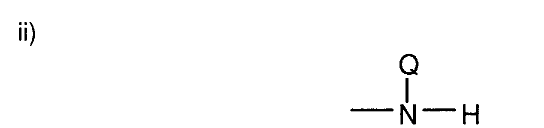
dans lequel Q est un atome d'H, un groupe hydrocarbyle en C₁ à C₅, ou un groupe -R₁₀-U, dans lequel R₁₀ représente une liaison ou un groupe alkylène en C₁ à C₃ et U représente un groupe aryle, un groupe aryle substitué, un groupe hétérocyclique ou un groupe hétérocyclique substitué, 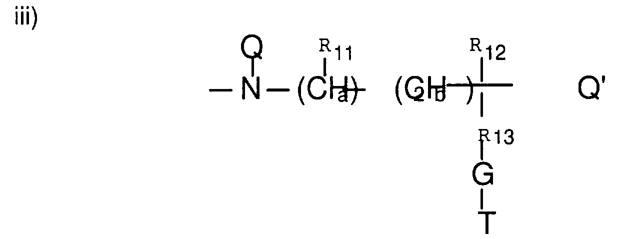
dans lequel a se situe entre 0 ou 1 et b se situe entre 0 et 3
R₁₁ représente un atome d'H ou un groupe méthyle
R₁₂ représente un atome d'H ou un groupe méthyle; ou R₁₂ représente un groupe CH₂= et Q' est absent; ou R₁₁ et R₁₂ sont liés pour former un cycle de 3 à 7 membres,
R₁₃ représente une liaison ou un groupe hydrocarbylène en C₁ à C₃
G représente une liaison, un groupe -CHOH- ou un groupe -C(O)-
Q' est comme énuméré ci-dessus pour Q ou un groupe -R₁₀-(C(O))_{d}-L-(C(O))ₑ-R₉ (dans lequel R₉ et R₁₀ sont définis comme ci-dessus, L est un atome d'O, un atome de S ou un groupe -N(R₁₄)-, dans lequel R₁₄ est défini comme l'est R₈ ci-dessus, et d et e représentent 0 ou 1, pour autant que la somme d+e soit inférieure à 2); ou alors Q' et R₁₂, ainsi que l'atome de carbone auquel ils sont liés, forment un cycle renfermant de 3 à 7 membres.
Q est défini comme ci-dessus; ou alors Q et R₁₂ forment ensemble un groupe de formule -(CH₂)_{f}-V-(CH₂)_{g}- dans lequel V représente un groupe -S-, un groupe -S(O)-, un groupe -S(O)₂-, un groupe -CH₂-, un groupe -CHOH- ou un groupe -C(O)-, f se situe entre 0 et 2 et g se situe entre 0 et 3; ou alors, si Q' représente un groupe -R₁₀-U avec U étant un groupe aromatique, Q peut additionnellement représenter une liaison méthylène avec U, dont la liaison est *ortho* par rapport à la liaison entre R₁₀ et U,
T représente un atome d'H, un groupe cyano, un groupe alkyle en C₁ à C₄, un groupe -CH₂OH-, un groupe carboxy, un groupe carboxy esterifié ou un groupe carboxy amidé; ou alors 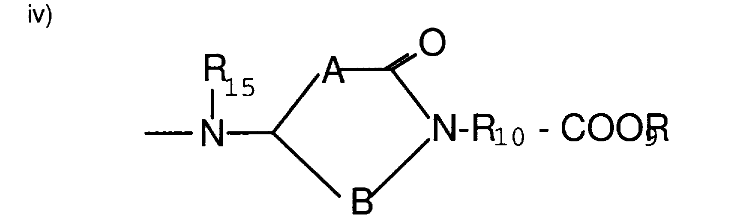
dans lequel A et B représentent de manière indépendante une liaison, ou un groupe alkylène en C₁ à C₃, pour autant que A et B fournissent ensemble entre 2 et 4 atomes de carbone dans le cycle; R₉ et R₁₀ sont définis comme ci-dessus, et R₁₅ est comme l'a été défini R₈ ci-dessus,
ou alors Z est absent et W est un atome d'H,
R₁ représente un atome d'H, un groupe méthyle, un groupe halo, un groupe carboxy, un groupe carboxy esterifié, un groupe carboxy amidé, un groupe carboxyméthyle, un groupe carboxyméthyle esterifié, ou un groupe carboxyméthyle amidé,
R₂ est choisi parmi les groupes énumérés ci-dessus pour R₁; ou alors lorsque Z est absent et W représente un atome d'H, R₂ peut additionnellement représenter un groupe -C(O)-Z', dans lequel Z' est choisi parmi les groupes énumérés ci-dessus pour Z; ou alors R₁ et R₂ forment ensemble une seconde liaison entre les atomes de carbone auxquels ils sont liés,
R₃ et R₄ (ou chacun des groupes R₃ et R₄, lorsque m ou n est égal à 2 ou plus) sont choisis de manière indépendante parmi le groupe halo, le groupe amino, le groupe nitro, le groupe cyano, le groupe sulfamoyle, le groupe alkyle en C₁ à C₃, le groupe alkoxy en C₁ à C₃, le groupe carboxy, le groupe carboxy esterifié et le groupe carboxy amidé,
R₅ et R₆ sont choisis de manière indépendante entre l'atome d'H et les groupes énumérés ci-dessus pour R₃
m se situe entre 0 et 4, pour autant que m ne dépasse pas 2 sauf si R₃ est exclusivement un groupe halo,
n se situe entre 0 et 4, pour autant que n ne dépasse pas 2 sauf si R₄ est exclusivement un groupe halo,
ou alors un sel pharmaceutiquement acceptable correspondant, pour autant que
si l'un (et seulement un) des groupes R₁ et R₂ est un groupe méthyle, m et n ne sont pas tous deux 0,
Z ne représente pas un groupe hydroxy ou un groupe méthoxy lorsque Y représente un groupe hydroxy ou un groupe méthoxy,
Z et Y ne sont pas en *trans* l'un par rapport à l'autre lorsque Z est un groupe R₉-O- et Y est un groupe R₇-O-,
-X-Y ne sont pas identiques à -W-Z lorsque R₁=R₂=H et m=n=0, et lorsque Z est absent et que R₁ et R₂ sont tous deux des atomes d'H ou font partie d'une double liaison, Y ne représente pas un groupe R₇-O-
et, en outre pour autant que le dit composé ne représente pas le 7-(N,N-diméthylaminocarbonyl)-8-méthyl-2,3,5,6-dibenzobicyclo [2.2.2] octane ou le 7-(N-méthyl-N-phénylaminocarbonyl) - 8 - méthyl - 2,3,5,6-dibenzobicyclo [2.2.2] octane.

3. Composé selon la revendication 1 ou la revendication 2, dans lequel R₇ représente un groupe alkyle en C₆ à C₈ dont la chaîne est linéaire ou ramifiée, ou un groupe R₂₅-(CH₂)ₚ-, dans lequel R₂₅ est choisi parmi un groupe phényle, un groupe 1-naphtyle, un groupe 2-naphtyle, un groupe indolyle, un groupe norbornyle, un groupe adamantyle ou un groupe cyclohexyle, et p se situe entre 0 et 3.

4. Composé selon l'une quelconque des revendications précédentes, dans lequel W représente un groupe carbonyle et X représente un groupe sulfonyle.

5. Composé selon l'une quelconque des revendications 1 à 3, dans lequel W représente un groupe carbonyle et X représente un groupe carbonyle.

6. Composé selon l'une quelconque des revendications 1 à 3, dans lequel W est un groupe sulfonyle et X est un groupe carbonyle.

7. Composé selon l'une quelconque des revendications précédentes, dans lequel R₁ et R₂ sont tous les deux des atomes d'H.

8. Composé selon l'une quelconque des revendications précédentes, dans lequel m et n sont tous les deux 0.

9. Composition pharmaceutique renfermant un composé selon l'une quelconque des revendications précédentes, en même temps qu'un diluant ou un support pharmaceutiqement acceptables.

10. Méthode de fabrication d'un composé selon la revendication 2, dans laquelle W représente un groupe carbonyle, cette dite méthode incluant l'étape de réaction entre un composé répondant à la formule 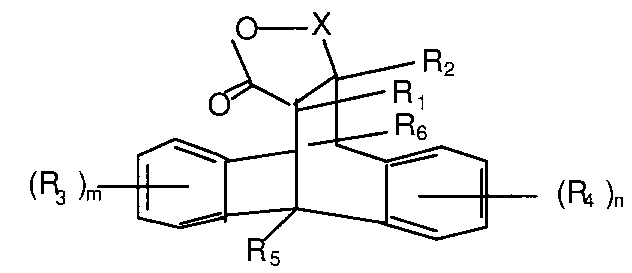 et un composé répondant à la formule YH, dans laquelle Y est défini comme dans la revendication 2.

11. Méthode de fabrication d'un composé selon la revendication 2, dans laquelle W est un groupe sufonyle, cette dite méthode comprenant l'étape de réaction entre un composé répondant à la formule 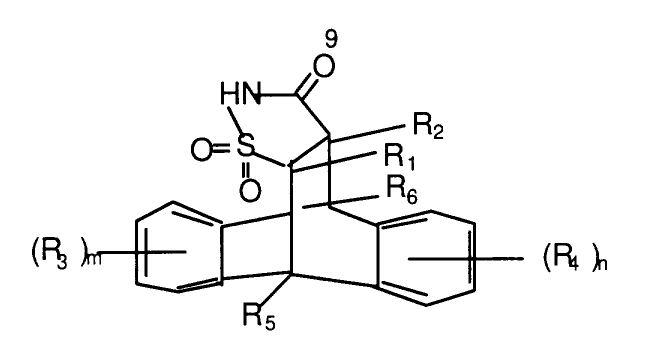 et un composé répondant à la formule R₇-Hal, dans laquelle Hal représente un atome d'halogène et R₇ est défini comme dans la revendication 2, faisant ensuite réagir le produit avec un groupe alkoxyde.

12. Méthode de fabrication d'un composé selon la revendication 2 dans laquelle W ou X est un groupe sulfonyle, cette dite méthode comprenant l'étape de réaction entre un composé répondant à la formule 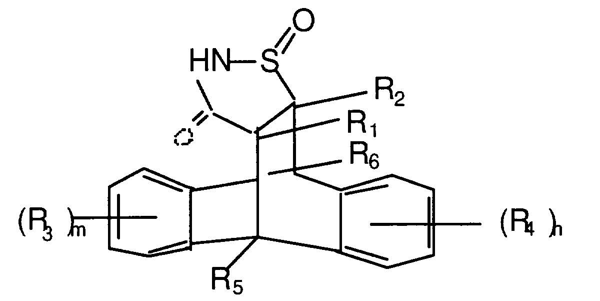 et un composé répondant à la formule R₇-Hal, dans lequel Hal représente un atome d'halogène et R₇ est défini comme dans la revendication 2, faisant ensuite réagir le produit avec un alkoxyde.

13. Méthode de fabrication d'une composition pharmaceutiquement acceptable selon la revendication 9, comprenant un mélange d'un composé selon l'une quelconque des revendications 1 à 8 avec un diluant ou un support pharmaceutiquement acceptables.
